# EUROPEAN PATENT APPLICATION

(11) **EP 4 169 578 A1**
(43) Date of publication of application: **26.04.2023**
(21) Application number: 21382958.3
(22) Date of filing: 22.10.2021
(51) Int. Cl.: A61P 35/00, C07H 15/14, C07H 15/26, C07H 21/02, C07H 21/04, C07H 99/00

(54) **OLIGOSACCHARIDE COMPOUNDS AND COMPLEXES**

(71) Applicant: BioNTech SE, 55131 Mainz (DE); Consejo Superior de Investigaciones Científicas (CSIC), 41013 Sevilla (ES); Universidad de Sevilla, 41013 Sevilla (ES)
(72) Inventor: Moreno Herrero, Jorge, 55131 Mainz (DE); Haas, Heinrich, 55131 Mainz (DE); Erbar, Stephanie, 55131 Mainz (DE); Garcia Fernandez, José Manuel, 41092 Sevilla (ES); Ortiz Mellet, Maria del Carmen, 41012 Sevilla (ES); Benito Hernandez, Juan Manuel, 41092 Sevilla (ES); López Fernandez, José, 41092 Sevilla (ES); Jiménez Blanco, José Luis, 41012 Sevilla (ES)
(74) Representative: Carlos Hernando, Borja

(57) **Abstract**

The present disclosure provides compositions comprising an oligosaccharide composition of formula I, wherein said compositions are useful for delivery of certain agents, including, for example, nucleic acids.

## Description

### Background

Targeted delivery and expression of therapeutic and/or prophylactic agents such as nucleic acids present certain challenges due to the instability of nucleic acids and their inability to permeate the cell membrane. Certain approaches, including use of lipid or polymer-based systems, exhibit promise but suffer from drawbacks related to manufacturing difficulties, poor structural definition, and high polydispersity. Still further, many such compositions lack satisfactory safety and efficacy for use in delivery of therapeutic agents.

### Summary

The present disclosure provides, among other things, cationic and/or ionizable oligosaccharide complexes that overcome certain deficiencies associated with previous compositions, and are useful for targeted delivery of biological agents, including small molecules and nucleic acids.

In some embodiments, the presently disclosure provides a composition comprising an oligosaccharide of formula I: 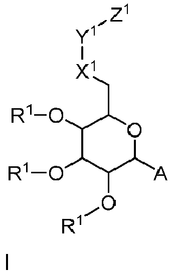 or a pharmaceutically acceptable salt thereof, wherein:
A is A¹, A², or A³: 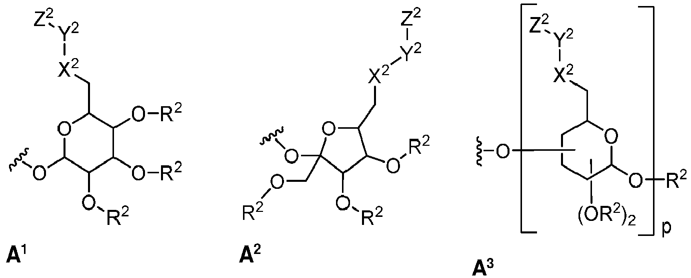
R¹ and R² are each independently selected from H, R^{a}, and -C(O)-R^{a};
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, and -NH-;
Y¹ and Y² are each independently an optionally substituted C₁₋₃₀ aliphatic group wherein one or more carbons are optionally and independently replaced by -Cy-, -NH-, -NHC(O)-, -C(O)NH-, -NHC(O)O-, -OC(O)NH-, -NHC(O)NH-, -NHC(S)NH-, -C(S)NH- - C(O)NHSO₂-, -SO₂NHC(O)-,-OC(O)O-, -O-, -C(O)-, -OC(O)-, -C(O)O-, -SO-, or -SO₂-;
each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, -N⁺(M)₃, 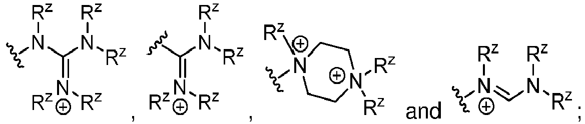
each M is independently ―C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5;
provided that when A is A¹, then:
   (i) each R¹ and R² is not ―CO-(CH₂)₄-CH₃, X¹ and X² are not both -S-, Y¹ and Y² are not both ―(CH₂)₂- and Z¹ and Z² are not both ―N⁺(H)₃,
   (ii) each R¹ and R² is not ―O-(CH₂)₅-CH₃ or ―O-(CH₂)₁₃-CH₃, X¹ and X² are not both -S-, Y¹ and Y² are not both ―(CH₂)₂-, and Z¹ and Z² are not both ―N⁺(H)₃,
   (iii) each R¹ and R² is not ―CO-(CH₂)₄-CH₃, X¹ and X² are not both -S-, Y¹ and Y² are not both ―(CH₂)₂-NH-C(S)-NH-(CH₂)₂-, and Z¹ and Z² are not both ―N⁺(H)₃, and
   (iv) Cy is not triazolyl.

In some embodiments, the present disclosure provides a complex comprising an oligosaccharide composition described herein and a nucleic acid.

In some embodiments, the present disclosure provides a method of increasing or causing increased expression of RNA in a target in a subject, the method comprising administering to the subject a complex as described herein.

In some embodiments, the present disclosure provides a method of treating and or providing prophylaxis for a disease, disorder, or condition in a subject comprising administering to the subject a complex as described herein.

In some embodiments, the present disclosure provides a method of preparing an oligosaccharide of formula **II:** 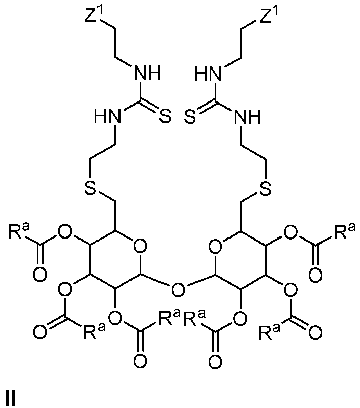 comprising contacting a compound of formula **III:** 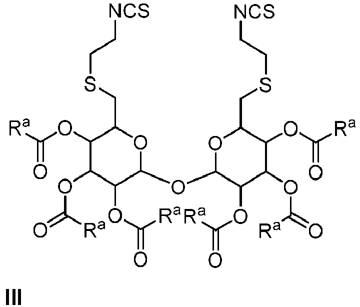 with a compound of formula **IV:** 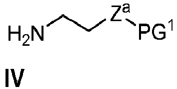
in the presence of an acid,
wherein
each R^{a} is optionally substituted -C₁-C₂₀ aliphatic;
Z¹ is a cationic or ionizable group selected from optionally substituted 5- to 14-membered heterocyclyl ring having 1-3 heteroatoms selected from N, O, and S, - N⁺(M)₃, 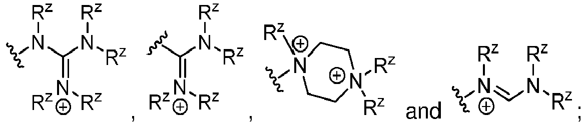
each M is independently ―C₀-C₆ aliphatic-R^{z} or ―C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S;
Z^{a}-PG¹ is Z¹ wherein one R^{z} has been replaced with a PG¹ group; and
PG¹ a suitable nitrogen protecting group.

### Brief Description of the Drawings

**FIG. 1A** is a plot indicating hydrodynamic diameter of modRNA formulated at different N/P ratios for certain oligosaccharides.
**FIG. 1B** is a plot indicating zeta potential at different N/P ratios for certain oligosaccharides.
**FIG. 1C** is a bar graph indicating quantified ex-vivo luminescence after 6h of injection of modRNA in different organs and at different N/P ratios.
**FIG. 1D** is a bar graph indicating hydrodynamic diameter of certain formulations comprising modRNA.
**FIG. 1E** is a bar graph indicating zeta potential for certain complexes.
**FIG. 1F** is a bar graph indicating quantified ex-vivo luminescence after 6h of injection of modRNA in different organs.
**FIG. 1G** is a bar graph indicating hydrodynamic diameter of modRNA formulated.
**FIG. 1H** is a bar graph indicating zeta potential for certain oligosaccharides.
**FIG. 11** is a bar graph indicating quantified ex-vivo luminescence after 6h of injection of modRNA in the spleen.

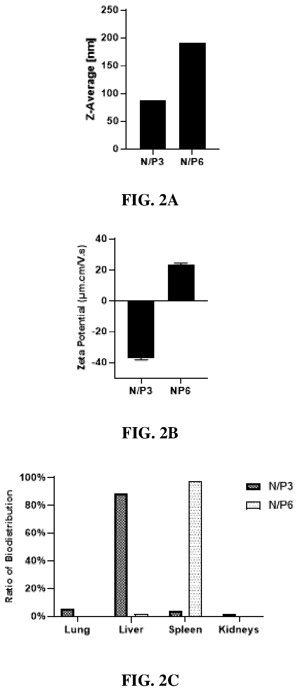

**FIG. 2A** is a bar graph indicating hydrodynamic diameter of modRNA formulated at different N/P ratios for certain oligosaccharides.
**FIG. 2B** is a bar graph indicating zeta potential at different N/P ratios for certain oligosaccharides.
**FIG. 2C** is a bar graph quantified ex-vivo luminescence after 6h of injection of modRNA in different organs and at different N/P ratios.

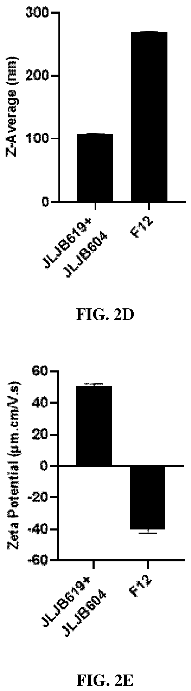

**FIG. 2D** is a bar graph indicating hydrodynamic diameter of modRNA formulated with the two different formulations.
**FIG. 2E** is a bar graph indicating zeta potential of the two different formulations.

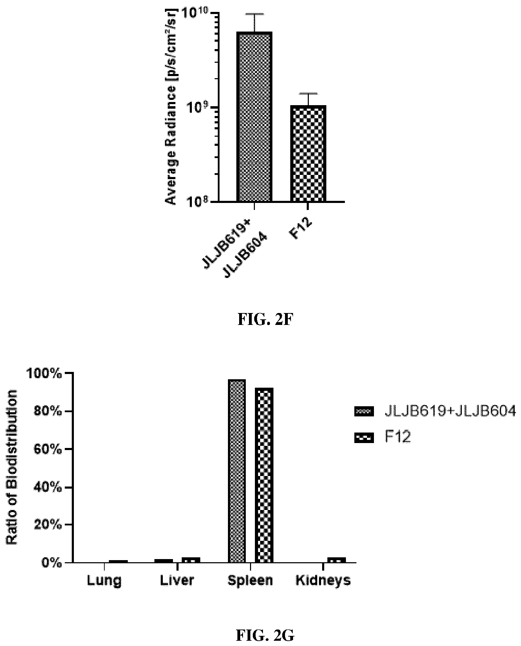

**FIG. 2F** is a bar graph indicating quantified ex-vivo luminescence after 6h of injection of modRNA in spleen.
**FIG. 2G** is a bar graph indicating quantified ex-vivo luminescence after 6h of injection of modRNA in different organs.

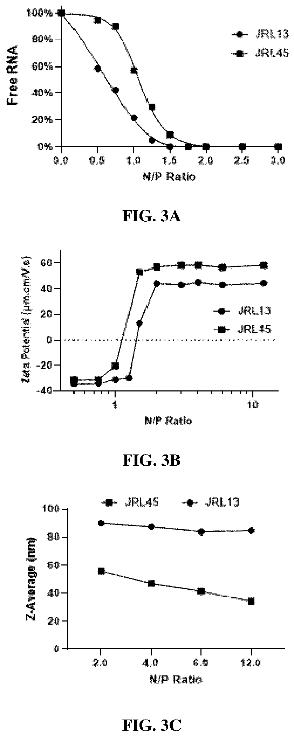

**FIG. 3A** is a bar graph indicating a percentage of free RNA quantified by gel electrophoresis of formulated modRNA at different N/P ratios between 0-3 for either JRL13 or JRL45.
**FIG. 3B** is a plot indicating zeta potential after formulation of modRNA at different N/P ratios with either JRL13 or JRL45.
**FIG. 3C** is a plot indicating hydrodynamic diameter for formulated modRNA at different N/P ratios with either JRL13 or JRL45.

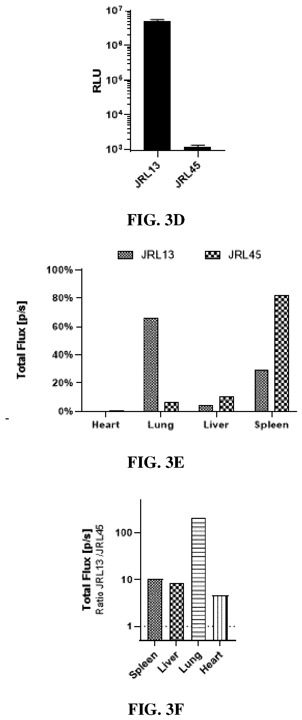

**FIG. 3D** is a bar graph indicating expression of luciferase-encoding modRNA after 24 of transfection of C2C12 cells, N/P 6 formulated modRNA with either JRL13 or JRL45, at 25 ng of RNA/well.
**FIG. 3E** is a bar graph indicating quantified ex-vivo luminescence in different organs after 6h of injection of 10 µg of modRNA formulated at N/P 3 with either JRL13 or JRL45.
**FIG. 3F** is a bar graph indicating quantified ex-vivo luminescence in different organs after 6h of the total flux [p/s] ratio from the JRL13 group to JRL45 group.

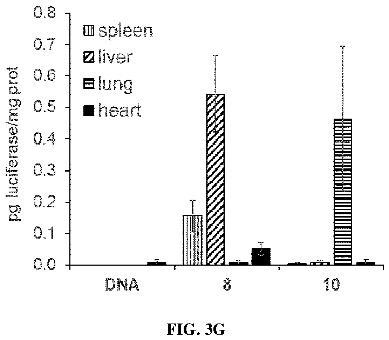

**FIG. 3G** is a bar graph indicating quantified ex-vivo luminescence in different organs after injection of pDNA complexed with either JRL13 (compound 8) or JRL45 (compound 10).

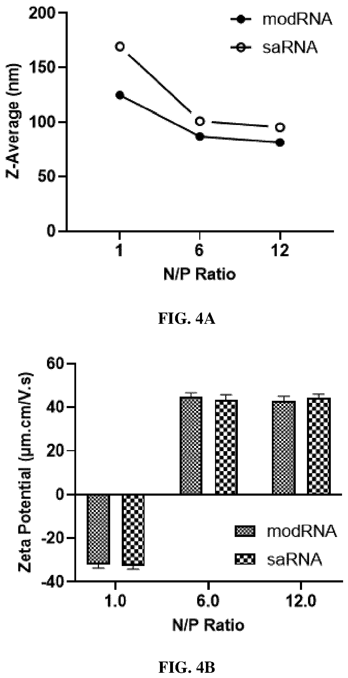

**FIG. 4A** is a plot indicating hydrodynamic diameter of complexes formulated at different N/P Ratios and RNA.
**FIG. 4B** is a bar graph indicating zeta potential at different N/P Ratios and RNA.

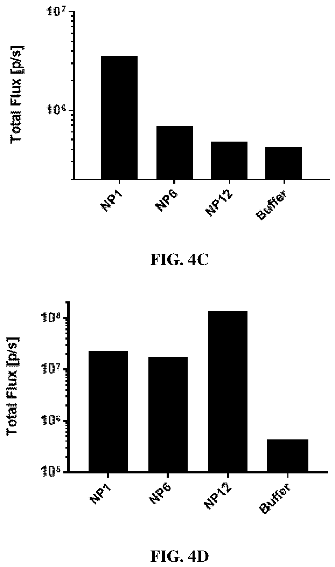

**FIG. 4C** is a bar graph indicating quantified bioluminescence over the whole duration of the experiment, per injected N/P ratio of modRNA-JRL13 formulations.
**FIG. 4D** is a bar graph indicating quantified bioluminescence over the whole duration of the experiment, per injected N/P ratio of saRNA-JRL13 formulations

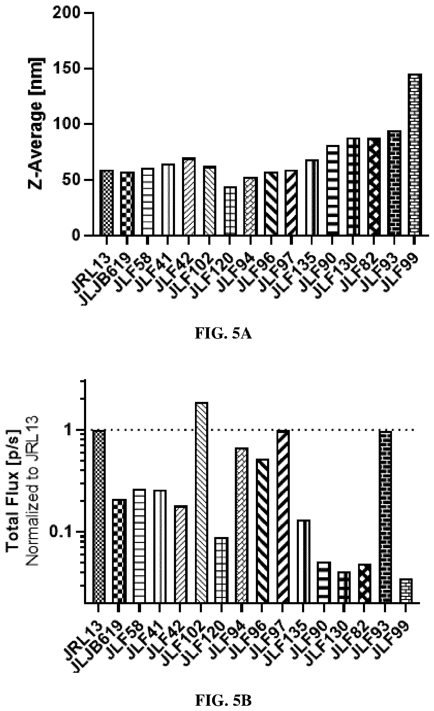

**FIG. 5A** is a bar graph indicating hydrodynamic diameter of complexes formulated at N/P6 Ratios with saRNA and different oligosaccharides.
**FIG. 5B** is a bar graph indicating quantified bioluminescence over the whole duration of the experiment and normalized to the JRL13-saRNA group.

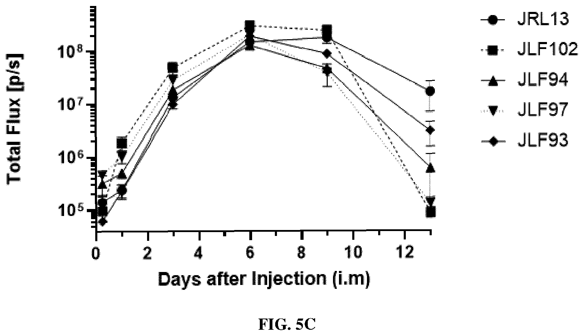

**FIG. 5C** is a plot indicating quantified bioluminescence at each measured timepoint of certain formulations.

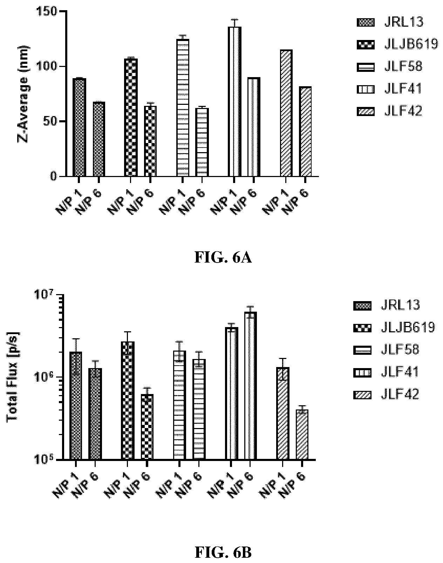

**FIG. 6A** is a bar graph indicating hydrodynamic diameter of complexes formulated at different N/P Ratios with modRNA and different oligosaccharides.
**FIG. 6B** is a bar graph indicating quantified bioluminescence over the whole duration of the experiment.

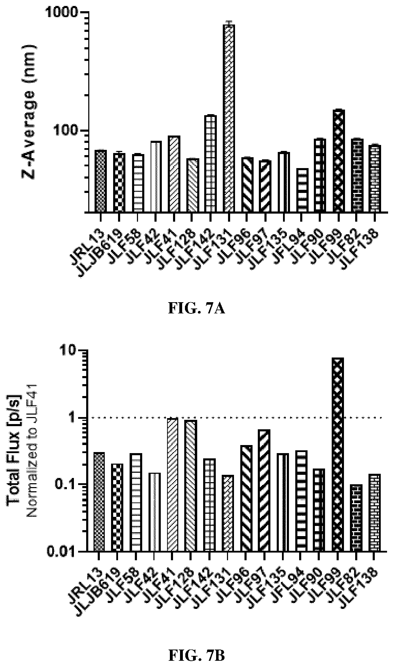

**FIG. 7A** is a bar graph indicating hydrodynamic diameter of complexes formulated at N/P 6 ratios with modRNA and different oligosaccharides.
**FIG. 7B** is a bar graph indicating quantified bioluminescence over the whole duration of the experiment and normalized to the JLF41-modRNA group.

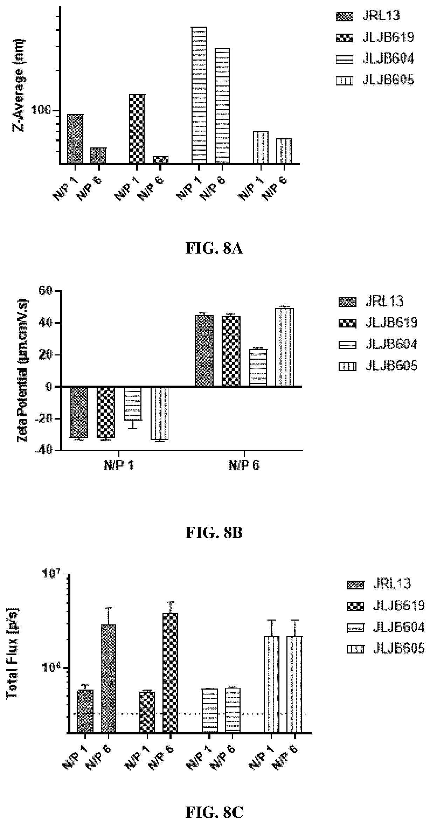

**FIG. 8A** is a bar graph indicating hydrodynamic diameter of complexes formulated at different N/P Ratios.
**FIG. 8B** is a bar graph indicating zeta potential at different N/P Ratios of injected modRNA formulations.
**FIG. 8C** is a bar graph indicating quantified bioluminescence over the whole duration of the experiment, per injected oligosaccharide formulation.

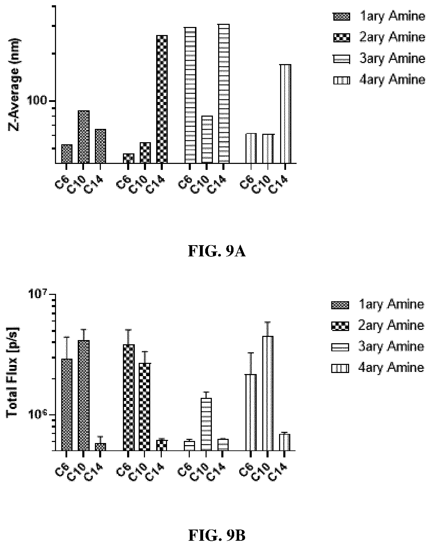

**FIG. 9A** is a bar graph indicating hydrodynamic diameter of complexes formulated.
**FIG. 9B** is a bar graph indicating quantified bioluminescence over the whole duration of the experiment, per injected oligosaccharides formulation.

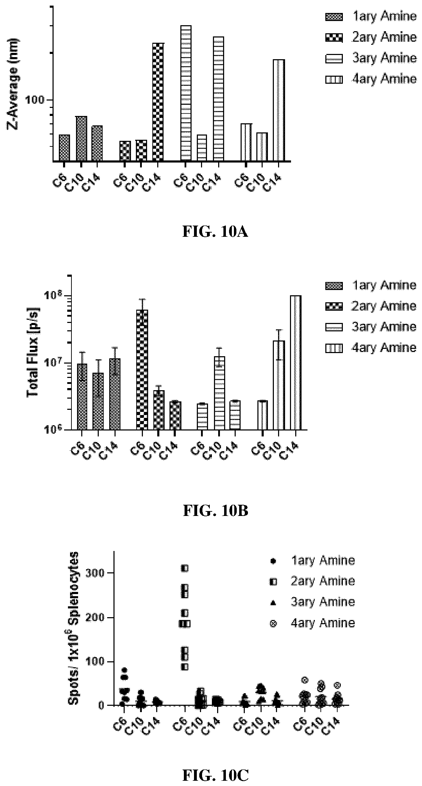

**FIG. 10A** is a bar graph indicating hydrodynamic diameter of certain complexes.
**FIG. 10B** is a bar graph indicating quantified bioluminescence over the whole duration of the experiment, per injected oligosaccharides formulation.
**FIG. 10C** is a bar graph indicating quantified response of CD8 positive T cells from spleen samples of treated mice 20 days after s.c application.

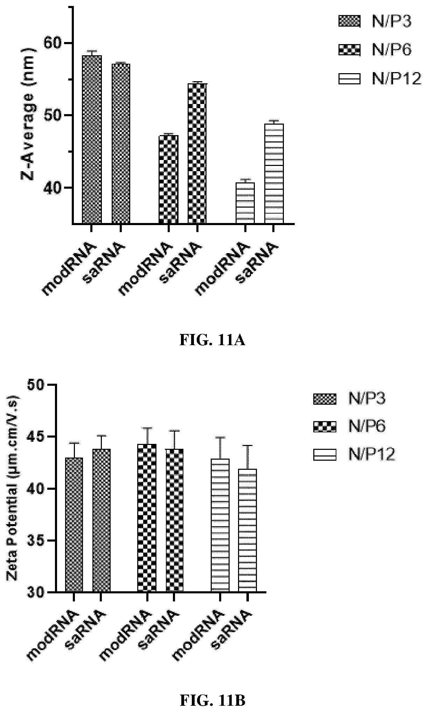

**FIG. 11A** is a bar graph indicating hydrodynamic diameter of complexes formulated at different N/P Ratios and RNA.
**FIG. 11B** is a bar graph indicating zeta potential at different N/P Ratios and RNA.

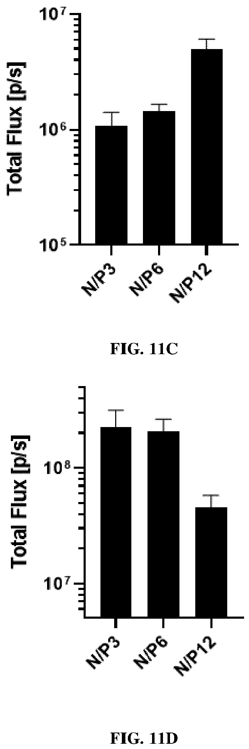

**FIG. 11C** is a bar graph indicating quantified bioluminescence over the whole duration of the experiment for modRNA groups.
**FIG. 11D** is a bar graph indicating quantified bioluminescence over the whole duration of the experiment for saRNA groups.

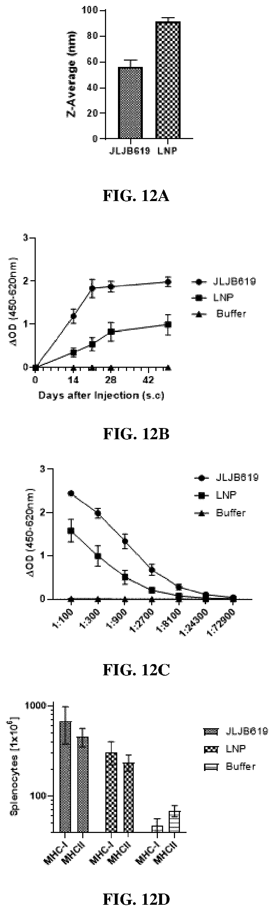

**FIG. 12A** is a bar graph indicating hydrodynamic diameter of complexes formulated at different N/P Ratios and RNA.
**FIG. 12B** is a plot showing serological levels of specific IgG against HA during the curse of experiment at a serum dilution of 1:300.
**FIG. 12C** is a plot showing end-point titration of the serological levels of specific IgG against HA.
**FIG. 12D** is a bar graph indicates the CD8/CD4 response against HA-peptide pools.

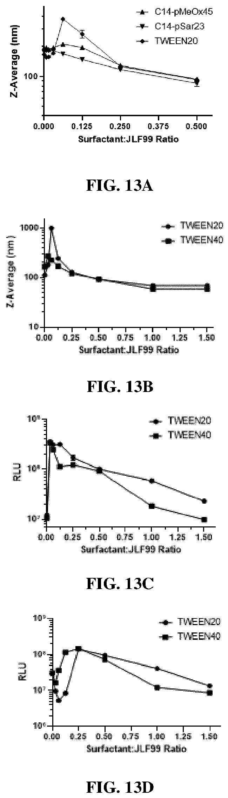

**FIG. 13A** is a plot illustrating hydrodynamic diameter of complexes formulated at N/P 6 but with different surfactants and with a surfactant-to-JLF99 ratio between 0:1 and 0.5:1 (where C14-pMeOx45 is C₁₄ alkyl-poly(2-methyl-2-oxazoline)₄₅, C14pSar23 is C14 alkyl-poly(sarcosine)₂₃, and Tween20 is polysorbate 20).
**FIG. 13B** is a plot illustrating hydrodynamic diameter of complexes formulated at N/P 6 with either Tween20 or Tween40 and within a surfactant-to-JLF99 ratio between 0 and 1.5.
**FIG. 13C** is a plot illustrating expression of luciferase-encoding modRNA after 24 of transfection of C2C12 cells, N/P6 formulated modRNA with either Tween20 or Tween40 at different surfactant-to-JLF99 ratio, at 100ng of RNA/well. Data here represents 3 independent experiments (n=3) with technical triplicates.
**FIG. 13D** is a plot illustrating expression of luciferase-encoding modRNA after 24 of transfection of HepG2 cells, N/P6 formulated modRNA with either Tween20 or Tween40 at different surfactant-to-JLF99 ratio, at 100ng of RNA/well. Data here represents 3 independent experiments (n=3) with technical triplicates.

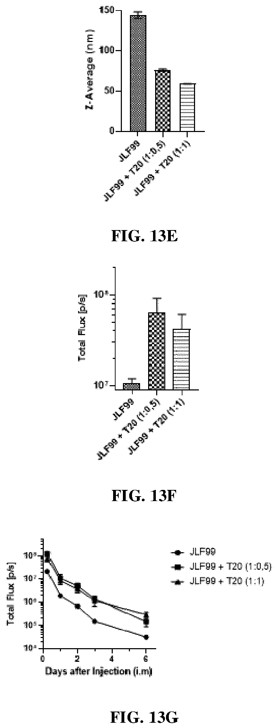

**FIG. 13E** is a bar graph illustrating hydrodynamic diameter of complexes formulated at different N/P6 (where T20 refers to Tween20).
**FIG. 13F** is a bar graph illustrating quantified bioluminescence over the whole duration of the experiment represented as area under the curve (where T20 refers to Tween20).
**FIG. 13G** is a plot illustrating quantified bioluminescence over the whole duration of the experiment (where T20 refers to Tween20).

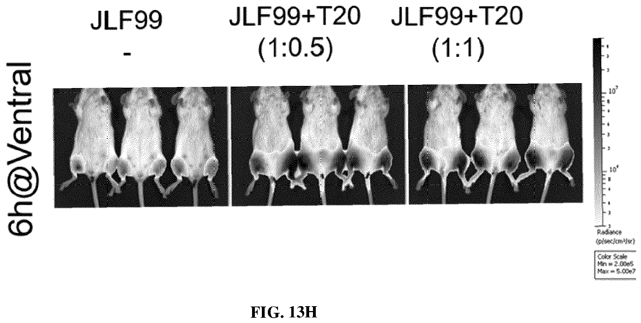

**FIG. 13H** is an image showing bioluminescence of on the ventral axis after 6hours of injection of modRNA into each group (where T20 refers to Tween20).

### Detailed Description of Certain Embodiments

### Definitions

***About or approximately:*** As used herein, the term "approximately" or "about," as applied to one or more values of interest, refers to a value that is similar to a stated reference value. In general, those skilled in the art, familiar within the context, will appreciate the relevant degree of variance encompassed by "about" or "approximately" in that context. For example, in some embodiments, the term "approximately" or "about" may encompass a range of values that are within (i.e., ±) 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value.

***Administering:*** As used herein, the term "administering" or "administration" typically refers to the administration of a composition to a subject to achieve delivery of an agent that is, or is included in, a composition to a target site or a site to be treated. Those of ordinary skill in the art will be aware of a variety of routes that may, in appropriate circumstances, be utilized for administration to a subject, for example a human. For example, in some embodiments, administration may be ocular, oral, parenteral, topical, *etc.* In some particular embodiments, administration may be bronchial (*e.g.,* by bronchial instillation), buccal, dermal (which may be or comprise, for example, one or more of topical to the dermis, intradermal, interdermal, transdermal, *etc.*), enteral, intra-arterial, intradermal, intragastric, intramedullary, intramuscular, intranasal, intraperitoneal, intrathecal, intravenous, intraventricular, within a specific organ (*e.g*., intrahepatic), mucosal, nasal, oral, rectal, subcutaneous, sublingual, topical, tracheal (*e.g.,* by intratracheal instillation), vaginal, vitreal, *etc.* In some embodiments, administration may be parenteral. In some embodiments, administration may be oral. In some particular embodiments, administration may be intravenous. In some particular embodiments, administration may be subcutaneous. In some embodiments, administration may involve only a single dose. In some embodiments, administration may involve application of a fixed number of doses. In some embodiments, administration may involve dosing that is intermittent (*e.g*., a plurality of doses separated in time) and/or periodic (*e.g*., individual doses separated by a common period of time) dosing. In some embodiments, administration may involve continuous dosing (*e.g*., perfusion) for at least a selected period of time. In some embodiments, administration may comprise a prime-and-boost protocol. A prime-and-boost protocol can include administration of a first dose of a pharmaceutical composition (e.g., an immunogenic composition, e.g., a vaccine) followed by, after an interval of time, administration of a second or subsequent dose of a pharmaceutical composition (e.g., an immunogenic composition, e.g., a vaccine). In the case of an immunogenic composition, a prime-and-boost protocol can result in an increased immune response in a patient.

***Aliphatic:*** The term "aliphatic" refers to a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "cycloaliphatic"), that has a single point or more than one points of attachment to the rest of the molecule. Unless otherwise specified, aliphatic groups contain 1-12 aliphatic carbon atoms. As used herein, it is understood that an aliphatic group can also be bivalent (e.g., encompass a bivalent hydrocarbon chain that is saturated or contains one or more units of unsaturation, such as, for example, -CH₂-, -CH₂-CH₂-, -CH₂-CH₂-CH₂-, and so on). In some embodiments, aliphatic groups contain 1-6 aliphatic carbon atoms (e.g., C₁₋₆). In some embodiments, aliphatic groups contain 1-5 aliphatic carbon atoms (e.g., C₁₋₅). In other embodiments, aliphatic groups contain 1-4 aliphatic carbon atoms (e.g., C₁₋₄). In still other embodiments, aliphatic groups contain 1-3 aliphatic carbon atoms (e.g., C₁₋₃), and in yet other embodiments, aliphatic groups contain 1-2 aliphatic carbon atoms (e.g., C₁₋₂). In some embodiments, "cycloaliphatic" refers to a monocyclic C₃₋₈ hydrocarbon or a bicyclic C₇₋₁₀ hydrocarbon that is completely saturated or that contains one or more units of unsaturation, but which is not aromatic, that has a single point or more than one points of attachment to the rest of the molecule. Suitable aliphatic groups include, but are not limited to, linear or branched, substituted or unsubstituted alkyl, alkenyl, or alkynyl groups and hybrids thereof. A preferred aliphatic group is C₁₋₆ alkyl.

***Alkyl:*** The term "alkyl", used alone or as part of a larger moiety, refers to a saturated, optionally substituted straight or branched chain hydrocarbon group having (unless otherwise specified) 1-12, 1-10, 1-8, 1-6, 1-4, 1-3, or 1-2 carbon atoms (e.g., C₁₋₁₂, C₁₋₁₀, C₁₋₈, C₁₋₆, C₁₋₄, C₁₋₃, or C₁₋₂). Exemplary alkyl groups include methyl, ethyl, propyl, butyl, pentyl, hexyl, and heptyl.

***Alkenyl:*** The term "alkenyl", used alone or as part of a larger moiety, refers to an optionally substituted straight or branched chain or cyclic hydrocarbon group having at least one double bond and having (unless otherwise specified) 2-12, 2-10, 2-8, 2-6, 2-4, or 2-3 carbon atoms(e.g., C₂₋₁₂, C₂₋₁₀, C₂₋₈, C₂₋₆, C₂₋₄, or C₂₋₃). Exemplary alkenyl groups include ethenyl, propenyl, butenyl, pentenyl, hexenyl, and heptenyl. The term "cycloalkenyl" refers to an optionally substituted non-aromatic monocyclic or multicyclic ring system containing at least one carbon-carbon double bond and having about 3 to about 10 carbon atoms. Exemplary monocyclic cycloalkenyl rings include cyclopentenyl, cyclohexenyl, and cycloheptenyl.

***Alkynyl:*** The term "alkynyl", used alone or as part of a larger moiety, refers to an optionally substituted straight or branched chain hydrocarbon group having at least one triple bond and having (unless otherwise specified) 2-12, 2-10, 2-8, 2-6, 2-4, or 2-3 carbon atoms (e.g., C₂₋₁₂, C₂₋₁₀, C₂₋₈, C₂₋₆, C₂₋₄, or C₂₋₃). Exemplary alkynyl groups include ethynyl, propynyl, butynyl, pentynyl, hexynyl, and heptynyl.

***Analog:*** As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, e.g., by chemical manipulation of the reference substance. In some embodiments, an analog is a substance that can be generated through performance of a synthetic process substantially similar to (e.g., sharing a plurality of steps with) one that generates the reference substance. In some embodiments, an analog is or can be generated through performance of a synthetic process different from that used to generate the reference substance.

***Aryl:*** The term "aryl" refers to monocyclic and bicyclic ring systems having a total of five to fourteen ring members (e.g., C₅-C₁₄), wherein at least one ring in the system is aromatic and wherein each ring in the system contains three to seven ring members. In some embodiments, an "aryl" group contains between six and twelve total ring members (e.g., C₆-C₁₂). The term "aryl" may be used interchangeably with the term "aryl ring". In certain embodiments of the present invention, "aryl" refers to an aromatic ring system which includes, but not limited to, phenyl, biphenyl, naphthyl, anthracyl and the like, which may bear one or more substituents. Unless otherwise specified, "aryl" groups are hydrocarbons. In some embodiments, an "aryl" ring system is an aromatic ring (e.g., phenyl) that is fused to a non-aromatic ring (e.g., cycloalkyl). Examples of aryl rings include that are fused include

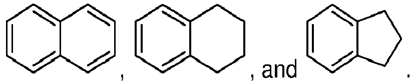

***Associated:*** Two events or entities are "associated" with one another, as that term is used herein, if the presence, level and/or form of one is correlated with that of the other. For example, a particular entity (e.g., polypeptide, genetic signature, metabolite, microbe, etc) is considered to be associated with a particular disease, disorder, or condition, if its presence, level and/or form correlates with incidence of and/or susceptibility to the disease, disorder, or condition (e.g., across a relevant population). In some embodiments, two or more entities are physically "associated" with one another if they interact, directly or indirectly, so that they are and/or remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

***Biological sample:*** As used herein, the term "biological sample" typically refers to a sample obtained or derived from a biological source (e.g., a tissue or organism or cell culture) of interest, as described herein. In some embodiments, a source of interest comprises an organism, such as an animal or human. In some embodiments, a biological sample is or comprises biological tissue or fluid. In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids (e.g., sperm, sweat, tears), secretions, and/or excretions; and/or cells therefrom, etc. In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (e.g., fine needle aspiration or tissue biopsy), surgery, collection of body fluid (e.g., blood, lymph, feces etc.), etc. In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example, nucleic acids or proteins extracted from a sample or obtained by subjecting a primary sample to techniques such as amplification or reverse transcription of mRNA, isolation and/or purification of certain components, etc.

***Carrier:*** As used herein, the term "carrier" refers to a diluent, adjuvant, excipient, or vehicle with which a composition is administered. In some exemplary embodiments, carriers can include sterile liquids, such as, for example, water and oils, including oils of petroleum, animal, vegetable or synthetic origin, such as, for example, peanut oil, soybean oil, mineral oil, sesame oil and the like. In some embodiments, carriers are or include one or more solid components.

***Combination therapy:*** As used herein, the term "combination therapy" refers to those situations in which a subject is simultaneously exposed to two or more therapeutic regimens (e.g., two or more therapeutic agents or modality(ies)). In some embodiments, the two or more regimens may be administered simultaneously; in some embodiments, such regimens may be administered sequentially (e.g., all "doses" of a first regimen are administered prior to administration of any doses of a second regimen); in some embodiments, such agents are administered in overlapping dosing regimens. In some embodiments, "administration" of combination therapy may involve administration of one or more agent(s) or modality(ies) to a subject receiving the other agent(s) or modality(ies) in the combination. For clarity, combination therapy does not require that individual agents be administered together in a single composition (or even necessarily at the same time), although in some embodiments, two or more agents, or active moieties thereof, may be administered together in a combination composition, or even in a combination compound (e.g., as part of a single chemical complex or covalent entity).

***Comparable*:** As used herein, the term "comparable" refers to two or more agents, entities, situations, sets of conditions, etc., that may not be identical to one another but that are sufficiently similar to permit comparison therebetween so that one skilled in the art will appreciate that conclusions may reasonably be drawn based on differences or similarities observed. In some embodiments, comparable sets of conditions, circumstances, individuals, or populations are characterized by a plurality of substantially identical features and one or a small number of varied features. Those of ordinary skill in the art will understand, in context, what degree of identity is required in any given circumstance for two or more such agents, entities, situations, sets of conditions, etc. to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

***Composition:*** Those skilled in the art will appreciate that the term "composition" may be used to refer to a discrete physical entity that comprises one or more specified components. In general, unless otherwise specified, a composition may be of any form - e.g., gas, gel, liquid, solid, etc.

***Cycloaliphatic*:** As used herein, the term "cycloaliphatic" refers to a monocyclic C₃₋₈ hydrocarbon or a bicyclic C₇₋₁₀ hydrocarbon that is completely saturated or that contains one or more units of unsaturation, but which is not aromatic, that has a single point or more than one points of attachment to the rest of the molecule.

***Cycloalkyl*:** As used herein, the term "cycloalkyl" refers to an optionally substituted saturated ring monocyclic or polycyclic system of about 3 to about 10 ring carbon atoms. Exemplary monocyclic cycloalkyl rings include cyclopropyl, cyclobutyl, cyclopentyl, cyclohexyl, and cycloheptyl.

***Dosage form*** or ***unit dosage form:*** Those skilled in the art will appreciate that the term "dosage form" may be used to refer to a physically discrete unit of an active agent *(e.g.,* a therapeutic or diagnostic agent) for administration to a subject. Typically, each such unit contains a predetermined quantity of active agent. In some embodiments, such quantity is a unit dosage amount (or a whole fraction thereof) appropriate for administration in accordance with a dosing regimen that has been determined to correlate with a desired or beneficial outcome when administered to a relevant population (*i.e*., with a therapeutic dosing regimen). Those of ordinary skill in the art appreciate that the total amount of a therapeutic composition or agent administered to a particular subject is determined by one or more attending physicians and may involve administration of multiple dosage forms.

***Dosing regimen*** or ***therapeutic regimen:*** Those skilled in the art will appreciate that the terms "dosing regimen" and "therapeutic regimen" may be used to refer to a set of unit doses (typically more than one) that are administered individually to a subject, typically separated by periods of time. In some embodiments, a given therapeutic agent has a recommended dosing regimen, which may involve one or more doses. In some embodiments, a dosing regimen comprises a plurality of doses each of which is separated in time from other doses. In some embodiments, individual doses are separated from one another by a time period of the same length; in some embodiments, a dosing regimen comprises a plurality of doses and at least two different time periods separating individual doses. In some embodiments, all doses within a dosing regimen are of the same unit dose amount. In some embodiments, different doses within a dosing regimen are of different amounts. In some embodiments, a dosing regimen comprises a first dose in a first dose amount, followed by one or more additional doses in a second dose amount different from the first dose amount. In some embodiments, a dosing regimen comprises a first dose in a first dose amount, followed by one or more additional doses in a second dose amount same as the first dose amount. In some embodiments, a dosing regimen is correlated with a desired or beneficial outcome when administered across a relevant population (*i.e.,* is a therapeutic dosing regimen).

***Excipient:*** As used herein, the term "excipient" refers to a non-therapeutic agent that may be included in a pharmaceutical composition, for example, to provide or contribute to a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

***Heteroaliphatic*:** The term "heteroaliphatic" or "heteroaliphatic group", as used herein, denotes an optionally substituted hydrocarbon moiety having, in addition to carbon atoms, from one to five heteroatoms, that may be straight-chain (*i.e.,* unbranched), branched, or cyclic ("heterocyclic") and may be completely saturated or may contain one or more units of unsaturation, but which is not aromatic. The term "heteroatom" refers to nitrogen, oxygen, or sulfur, and includes any oxidized form of nitrogen or sulfur, and any quaternized form of a basic nitrogen. The term "nitrogen" also includes a substituted nitrogen. Unless otherwise specified, heteroaliphatic groups contain 1-10 carbon atoms wherein 1-3 carbon atoms are optionally and independently replaced with heteroatoms selected from oxygen, nitrogen, and sulfur. In some embodiments, heteroaliphatic groups contain 1-4 carbon atoms, wherein 1-2 carbon atoms are optionally and independently replaced with heteroatoms selected from oxygen, nitrogen, and sulfur. In yet other embodiments, heteroaliphatic groups contain 1-3 carbon atoms, wherein 1 carbon atom is optionally and independently replaced with a heteroatom selected from oxygen, nitrogen, and sulfur. Suitable heteroaliphatic groups include, but are not limited to, linear or branched, heteroalkyl, heteroalkenyl, and heteroalkynyl groups. For example, a 1- to 10 atom heteroaliphatic group includes the following exemplary groups: -O-CH₃, -CH₂-O-CH₃, -O-CH₂-CH₂-O-CH₂-CH₂-O-CH₃, and the like.

***Heteroaryl:*** The terms "heteroaryl" and "heteroar-", used alone or as part of a larger moiety, e.g., "heteroaralkyl", or "heteroaralkoxy", refer to monocyclic or bicyclic ring groups having 5 to 12 ring atoms (e.g., 5- to 6- membered monocyclic heteroaryl or 9-to 12-membered bicyclic heteroaryl); having 6, 10, or 14 π-electrons shared in a cyclic array; and having, in addition to carbon atoms, from one to five heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, pteridinyl, imidazo[1,2-a]pyrimidinyl, imidazo[1,2-a]pyridyl, imidazo[4,5-b]pyridyl, imidazo[4,5-c]pyridyl, pyrrolopyridyl, pyrrolopyrazinyl, thienopyrimidinyl, triazolopyridyl, and benzoisoxazolyl. The terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring (i.e., a bicyclic heteroaryl ring having 1 to 3 heteroatoms). Nonlimiting examples include indolyl, isoindolyl, benzothienyl, benzofuranyl, dibenzofuranyl, indazolyl, benzimidazolyl, benzotriazolyl, benzothiazolyl, benzothiadiazolyl, benzoxazolyl, quinolyl, isoquinolyl, cinnolinyl, phthalazinyl, quinazolinyl, quinoxalinyl, 4*H*-quinolizinyl, carbazolyl, acridinyl, phenazinyl, phenothiazinyl, phenoxazinyl, tetrahydroquinolinyl, tetrahydroisoquinolinyl, pyrido[2,3-b]-1,4-oxazin-3(4H)-one, 4H-thieno[3,2-b]pyrrole, and benzoisoxazolyl. A heteroaryl group may be mono- or bicyclic. The term "heteroaryl" may be used interchangeably with the terms "heteroaryl ring", "heteroaryl group", or "heteroaromatic", any of which terms include rings that are optionally substituted. The term "heteroaralkyl" refers to an alkyl group substituted by a heteroaryl, wherein the alkyl and heteroaryl portions independently are optionally substituted.

***Heteroatom:*** The term "heteroatom" as used herein refers to nitrogen, oxygen, or sulfur, and includes any oxidized form of nitrogen or sulfur, and any quaternized form of a basic nitrogen.

***Heterocycle:*** As used herein, the terms "heterocycle", "heterocyclyl", "heterocyclic radical", and "heterocyclic ring" are used interchangeably and refer to a stable 3- to 8-membered monocyclic, a 7- to 12-membered bicyclic, or a 10- to 16-membered polycyclic heterocyclic moiety that is either saturated or partially unsaturated, and having, in addition to carbon atoms, one or more, such as one to four, heteroatoms, as defined above. When used in reference to a ring atom of a heterocycle, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2H-pyrrolyl), NH (as in pyrrolidinyl), or NR+ (as in N-substituted pyrrolidinyl). A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, azetidinyl, oxetanyl, tetrahydrofuranyl, tetrahydrothienyl, pyrrolidinyl, piperidinyl, decahydroquinolinyl, oxazolidinyl, piperazinyl, dioxanyl, dioxolanyl, diazepinyl, oxazepinyl, thiazepinyl, morpholinyl, and thiamorpholinyl. A heterocyclyl group may be mono-, bi-, tri-, or polycyclic, preferably mono-, bi-, or tricyclic, more preferably mono- or bicyclic. The term "heterocyclylalkyl" refers to an alkyl group substituted by a heterocyclyl, wherein the alkyl and heterocyclyl portions independently are optionally substituted. A bicyclic heterocyclic ring also includes groups in which the heterocyclic ring is fused to one or more aryl rings. Exemplary bicyclic heterocyclic groups include indolinyl, isoindolinyl, benzodioxolyl, 1,3-dihydroisobenzofuranyl, 2,3-dihydrobenzofuranyl, tetrahydroquinolinyl, 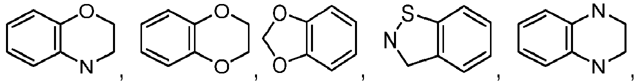 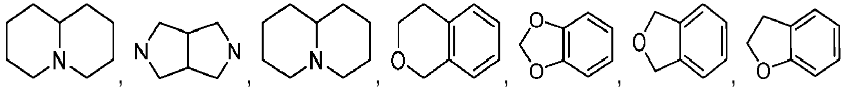 , and 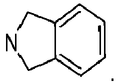 A bicyclic heterocyclic ring can also be a spirocyclic ring system (e.g., 7- to 11-membered spirocyclic fused heterocyclic ring having, in addition to carbon atoms, one or more heteroatoms as defined above (e.g., one, two, three or four heteroatoms)). A bicyclic heterocyclic ring can also be a bridged ring system (e.g., 7-to 11-membered bridged heterocyclic ring having one, two, or three bridging atoms.

***Nucleic acid*/ *Polynucleotide*:** As used herein, the term "nucleic acid" refers to a polymer of at least 10 nucleotides or more. In some embodiments, a nucleic acid is or comprises DNA. In some embodiments, a nucleic acid is or comprises RNA. In some embodiments, a nucleic acid is or comprises peptide nucleic acid (PNA). In some embodiments, a nucleic acid is or comprises a single stranded nucleic acid. In some embodiments, a nucleic acid is or comprises a double-stranded nucleic acid. In some embodiments, a nucleic acid comprises both single and double-stranded portions. In some embodiments, a nucleic acid comprises a backbone that comprises one or more phosphodiester linkages. In some embodiments, a nucleic acid comprises a backbone that comprises both phosphodiester and non-phosphodiester linkages. For example, in some embodiments, a nucleic acid may comprise a backbone that comprises one or more phosphorothioate or 5'-N-phosphoramidite linkages and/or one or more peptide bonds, *e.g.,* as in a "peptide nucleic acid". In some embodiments, a nucleic acid comprises one or more, or all, natural residues (*e.g.,* adenine, cytosine, deoxyadenosine, deoxycytidine, deoxyguanosine, deoxythymidine, guanine, thymine, uracil). In some embodiments, a nucleic acid comprises on or more, or all, non-natural residues. In some embodiments, a non-natural residue comprises a nucleoside analog (*e.g.,* 2-aminoadenosine, 2-thiothymidine, inosine, pyrrolo-pyrimidine, 3 -methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 6-0-methylguanine, 2-thiocytidine, methylated bases, intercalated bases, and combinations thereof). In some embodiments, a non-natural residue comprises one or more modified sugars (*e.g.,* 2'-fluororibose, ribose, 2'-deoxyribose, arabinose, and hexose) as compared to those in natural residues. In some embodiments, a nucleic acid has a nucleotide sequence that encodes a functional gene product such as an RNA or polypeptide. In some embodiments, a nucleic acid has a nucleotide sequence that comprises one or more introns. In some embodiments, a nucleic acid may be prepared by isolation from a natural source, enzymatic synthesis (*e.g*., by polymerization based on a complementary template, *e.g., in vivo* or *in vitro,* reproduction in a recombinant cell or system, or chemical synthesis. In some embodiments, a nucleic acid is at least 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, or 20,000 or more residues or nucleotides long.

***Nucleic acid particle:*** A "nucleic acid particle" can be used to deliver nucleic acid to a target site of interest (e.g., cell, tissue, organ, and the like). A nucleic acid particle may be formed from at least one cationic or cationically ionizable lipid or lipid-like material, at least one cationic polymer such as protamine, or a mixture thereof and nucleic acid. Nucleic acid particles include lipid nanoparticle (LNP)-based and lipoplex (LPX)-based formulations.

***Nucleotide:*** As used herein, the term "nucleotide" refers to its art-recognized meaning. When a number of nucleotides is used as an indication of size, *e.g.,* of a polynucleotide, a certain number of nucleotides refers to the number of nucleotides on a single strand, *e.g.,* of a polynucleotide.

***Oral:*** The phrases "oral administration" and "administered orally" as used herein have their art-understood meaning referring to administration by mouth of a compound or composition.

***Parenteral:*** The phrases "parenteral administration" and "administered parenterally" as used herein have their art-understood meaning referring to modes of administration other than enteral and topical administration, usually by injection, and include, without limitation, intravenous, intramuscular, intra-arterial, intrathecal, intracapsular, intraorbital, intracardiac, intradermal, intraperitoneal, transtracheal, subcutaneous, subcuticular, intraarticulare, subcapsular, subarachnoid, intraspinal, and intrasternal injection and infusion.

***Partially unsaturated:*** As used herein, the term "partially unsaturated" refers to a ring moiety that includes at least one double or triple bond between ring atoms. The term "partially unsaturated" is intended to encompass rings having multiple sites of unsaturation, but is not intended to include aromatic (e.g., aryl or heteroaryl) moieties, as herein defined.

***Patient*** or ***subject:*** As used herein, the term "patient" or "subject" refers to any organism to which a provided composition is or may be administered, *e.g.,* for experimental, diagnostic, prophylactic, cosmetic, and/or therapeutic purposes. Typical patients or subjects include animals (*e.g.*, mammals such as mice, rats, rabbits, non-human primates, and/or humans). In some embodiments, a patient is a human. In some embodiments, a patient or a subject is suffering from or susceptible to one or more disorders or conditions. In some embodiments, a patient or subject displays one or more symptoms of a disorder or condition. In some embodiments, a patient or subject has been diagnosed with one or more disorders or conditions. In some embodiments, a patient or a subject is receiving or has received certain therapy to diagnose and/or to treat a disease, disorder, or condition.

***Pharmaceutical composition:*** As used herein, the term "pharmaceutical composition" refers to an active agent, formulated together with one or more pharmaceutically acceptable carriers. In some embodiments, the active agent is present in unit dose amount appropriate for administration in a therapeutic or dosing regimen that shows a statistically significant probability of achieving a predetermined therapeutic effect when administered to a relevant population. In some embodiments, pharmaceutical compositions may be specially formulated for administration in solid or liquid form, including those adapted for the following: oral administration, for example, drenches (aqueous or non-aqueous solutions or suspensions), tablets, *e.g.,* those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

***Pharmaceutically acceptable:*** As used herein, the phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

***Pharmaceutically acceptable carrier:*** As used herein, the term "pharmaceutically acceptable carrier" means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

***Pharmaceutically acceptable salt:*** The term "pharmaceutically acceptable salt", as used herein, refers to salts of such compounds that are appropriate for use in pharmaceutical contexts, *i.e.,* salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describes pharmaceutically acceptable salts in detail in J. Pharmaceutical Sciences, 66: 1-19 (1977). In some embodiments, pharmaceutically acceptable salts include, but are not limited to, nontoxic acid addition salts, which are salts of an amino group formed with inorganic acids such as hydrochloric acid, hydrobromic acid, phosphoric acid, sulfuric acid and perchloric acid or with organic acids such as acetic acid, maleic acid, tartaric acid, citric acid, succinic acid or malonic acid or by using other methods used in the art such as ion exchange. In some embodiments, pharmaceutically acceptable salts include, but are not limited to, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. In some embodiments, pharmaceutically acceptable salts include, when appropriate, nontoxic ammonium, quaternary ammonium, and amine cations formed using counterions such as halide, hydroxide, carboxylate, sulfate, phosphate, nitrate, alkyl having from 1 to 6 carbon atoms, sulfonate and aryl sulfonate.

***Polycyclic*:** As used herein, the term "polycyclic" refers to a saturated or unsaturated ring system having two or more rings (for example, heterocyclyl rings, heteroaryl rings, cycloalkyl rings, or aryl rings), having between 7 and 20 atoms, in which one or more carbon atoms are common to two adjacent rings. For example, in some embodiments, a polycyclic ring system refers to a saturated or unsaturated ring system having three or more rings (for example, heterocyclyl rings, heteroaryl rings, cycloalkyl rings, or aryl rings), having between 14 and 20 atoms, in which one or more carbon atoms are common to two adjacent rings. The rings in a polycyclic ring system may be fused (i.e., bicyclic or tricyclic), spirocyclic, or a combination thereof.

***Polypeptide*:** The term "polypeptide", as used herein, typically has its art-recognized meaning of a polymer of at least three amino acids or more. Those of ordinary skill in the art will appreciate that the term "polypeptide" is intended to be sufficiently general as to encompass not only polypeptides having a complete sequence recited herein, but also to encompass polypeptides that represent functional, biologically active, or characteristic fragments, portions or domains (*e.g.,* fragments, portions, or domains retaining at least one activity) of such complete polypeptides. In some embodiments, polypeptides may contain L-amino acids, D-amino acids, or both and/or may contain any of a variety of amino acid modifications or analogs known in the art. Useful modifications include, *e.g.,* terminal acetylation, amidation, methylation, *etc.* In some embodiments, polypeptides may comprise natural amino acids, non-natural amino acids, synthetic amino acids, and combinations thereof (*e.g.,* may be or comprise peptidomimetics).

***Prevent*** or ***prevention:*** As used herein, the terms "prevent" or "prevention", when used in connection with the occurrence of a disease, disorder, and/or condition, refer to reducing the risk of developing the disease, disorder and/or condition and/or to delaying onset of one or more characteristics or symptoms of the disease, disorder or condition. Prevention may be considered complete when onset of a disease, disorder or condition has been delayed for a predefined period of time.

***Reference:*** As used herein describes a standard or control relative to which a comparison is performed. For example, in some embodiments, an agent, animal, individual, population, sample, sequence or value of interest is compared with a reference or control agent, animal, individual, population, sample, sequence or value. In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control.

***Ribonucleotide:*** As used herein, the term "ribonucleotide" encompasses unmodified ribonucleotides and modified ribonucleotides. For example, unmodified ribonucleotides include the purine bases adenine (A) and guanine (G), and the pyrimidine bases cytosine (C) and uracil (U). Modified ribonucleotides may include one or more modifications including, but not limited to, for example, (a) end modifications, *e.g.,* 5' end modifications (e.g., phosphorylation, dephosphorylation, conjugation, inverted linkages, *etc.),* 3' end modifications *(e.g.,* conjugation, inverted linkages, *etc.),* (b) base modifications, e.g. , replacement with modified bases, stabilizing bases, destabilizing bases, or bases that base pair with an expanded repertoire of partners, or conjugated bases, (c) sugar modifications (*e.g.,* at the 2' position or 4' position) or replacement of the sugar, and (d) internucleoside linkage modifications, including modification or replacement of the phosphodiester linkages. The term "ribonucleotide" also encompasses ribonucleotide triphosphates including modified and non-modified ribonucleotide triphosphates.

***Ribonucleic acid (RNA):*** As used herein, the term "RNA" refers to a polymer of ribonucleotides. In some embodiments, an RNA is single stranded. In some embodiments, an RNA is double stranded. In some embodiments, an RNA comprises both single and double stranded portions. In some embodiments, an RNA can comprise a backbone structure as described in the definition of *"Nucleic acid* / *Polynucleotide"* above. An RNA can be a regulatory RNA (*e.g.,* siRNA, microRNA, *etc*.), or a messenger RNA (mRNA). In some embodiments where an RNA is a mRNA. In some embodiments where an RNA is a mRNA, a RNA typically comprises at its 3' end a poly(A) region. In some embodiments where an RNA is a mRNA, an RNA typically comprises at its 5' end an art-recognized cap structure, *e.g.,* for recognizing and attachment of a mRNA to a ribosome to initiate translation. In some embodiments, a RNA is a synthetic RNA. Synthetic RNAs include RNAs that are synthesized *in vitro* (*e.g.,* by enzymatic synthesis methods and/or by chemical synthesis methods). ***Sample:*** As used herein, the term "sample" typically refers to an aliquot of material obtained or derived from a source of interest. In some embodiments, a source of interest is a biological or environmental source. In some embodiments, a source of interest may be or comprise a cell, tissue, or organism, such as a microbe, a plant, or an animal (e.g., a human). In some embodiments, a source of interest is or comprises biological tissue or fluid. In some embodiments, a source of interest may be or comprise a preparation generated in a production run. In some embodiments, a sample is a "primary sample" obtained directly from a source of interest by any appropriate means. In some embodiments, as will be clear from context, the term "sample" refers to a preparation that is obtained by processing (e.g., by removing one or more components of and/or by adding one or more agents to) a primary sample.

***Substituted*** or ***optionally substituted:*** As described herein, compounds of the invention may contain "optionally substituted" moieties. In general, the term "substituted," whether preceded by the term "optionally" or not, means that one or more hydrogens of the designated moiety are replaced with a suitable substituent. "Substituted" applies to one or more hydrogens that are either explicit or implicit from the structure (e.g., 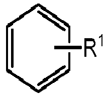 refers to at least 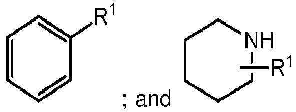 refers to at least 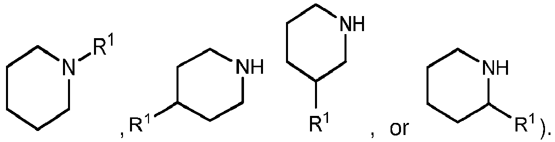 Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable," as used herein, refers to compounds that are not substantially altered when subjected to conditions to allow for their production, detection, and, in certain embodiments, their recovery, purification, and use for one or more of the purposes provided herein. Groups described as being "substituted" preferably have between 1 and 4 substituents, more preferably 1 or 2 substituents. Groups described as being "optionally substituted" may be unsubstituted or be "substituted" as described above.

Suitable monovalent substituents on a substitutable carbon atom of an "optionally substituted" group are independently halogen; ―(CH₂)_{0―4}R°; ―(CH₂)_{0―4}OR°; -O(CH₂)₀₋₄R°, ―O―(CH₂)_{0―4}C(O)OR°; ―(CH₂)_{0―4}CH(OR°)₂; ―(CH₂)_{0―4}SR°; ―(CH₂)_{0―4}Ph, which may be substituted with R°; ―(CH₂)_{0―4}O(CH₂)_{0―1}Ph which may be substituted with R°; - CH=CHPh, which may be substituted with R°; ―(CH₂)_{0―4}O(CH₂)_{0―1}-pyridyl which may be substituted with R°; ―NO₂; -CN; ―N₃; (CH₂)_{0―4}N(R°)₂; ―(CH₂)_{0―4}N(R°)C(O)R°; - N(R°)C(S)R°; ―(CH₂)_{0―4}N(R°)C(O)NR°₂; N(R°)C(S)NR°₂; ―(CH₂)_{0―4}N(R°)C(O)OR°; - N(R°)N(R°)C(O)R°; N(R°)N(R°)C(O)NR°₂; N(R°)N(R°)C(O)OR°; ―(CH₂)_{0―4}C(O)R°; C(S)R°; ―(CH₂)_{0―4}C(O)OR°; ―(CH₂)_{0―4}C(O)SR°; (CH₂)_{0―4}C(O)OSiR°₃; ―(CH₂)_{0― 4}OC(O)R°; ―OC(O)(CH₂)_{0―4}SR°; ―(CH₂)_{0―4}SC(O)R°; ―(CH₂)_{0―4}C(O)NR°₂; ―C(S)NR°₂; - C(S)SR°; ―SC(S)SR°, (CH₂)_{0―4}OC(O)NR°₂; C(O)N(ORO)R°; -C(O)C(O)R°; ― C(O)CH₂C(O)R°; -C(NOR°)R°; (CH₂)_{0―4}SSR°; ―(CH₂)_{0―4}S(O)₂R°; ―(CH₂)_{0―4}S(O)₂OR°; - (CH₂)_{0―4}OS(O)₂R°; ―S(O)₂NR°₂; (CH₂)_{0―4}S(O)R°; N(R°)S(O)₂NR°₂; ―N(R°)S(O)₂R°; - N(OR°)R°; ―C(NH)NR°₂; ―P(O)₂R°; P(O)R°₂; OP(O)R°₂; ―OP(O)(OR°)₂; SiR°₃; ―(C_{1―4} straight or branched alkylene)O-N(R°)₂; or ―(C_{1―4} straight or branched alkylene)C(O)O-N(R°)₂, wherein each R° may be substituted as defined below and is independently hydrogen, C_{1―6} aliphatic, ―CH₂Ph, ―O(CH₂)_{0―1}Ph, -CH₂-(5- to 6-membered heteroaryl ring), or a 3- to 6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3- to 12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, - (CH₂)_{0―2}R^{•}, ―(haloR^{•}), ―(CH₂)_{0―2}OH, ―(CH₂)_{0―2}OR^{•}, ―(CH₂)_{0―2}CH(OR^{•})₂, O(haloR^{•}), - CN, -N₃, ―(CH₂)_{0―2}C(O)R^{•}, ―(CH₂)_{0―2}C(O)OH, ―(CH₂)_{0―2}C(O)OR^{•}, ―(CH₂)_{0―2}SR^{•}, - (CH₂)_{0―2}SH, ―(CH₂)_{0―2}NH₂, ―(CH₂)_{0―2}NHR^{•}, ―(CH₂)_{0―2}NR^{•}₂, ―NO₂, ―SiR^{•}₃, ―OSiR^{•}₃, C(O)SR^{•}, ―(C_{1―4} straight or branched alkylene)C(O)OR^{•}, or ―SSR^{•} wherein each R^{•} is unsubstituted or where preceded by "halo" is substituted only with one or more halogens, and is independently selected from C_{1―4} aliphatic, -CH₂Ph, ―O(CH₂)_{0―1} Ph, or a 3- to 6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O ("oxo"), =S, =NNR*₂, =NNHC(O)R*, =NNHC(O)OR*, =NNHS(O)₂R*, =NR*, =NOR*, ―O(C(R*₂))_{2―3}O―, or ―S(C(R*₂))_{2―3}S―, wherein each independent occurrence of R* is selected from hydrogen, C_{1―6} aliphatic which may be substituted as defined below, or an unsubstituted 5- to 6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: ―O(CR*₂)_{2―3}O―, wherein each independent occurrence of R* is selected from hydrogen, C_{1―6} aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R* include halogen, ―R^{•}, (haloR^{•}), OH, - OR^{•}, ―O(haloR^{•}), -CN, -C(O)OH, ―C(O)OR^{•}, ―NH₂, ―NHR^{•}, ―NR^{•}₂, or ―NO₂, wherein each R^{•} is unsubstituted or where preceded by "halo" is substituted only with one or more halogens, and is independently C_{1―4} aliphatic, -CH₂Ph, ―O(CH₂)_{0―1}Ph, or a 5- to 6-membered saturated, partially unsaturated, or aryl ring having 0-4 heteroatoms independently selected from nitrogen, oxygen, or sulfur.

Suitable substituents on a substitutable nitrogen of an "optionally substituted" group include ―R^{†}, ―NR^{†}₂, ―C(O)R^{†}, ―C(O)OR^{†}, ―C(O)C(O)R^{†}, ―C(O)CH₂C(O)R^{†}, S(O)₂R^{†}, S(O)₂NR^{†}₂, ―C(S)NR^{†}₂, ―C(NH)NR^{†}₂, or ―N(R^{†})S(O)₂R^{†}; wherein each R^{†} is independently hydrogen, C_{1―6} aliphatic which may be substituted as defined below, unsubstituted -OPh, or an unsubstituted 3- to 6-membered saturated, partially unsaturated, or aryl ring having 0-4 heteroatoms independently selected from nitrogen, oxygen, or sulfur, or, notwithstanding the definition above, two independent occurrences of R^{†}, taken together with their intervening atom(s) form an unsubstituted 3- to 12-membered saturated, partially unsaturated, or aryl mono- or bicyclic ring having 0-4 heteroatoms independently selected from nitrogen, oxygen, or sulfur. Suitable substituents on the aliphatic group of R^{†} are independently halogen, ―R^{•}, (haloR^{•}), -OH, ―OR^{•}, ―O(haloR^{•}), -CN, -C(O)OH, ―C(O)OR^{•}, ―NH₂, ―NHR^{•}, ―NR^{•}₂, or NO₂, wherein each R^{•} is unsubstituted or where preceded by "halo" is substituted only with one or more halogens, and is independently C_{1―4} aliphatic, ―CH₂Ph, ― O(CH₂)_{0―1}Ph, or a 3- to 6-membered saturated, partially unsaturated, or aryl ring having 0-4 heteroatoms independently selected from nitrogen, oxygen, or sulfur.

***Small molecule:*** As used herein, the term "small molecule" means a low molecular weight organic and/or inorganic compound. In general, a "small molecule" is a molecule that is less than about 5 kilodaltons (kD) in size. In some embodiments, a small molecule is less than about 4 kD, 3 kD, about 2 kD, or about 1 kD. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, a small molecule is not a polymer.

Those of ordinary skill in the art, reading the present disclosure, will appreciate that certain small molecule compounds described herein, including, for example, oligosaccharide compounds described herein, may be provided and/or utilized in any of a variety of forms such as, for example, crystal forms (e.g., polymorphs, solvates, etc), salt forms, protected forms, pro-drug forms, ester forms, isomeric forms (e.g., optical and/or structural isomers), isotopic forms, etc.

Those of ordinary skill in the art will appreciate that certain small molecule compounds (e.g., oligosaccharide compounds described herein) have structures that can exist in one or more steroisomeric forms. In some embodiments, such a small molecule may be utilized in accordance with the present disclosure in the form of an individual enantiomer, diastereomer or geometric isomer, or may be in the form of a mixture of stereoisomers; in some embodiments, such a small molecule may be utilized in accordance with the present disclosure in a racemic mixture form.

Those of skill in the art will appreciate that certain small molecule compounds (e.g., oligosaccharide compounds described herein) have structures that can exist in one or more tautomeric forms. In some embodiments, such a small molecule may be utilized in accordance with the present disclosure in the form of an individual tautomer, or in a form that interconverts between tautomeric forms.

Those of skill in the art will appreciate that certain small molecule compounds (e.g., oligosaccharide compounds described herein) have structures that permit isotopic substitution (e.g., ²H or ³H for H; ¹¹C, ¹³C or ¹⁴C for ¹²C; ¹³N or ¹⁵N for ¹⁴N; ¹⁷O or ¹⁸O for ¹⁶O; ³⁶Cl for ³⁵Cl or ³⁷Cl; ¹⁸F for ¹⁹F; ¹³¹I for ¹²⁷I; etc.). In some embodiments, such a small molecule may be utilized in accordance with the present disclosure in one or more isotopically modified forms, or mixtures thereof.

In some embodiments, reference to a particular small molecule compound (e.g., oligosaccharide compounds described herein) may relate to a specific form of that compound. In some embodiments, a particular small molecule compound may be provided and/or utilized in a salt form (e.g., in an acid-addition or base-addition salt form, depending on the compound); in some such embodiments, the salt form may be a pharmaceutically acceptable salt form.

In some embodiments, where a small molecule compound is one that exists or is found in nature, that compound may be provided and/or utilized in accordance in the present disclosure in a form different from that in which it exists or is found in nature. Those of ordinary skill in the art will appreciate that, in some embodiments, a preparation of a particular small molecule compound (e.g., an oligosaccharide compound described herein) that contains an absolute or relative amount of the compound, or of a particular form thereof, that is different from the absolute or relative (with respect to another component of the preparation including, for example, another form of the compound) amount of the compound or form that is present in a reference preparation of interest (e.g., in a primary sample from a source of interest such as a biological or environmental source) is distinct from the compound as it exists in the reference preparation or source. Thus, in some embodiments, for example, a preparation of a single stereoisomer of a small molecule compound may be considered to be a different form of the compound than a racemic mixture of the compound; a particular salt of a small molecule compound may be considered to be a different form from another salt form of the compound; a preparation that contains only a form of the compound that contains one conformational isomer ((Z) or (E)) of a double bond may be considered to be a different form of the compound from one that contains the other conformational isomer ((E) or (Z)) of the double bond; a preparation in which one or more atoms is a different isotope than is present in a reference preparation may be considered to be a different form; etc.

Those skilled in the art will further appreciate that, in small molecule structures, the symbol 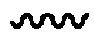 , as used herein, refers to a point of attachment between two atoms.

***Therapeutic agent:*** As used herein, the phrase "therapeutic agent" in general refers to any agent that elicits a desired pharmacological effect when administered to an organism. In some embodiments, an agent is considered to be a therapeutic agent if it demonstrates a statistically significant effect across an appropriate population. In some embodiments, the appropriate population may be a population of model organisms. In some embodiments, an appropriate population may be defined by various criteria, such as a certain age group, gender, genetic background, preexisting clinical conditions, etc. In some embodiments, a therapeutic agent is a substance that can be used to alleviate, ameliorate, relieve, inhibit, prevent, delay onset of, reduce severity of, and/or reduce incidence of one or more symptoms or features of a disease, disorder, and/or condition. In some embodiments, a "therapeutic agent" is an agent that has been or is required to be approved by a government agency before it can be marketed for administration to humans. In some embodiments, a "therapeutic agent" is an agent for which a medical prescription is required for administration to humans. ***Treat:*** As used herein, the terms "treat," "treatment," or "treating" refer to any method used to partially or completely alleviate, ameliorate, relieve, inhibit, prevent, delay onset of, reduce severity of, and/or reduce incidence of one or more symptoms or features of a disease, disorder, and/or condition. Treatment may be administered to a subject who does not exhibit signs of a disease, disorder, and/or condition. In some embodiments, treatment may be administered to a subject who exhibits only early signs of the disease, disorder, and/or condition, for example, for the purpose of decreasing the risk of developing pathology associated with the disease, disorder, and/or condition.

### Polycationic Oligosaccharide Compositions

The present disclosure, provides, among other things, compositions comprising an oligosaccharide of formula I: 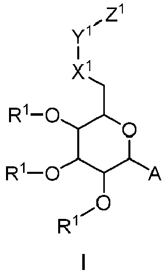 or a pharmaceutically acceptable salt thereof, wherein:
A is A¹, A², or A³: 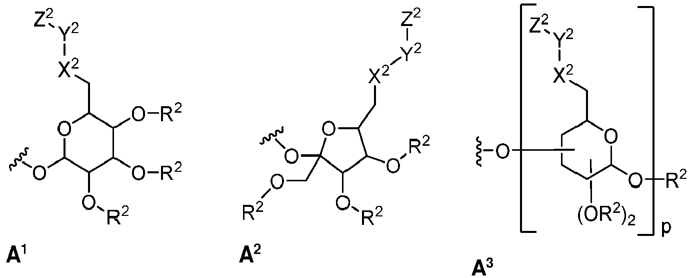
R¹ and R² are each independently selected from H, R^{a}, and -C(O)-R^{a};
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, and -NH-;
Y¹ and Y² are each independently an optionally substituted C₁₋₃₀ aliphatic group wherein one or more carbons are optionally and independently replaced by -Cy-, -NH-, -NHC(O)-, -C(O)NH-, -NHC(O)O-, -OC(O)NH-, -NHC(O)NH-, -NHC(S)NH-, -C(S)NH- - C(O)NHSO₂-, -SO₂NHC(O)-,-OC(O)O-, -O-, -C(O)-, -OC(O)-, -C(O)O-, -SO-, or -SO₂-;
each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, -N⁺(M)₃, 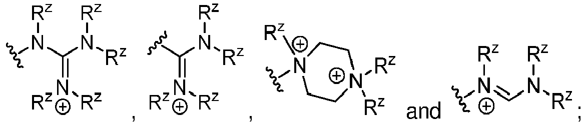
each M is independently ―C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5;
provided that when A is A¹, then:
   (i) each R¹ and R² is not ―CO-(CH₂)₄-CH₃, X¹ and X² are not both -S-, Y¹ and Y² are not both ―(CH₂)₂- and Z¹ and Z² are not both ―N⁺(H)₃,
   (ii) each R¹ and R² is not ―O-(CH₂)₅-CH₃ or ―O-(CH₂)₁₃-CH₃, X¹ and X² are not both -S-, Y¹ and Y² are not both ―(CH₂)₂-, and Z¹ and Z² are not both ―N⁺(H)₃,
   (iii) each R¹ and R² is not ―CO-(CH₂)₄-CH₃, X¹ and X² are not both -S-, Y¹ and Y² are not both ―(CH₂)₂-NH-C(S)-NH-(CH₂)₂-, and Z¹ and Z² are not both ―N⁺(H)₃, and
   (iv) Cy is not triazolyl.

As described herein, A is A¹, A², or A³:

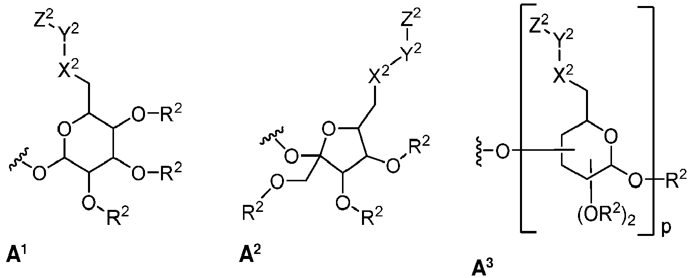

In some embodiments, A is A¹ or A². In some embodiments, A is A¹. In some embodiments, A is A². In some embodiments, A is A³. In some embodiments, A is A¹, and the oligosaccharide is of formula la: 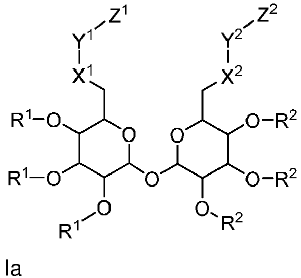 or a pharmaceutically acceptable salt thereof, wherein R¹, R², X¹, X², Y¹, Y², Z¹, and Z² are as described herein.

In some embodiments, A is A², and an oligosaccharide is of formula Ib: 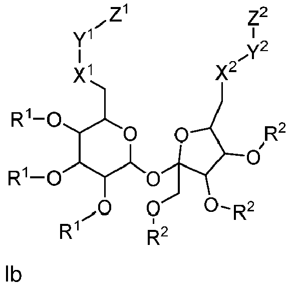 or a pharmaceutically acceptable salt thereof, wherein R¹, R², X¹, X², Y¹, Y², Z¹, and Z² are as described herein.

In some embodiments, A is A³, and an oligosaccharide is of formula Ic: 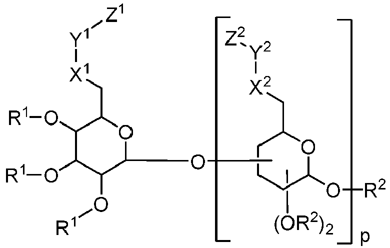 or a pharmaceutically acceptable salt thereof, , wherein p, R¹, R², X¹, X², Y¹, Y², Z¹, and Z² are as described herein. In some embodiments, p is 1, and an oligosaccharide is of formula Ic-i:

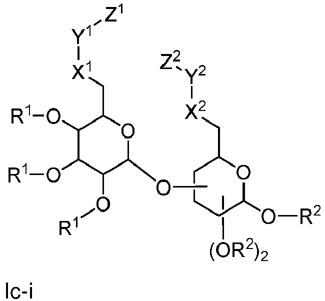

In some embodiments, p is 2, and an oligosaccharide is of formula Ic-ii:

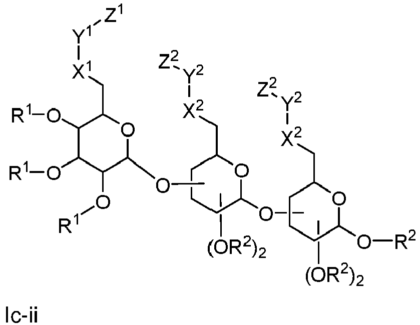

In some embodiments, p is 3, and an oligosaccharide is of formula Ic-iii:

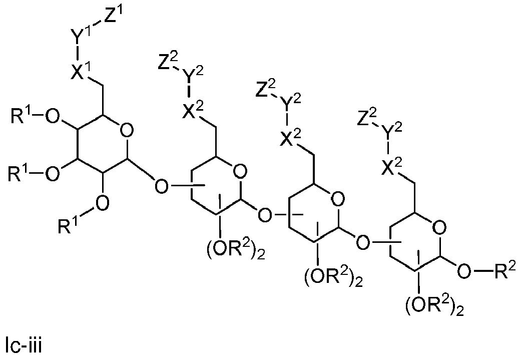

In some embodiments, p is 4, and an oligosaccharide is of formula Ic-iv:

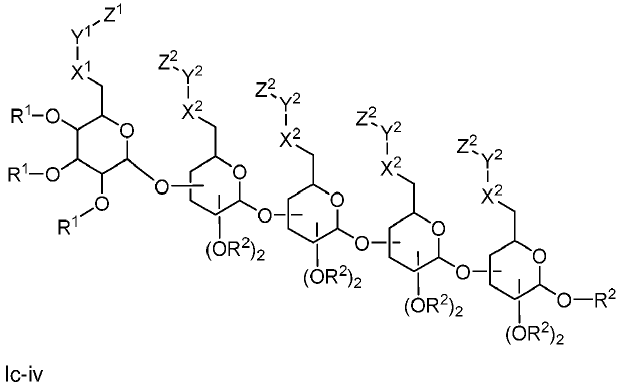

In some embodiments, p is 5, and an oligosaccharide is of formula Ic-v:

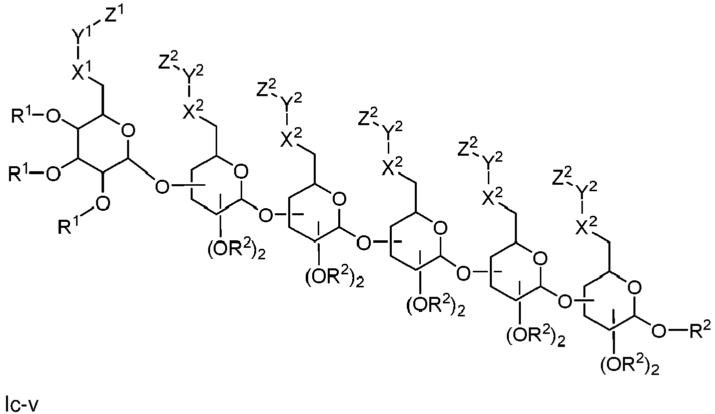

As described generally herein R¹ and R² are each independently selected from H, R^{a}, and -C(O)-R^{a}. In some embodiments, R¹ and R² are each independently selected from H, R^{a}, and -C(O)-R^{a}. In some embodiments, R¹ and R² are each independently selected from R^{a} and -C(O)-R^{a}. In some embodiments, each of R¹ and R² is R^{a}. In some embodiments, each of R¹ and R² is R^{a}, and each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b}.

In some embodiments, each of R¹ and R² is R^{a}, and R^{a} is C₁-C₂₀ aliphatic optionally substituted with one or more R^{b}. In some embodiments, each of R¹ and R² is R^{a}, and each R^{a} is C₁-C₁₄ aliphatic optionally substituted with one or more R^{b}. In some embodiments, each of R¹ and R² is R^{a}, and each R^{a} is C₁-C₁₀ aliphatic optionally substituted with one or more R^{b}. In some embodiments, each of R¹ and R² is R^{a}, and R^{a} is C₅-C₁₀ aliphatic optionally substituted with one or more R^{b}. In some embodiments, each of R¹ and R² is R^{a}, and R^{a} is C₅-C₁₀ alkyl optionally substituted with one or more R^{b}. In some embodiments, each of R¹ and R² is R^{a}, and R^{a} is C₅-C₁₀ linear alkyl. In some embodiments, each of R¹ and R² is R^{a}, and R^{a} is methyl, ethyl, n-propyl, n-butyl, n-pentyl, n-hexyl, n-heptyl, n-octyl, or n-nonyl. In some embodiments, each of R¹ and R² is R^{a}, and each R^{a} is independently selected from 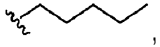 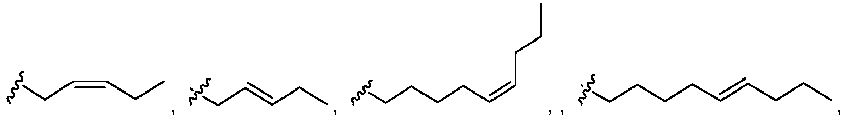 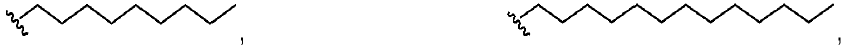 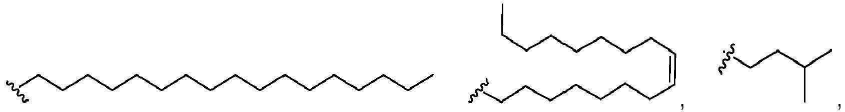 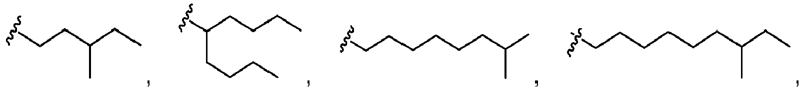 or

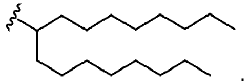

In some embodiments, each of R¹ and R² is R^{a}, and each R^{a} is C₃-C₂₀ cycloaliphatic, optionally substituted with one or more R^{b}. In some embodiments, each R¹ of and R² is R^{a}, and each R^{a} is C₃-C₆ cycloaliphatic optionally substituted with one or more R^{b}. In some embodiments, each of R¹ and R² is R^{a}, and R^{a} is cyclopropyl, cyclobutyl, cyclopentyl, or cyclohexyl.

In some embodiments, each of R¹ and R² is R^{a}, and R^{a} is C₅-C₆ aryl optionally substituted with one or more R^{b}. In some embodiments, each of R¹ and R² is R^{a}, and R^{a} is phenyl.

In some embodiments, each of R¹ and R² is R^{a}, and R^{a} is 3- to 12-membered heterocyclyl comprising 1 to 3 heteroatoms selected from N, O, and S optionally substituted with one or more R^{b}. In some embodiments, each of R¹ and R² is R^{a}, and R^{a} is 3- to 6-membered heterocyclyl comprising 1 to 3 heteroatoms selected from N, O, and S optionally substituted with one or more R^{b}.

In some embodiments, each of R¹ and R² is ―C(O)-R^{a}, and each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b}.

In some embodiments, each of R¹ and R² is -C(O)-R^{a}. In some embodiments, each of R¹ and R² is -C(O)-R^{a}, and R^{a} is C₁-C₂₀ aliphatic optionally substituted with one or more R^{b}. In some embodiments, each of R¹ and R² is -C(O)-R^{a}, and R^{a} is C₁-C₁₄ aliphatic optionally substituted with one or more R^{b}. In some embodiments, each of R¹ and R² is -C(O)-R^{a}, and R^{a} is C₁-C₁₀ aliphatic optionally substituted with one or more R^{b}. In some embodiments, each of R¹ and R² is -C(O)-R^{a}, and R^{a} is C₅-C₁₀ aliphatic optionally substituted with one or more R^{b}. In some embodiments, each of R¹ and R² is -C(O)-R^{a}, and R^{a} is C₅-C₁₀ alkyl optionally substituted with one or more R^{b}. In some embodiments, each of R¹ and R² is -C(O)-R^{a}, and R^{a} is C₅-C₁₀ linear alkyl. In some embodiments, each of R¹ and R² is -C(O)-R^{a}, and R^{a} is methyl, ethyl, n-propyl, n-butyl, n-pentyl, n-hexyl, n-heptyl, n-octyl, or n-nonyl. In some embodiments, each of R¹ and R² is -C(O)-R^{a}, and each R^{a} is independently selected from 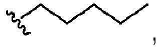 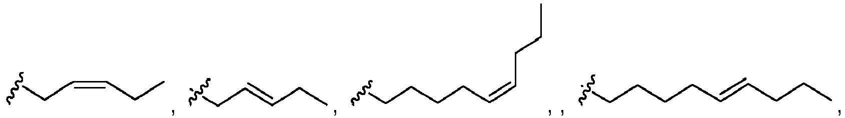 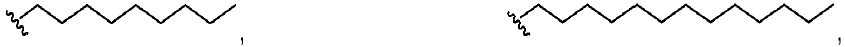 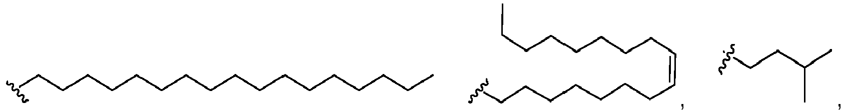 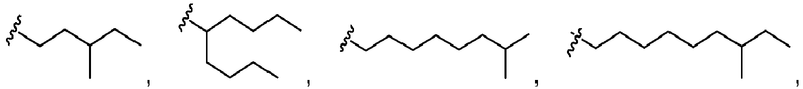 or

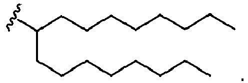

In some embodiments, each of R¹ and R² is -C(O)-R^{a}, and R^{a} is C₃-C₂₀ cycloaliphatic, optionally substituted with one or more R^{b}. In some embodiments, each of R¹ and R² is -C(O)-R^{a}, and R^{a} is C₃-C₆ cycloaliphatic optionally substituted with one or more R^{b}. In some embodiments, each of R¹ and R² is -C(O)-R^{a}, and each R^{a} is independently selected from cyclopropyl, cyclobutyl, cyclopentyl, or cyclohexyl.

In some embodiments, each of R¹ and R² is -C(O)-R^{a}, and R^{a} is C₅-C₆ aryl optionally substituted with one or more R^{b}. In some embodiments, each of R¹ and R² is -C(O)-R^{a}, and R^{a} is phenyl.

In some embodiments, each of R¹ and R² is -C(O)-R^{a}, and R^{a} is 3- to 12-membered heterocyclyl comprising 1 to 3 heteroatoms selected from N, O, and S optionally substituted with one or more R^{b}. In some embodiments, each of R¹ and R² is -C(O)-R^{a}, and R^{a} is 3- to 6-membered heterocyclyl comprising 1 to 3 heteroatoms selected from N, O, and S optionally substituted with one or more R^{b}.

In some embodiments, each of R¹ and R² is independently selected from:

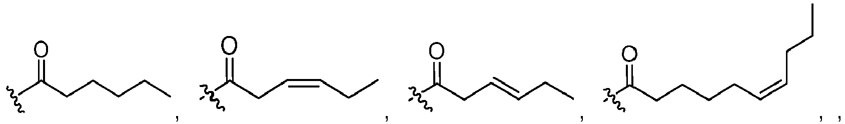

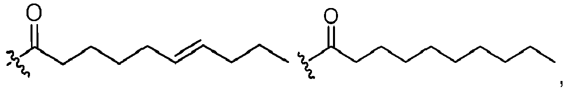

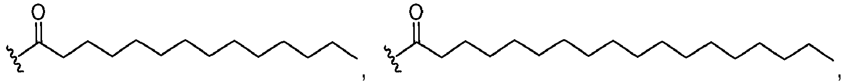

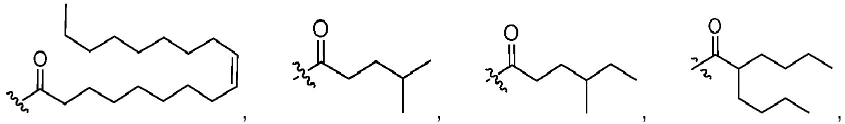

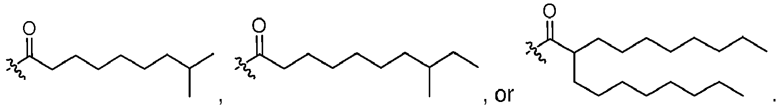

In some embodiments, each of R¹ and R² is

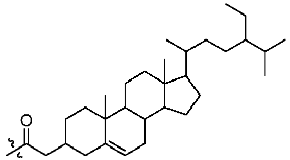

As described herein, each R^{b} is independently selected from halogen, -N₃, -R^{c}, -OR^{c}, - SR^{c}, -NHR^{c}, -C(O)-R^{c}, -OC(O)R^{c}, -NHC(O)R^{c}, -C(O)NHR^{c}, and -NHC(O)NHR^{c}. In some embodiments, R^{b} is halogen. In some embodiments, R^{b} is ―N₃. In some embodiments, R^{b} is R^{c}. In some embodiments, R^{b} is -OR^{c}. In some embodiments, R^{b} is -SR^{c}. In some embodiments, R^{b} is -NHR^{c}. In some embodiments, R^{b} is -C(O)-R^{c}. In some embodiments, R^{b} is -OC(O)R^{c}. In some embodiments, R^{b} is -NHC(O)R^{c}. In some embodiments, R^{b} is -C(O)NHR^{c}. In some embodiments, R^{b} is -NHC(O)NHR^{c}. As described herein, each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S. In some embodiments, R^{c} is optionally substituted C₁-C₂₀ aliphatic. In some embodiments, R^{c} is optionally substituted C₃-C₂₀ cycloaliphatic. In some embodiments, R^{c} is optionally substituted C₅-C₆ aryl. In some embodiments, R^{c} is optionally substituted 3- to 12-membered heterocyclyl comprising 1 to 3 heteroatoms selected from N, O, and S. In some embodiments, R^{c} is optionally substituted 4- to 12-membered heteroaryl comprising 1 to 3 heteroatoms selected from N, O, and S.

As described herein, X¹ and X² are each independently selected from -S- and -NH-. In some embodiments, X¹ is -S-. In some embodiments, X¹ is -NH-. In some embodiments, X² is -S-. In some embodiments, X² is -NH-. In some embodiments, X¹ and X² are each -S-. In some embodiments, X¹ and X² are each -NH-. In some embodiments, X¹ is -S- and X² is -NH-. In some embodiments, X¹ is -NH- and X² is - S-.

As described herein, Y¹ and Y² are each independently an optionally substituted C₁₃₀ aliphatic group wherein one or more carbons of a Y¹ and/or Y² group are optionally and independently replaced by -Cy-, -NH-, -NHC(O)-, -C(O)NH-, -NHC(O)O-, -OC(O)NH-, - NHC(O)NH-, -NHC(S)NH-, -C(S)NH- -C(O)NHSO₂-, -SO₂NHC(O)-,-OC(O)O-, -O-, - C(O)-, -OC(O)-, -C(O)O, SO, or SO₂. As described herein, it is understood that Y¹ and Y² are bivalent moieties, having one end bonded to a X¹ or X² group, and the other end bonded to a Z¹ or Z² group.

In some embodiments, Y¹ and Y² are each independently selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-NHC(O)-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, Y¹ and Y² are each C₁-C₁₀ aliphatic. In some embodiments, Y¹ and Y² are each methylene, ethylene, propylene, or butylene. In some embodiments, Y¹ and Y² are each ―CH₂-CH₂-.

In some embodiments, Y¹ and Y² are each C₀-C₄-aliphatic-NHC(O)NH-C₀-C₄ aliphatic. In some embodiments, Y¹ and Y² are each C₁-C₄-aliphatic-NHC(O)NH-C₁-C₄ aliphatic. In some embodiments, Y¹ and Y² are each ―CH₂-CH₂-NHC(O)NH-CH₂-CH₂-.

In some embodiments, Y¹ and Y² are each C₀-C₄-aliphatic-NHC(S)NH-C₀-C₄ aliphatic. In some embodiments, Y¹ and Y² are each C₁-C₄-aliphatic-NHC(S)NH-C₁-C₄ aliphatic. In some embodiments, Y¹ and Y² are each ―CH₂-CH₂-NHC(S)NH-CH₂-CH₂-.

In some embodiments, Y¹ and Y² are each C₀-C₄-aliphatic-C(O)NH-C₀-C₄ aliphatic. In some embodiments, Y¹ and Y² are each C₁-C₄-aliphatic-C(O)NH-C₁-C₄ aliphatic. In some embodiments, Y¹ and Y² are each ―CH₂-CH₂-C(O)NH-CH₂-CH₂-.

In some embodiments, Y¹ and Y² are each C₀-C₄-aliphatic-NHC(O)-C₀-C₄ aliphatic. In some embodiments, Y¹ and Y² are each C₁-C₄-aliphatic-NHC(O)-C₁-C₄ aliphatic. In some embodiments, Y¹ and Y² are each ―CH₂-CH₂-NHC(O)-CH₂-CH₂-.

In some embodiments, Y¹ and Y² are each C₀-C₄-aliphatic-NHSO₂-C₀-C₄ aliphatic. In some embodiments, Y¹ and Y² are each C₁-C₄-aliphatic-NHSO₂-C₁-C₄ aliphatic. In some embodiments, Y¹ and Y² are each ―CH₂-CH₂-NHSO₂-CH₂-CH₂-.

In some embodiments, Y¹ is selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-C(O)NH-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, embodiments, Y¹ is C₁-C₁₀ aliphatic. In some embodiments, Y¹ is methylene, ethylene, propylene, or butylene. In some embodiments, Y¹ is ―CH₂-CH₂-.

In some embodiments, Y¹ is C₀-C₄-aliphatic-NHC(O)NH-C₀-C₄ aliphatic. In some embodiments, Y¹ is C₁-C₄-aliphatic-NHC(O)NH-C₁-C₄ aliphatic. In some embodiments, Y¹ is ―CH₂-CH₂-NHC(O)NH-CH₂-CH₂-.

In some embodiments, Y¹ is C₀-C₄-aliphatic-NHC(S)NH-C₀-C₄ aliphatic. In some embodiments, Y¹ is C₁-C₄-aliphatic-NHC(S)NH-C₁-C₄ aliphatic. In some embodiments, Y¹ is ―CH₂-CH₂-NHC(S)NH-CH₂-CH₂-.

In some embodiments, Y¹ is C₀-C₄-aliphatic-C(O)NH-C₀-C₄ aliphatic. In some embodiments, Y¹ is C₁-C₄-aliphatic-C(O)NH-C₁-C₄ aliphatic. In some embodiments, Y¹ is ―CH₂-CH₂-C(O)NH-CH₂-CH₂-.

In some embodiments, Y¹ is C₀-C₄-aliphatic-NHC(O)-C₀-C₄ aliphatic. In some embodiments, Y¹ is C₁-C₄-aliphatic-NHC(O)-C₁-C₄ aliphatic. In some embodiments, Y¹ is ―CH₂-CH₂-NHC(O)-CH₂-CH₂-.

In some embodiments, Y¹ is C₀-C₄-aliphatic-NHSO₂-C₀-C₄ aliphatic. In some embodiments, Y¹ is C₁-C₄-aliphatic-NHSO₂-C₁-C₄ aliphatic. In some embodiments, Y¹ is ―CH₂-CH₂-NHSO₂-CH₂-CH₂-.

In some embodiments, Y² is selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-C(O)NH-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, embodiments, Y² is C₁-C₁₀ aliphatic. In some embodiments, Y² is methylene, ethylene, propylene, or butylene. In some embodiments, Y² is ―CH₂-CH₂-.

In some embodiments, Y² is C₀-C₄-aliphatic-NHC(O)NH-C₀-C₄ aliphatic. In some embodiments, Y² is C₁-C₄-aliphatic-NHC(O)NH-C₁-C₄ aliphatic. In some embodiments, Y² is ―CH₂-CH₂-NHC(O)NH-CH₂-CH₂-.

In some embodiments, Y² is C₀-C₄-aliphatic-NHC(S)NH-C₀-C₄ aliphatic. In some embodiments, Y² is C₁-C₄-aliphatic-NHC(S)NH-C₁-C₄ aliphatic. In some embodiments, Y² is ―CH₂-CH₂-NHC(S)NH-CH₂-CH₂-.

In some embodiments, Y² is C₀-C₄-aliphatic-C(O)NH-C₀-C₄ aliphatic. In some embodiments, Y² is C₁-C₄-aliphatic-C(O)NH-C₁-C₄ aliphatic. In some embodiments, Y² is ―CH₂-CH₂-C(O)NH-CH₂-CH₂-.

In some embodiments, Y² is C₀-C₄-aliphatic-NHC(O)-C₀-C₄ aliphatic. In some embodiments, Y² is C₁-C₄-aliphatic-NHC(O)-C₁-C₄ aliphatic. In some embodiments, Y² is ―CH₂-CH₂-NHC(O)-CH₂-CH₂-.

In some embodiments, Y² is C₀-C₄-aliphatic-NHSO₂-C₀-C₄ aliphatic. In some embodiments, Y² is C₁-C₄-aliphatic-NHSO₂-C₁-C₄ aliphatic. In some embodiments, Y² is ―CH₂-CH₂-NHSO₂-CH₂-CH₂-.

As described general herein, each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S.

In some embodiments, a moiety X¹-Y¹-Z¹ is:

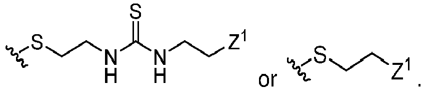

In some embodiments, a moiety X²-Y²-Z² is:

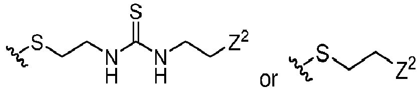

As described generally herein, Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃,

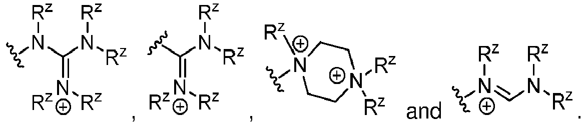

In some embodiments, Z¹ and Z² are each an optionally substituted 5- to 14-membered heterocyclyl ring having 1-3 heteroatoms selected from N, O, and S. In some embodiments, Z¹ and Z² are each an optionally substituted 5- to 6-membered heterocyclyl ring having 1-3 heteroatoms selected from N, O, and S. In some embodiments, Z¹ and Z² are each an optionally substituted 6-membered heterocyclyl ring having 1-3 heteroatoms selected from N, O, and S. In some embodiments, Z¹ and Z² are each selected from optionally substituted piperdinyl, piperazinyl, and morpholinyl.

In some embodiments, Z¹ and Z² are each -N⁺(M)₃. In some embodiments, Z¹ and Z² are each ―N⁺(C₀-C₆ aliphatic-R^{z})₃. In some embodiments, Z¹ and Z² are each ―N⁺(R^{z})₃. In some embodiments, Z¹ and Z² are each ―N⁺H₃. In some embodiments, Z¹ and Z² are each ―N⁺H(R^{z})₂. In some embodiments, Z¹ and Z² are each -N⁺H(C₁-C₆ aliphatic)₂. In some embodiments, Z¹ and Z² are each ―N⁺(H)₂CH₃. In some embodiments, Z¹ and Z² are each ―N⁺H(CH₃)₂. In some embodiments, Z¹ and Z² are each ―N⁺(CH₃)₃.

In some embodiments, Z¹ and Z² are each ―N⁺(R^{z})₂-C₁-C₆ aliphatic-N⁺(R^{z})₃. In some embodiments, Z¹ and Z² are each ―N⁺(R^{z})(C₁-C₆ aliphatic-N⁺(R^{z})₃)₂. In some embodiments, Z¹ and Z² are each:

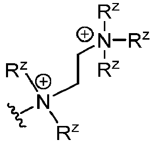

In some embodiments, Z¹ and Z² are each:

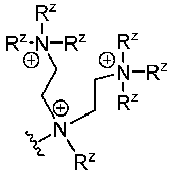

In some embodiments, Z¹ and Z² are each

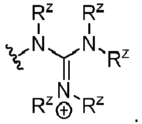

In some embodiments, Z¹ and Z² are each

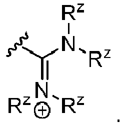

In some embodiments, Z¹ and Z² are each

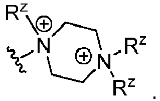

In some embodiments, Z¹ and Z² are each

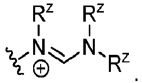

In some embodiments, Z¹ is optionally substituted 5- to 14-membered heterocyclyl ring having 1-3 heteroatoms selected from N, O, and S. In some embodiments, Z¹ is each an optionally substituted 5- to 6-membered heterocyclyl ring having 1-3 heteroatoms selected from N, O, and S. In some embodiments, Z¹ is an optionally substituted 6-membered heterocyclyl ring having 1-3 heteroatoms selected from N, O, and S. In some embodiments, Z¹ is selected from optionally substituted piperdinyl, piperazinyl, and morpholinyl.

In some embodiments, Z¹ is -N⁺(M)₃. In some embodiments, Z¹ is―N⁺(C₀-C₆ aliphatic-R^{z})₃. In some embodiments, Z¹ is―N⁺(R^{z})₃. In some embodiments, Z¹ is ―N⁺H₃. In some embodiments, Z¹ is ―N⁺H(R^{z})₂. In some embodiments, Z¹ is ―N⁺H(C₁-C₆ aliphatic)₂. In some embodiments, Z¹ is ―N⁺(H)₂CH₃. In some embodiments, Z¹ is - N⁺H(CH₃)₂. In some embodiments, Z¹ is ―N⁺(CH₃)₃.

In some embodiments, Z¹ is ―N⁺(R^{z})₂-C₁-C₆ aliphatic-N⁺(R^{z})₃. In some embodiments, Z¹ is ―N⁺(R^{z})(C₁-C₆ aliphatic-N⁺(R^{z})₃)₂. In some embodiments, Z¹ is:

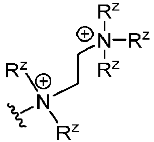

In some embodiments, Z¹ is:

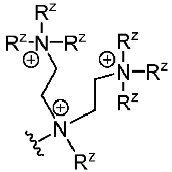

In some embodiments, Z¹ is:

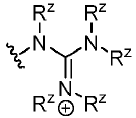

In some embodiments, Z¹ is:

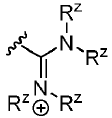

In some embodiments, Z¹ is

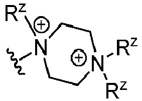

In some embodiments, Z¹ is

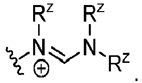

In some embodiments, Z² is optionally substituted 5- to 14-membered heterocyclyl ring having 1-3 heteroatoms selected from N, O, and S. In some embodiments, Z² is each an optionally substituted 5- to 6-membered heterocyclyl ring having 1-3 heteroatoms selected from N, O, and S. In some embodiments, Z² is an optionally substituted 6-membered heterocyclyl ring having 1-3 heteroatoms selected from N, O, and S. In some embodiments, Z² is selected from optionally substituted piperdinyl, piperazinyl, and morpholinyl.

In some embodiments, Z² is -N⁺(M)₃. In some embodiments, Z² is-N⁺(C₀-C₆ aliphatic-R^{z})₃. In some embodiments, Z² is―N⁺(R^{z})₃. In some embodiments, Z² is ―N⁺H₃. In some embodiments, Z² is ―N⁺H(R^{z})₂. In some embodiments, Z² is ―N⁺H(C₁-C₆ aliphatic)₂. In some embodiments, Z² is ―N⁺(H)₂CH₃. In some embodiments, Z² is - N⁺H(CH₃)₂. In some embodiments, Z² is ―N⁺(CH₃)₃.

In some embodiments, Z² is -N⁺(R^{z})₂-C₁-C₆ aliphatic-N⁺(R^{z})₃. In some embodiments, Z² is -N⁺(R^{z})(C₁-C₆ aliphatic-N⁺(R^{z})₃)₂. In some embodiments, Z² is:

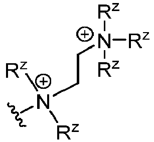

In some embodiments, Z² is:

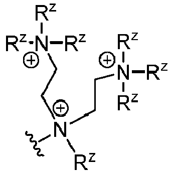

In some embodiments, Z² is:

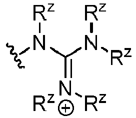

In some embodiments, Z² is:

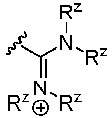

In some embodiments, Z² is

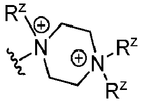

In some embodiments, Z² is

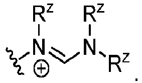

As described generally herein, each M is independently -C₀-C₆ aliphatic-R^{z} or -C₀-C₆ aliphatic-N⁺(R^{z})₃. In some embodiments, each M is C₀-C₆ aliphatic-R^{z}. In some embodiments, each M is -C₀-C₆ aliphatic-N⁺(R^{z})₃.

As described generally herein, each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4-to 12-membered heteroaryl comprising 1 to 3 heteroatoms selected from N, O, and S; or two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S.

In some embodiments, each R^{z} is H. In some embodiments, each R^{z} is optionally substituted C₁-C₆ aliphatic. In some embodiments, each R^{z} is optionally substituted C₃-C₂₀ cycloaliphatic. In some embodiments, each R^{z} is optionally substituted C₅-C₆ aryl. In some embodiments, each R^{z} is optionally substituted 3- to 12-membered heterocyclyl comprising 1 to 3 heteroatoms selected from N, O, and S. In some embodiments, each R^{z} is optionally substituted 4- to 12-membered heteroaryl comprising 1 to 3 heteroatoms selected from N, O, and S.

In some embodiments, two or more R^{z} can come together with the atoms to which they are attached to form an optionally substituted 3- to 12-membered heterocyclyl comprising 1 to 3 heteroatoms selected from N, O, and S. In some embodiments, two or more R^{z} can come together with the atoms to which they are attached to form an optionally substituted 4- to 12-membered heteroaryl comprising 1 to 3 heteroatoms selected from N, O, and S.

In some embodiments, an R^{z} on a Z¹ moiety and an R^{z} on a Z² moiety can come together to form a 4- to 6-membered heterocyclic ring having 1 to 3 heteroatoms selected from N, O, and S.

As described herein, a Z¹ and Z² moiety, which is a cationic or ionizable moiety described herein, further comprises one or more suitable counterions (e.g., an anion for each cationic or ionizable moiety). For example, in some embodiments, a Z¹ and Z² moiety comprises a counterion that is a halide, hydroxide, carboxylate, sulfate, phosphate, nitrate, alkyl having from 1 to 6 carbon atoms, sulfonate and aryl sulfonate. In some embodiments, a Z¹ and Z² moiety comprises a counterion that is acetate (CH₃COO⁻), chloride (Cl⁻), iodide (I⁻), bromide (Br⁻), or fluoride (F-).

In some embodiments, Z¹ and Z² are each independently selected from:

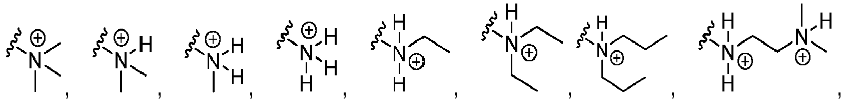

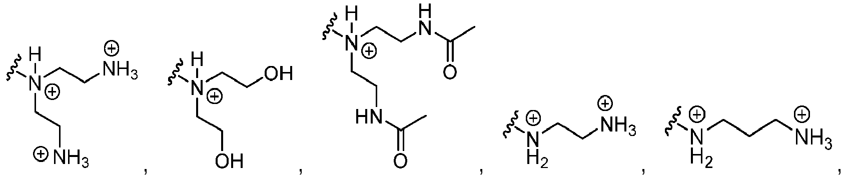

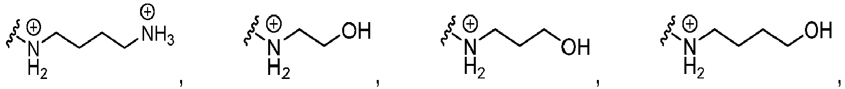

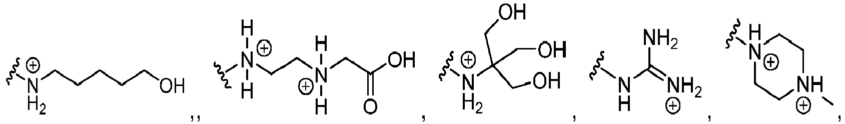

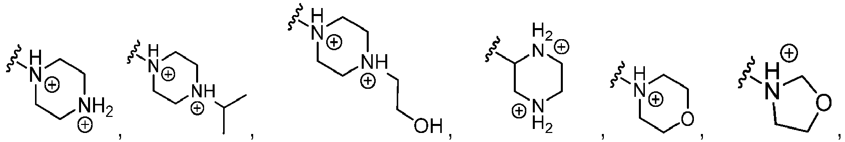

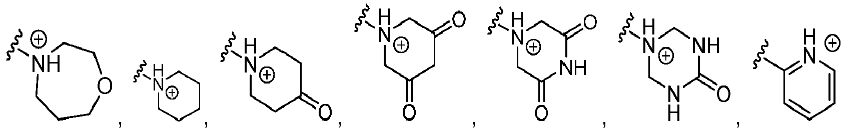

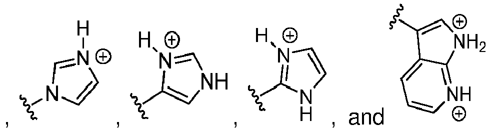

As described herein, with regard to formula I, when A is A¹, then (i) each R¹ and R² is not ―CO-(CH₂)₄-CH₃, X¹ and X² are not both ―S-, Y¹ and Y² are not both ―(CH₂)₂- and Z¹ and Z² are not both ―N⁺(H)₃; (ii) each R¹ and R² is not ―O-(CH₂)₅-CH₃ or ―O-(CH₂)₁₃-CH₃, X¹ and X² are not both ―S-, Y¹ and Y² are not both ―(CH₂)₂-, and Z¹ and Z² are not both ―N⁺(H)₃; (iii) each R¹ and R² is not ―CO-(CH₂)₄-CH₃, X¹ and X² are not both -S-, Y¹ and Y² are not both ―(CH₂)₂-NH-C(S)-NH-(CH₂)₂-, and Z¹ and Z² are not both - N⁺(H)₃; and (iv) Cy is not triazolyl.

In some embodiments, a compound of formula I is a compound of formula Id: 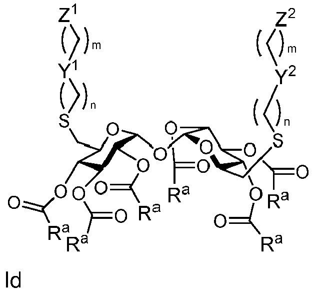 or a pharmaceutically acceptable salt thereof, wherein n and m are each independently selected from 0, 1, 2, 3, 4, 5, or 6, and R^{a}, Y¹, Y², Z¹, and Z² are as described herein.

In some embodiments, a compound of formula Id is a compound of formula Id-i: 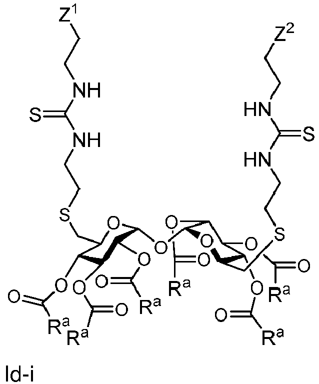 or a pharmaceutically acceptable salt thereof, wherein R^{a}, Z¹, and Z² are as described herein.

In some embodiments, the present disclosure provides a compound of Table A.

**Table A**

| **Name** | **Structure** |
|---|---|
| JLF19 | 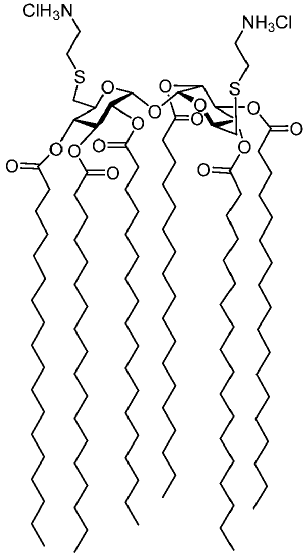 |
| JLF20 | 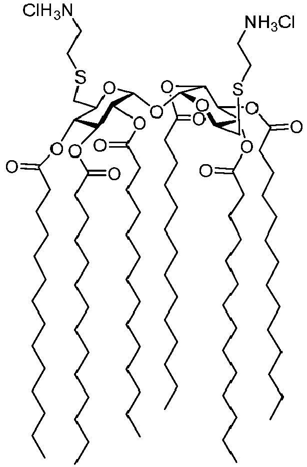 |
| JLF23 | 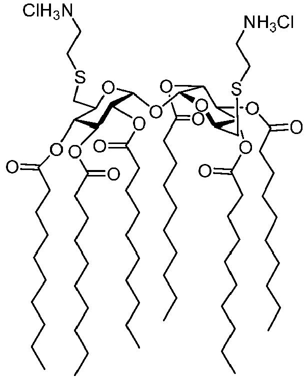 |
| JLF25 | 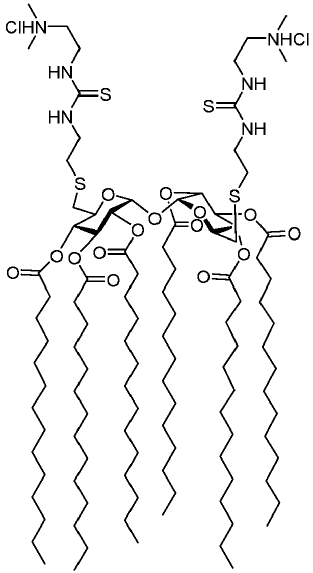 |
| JLF26 | 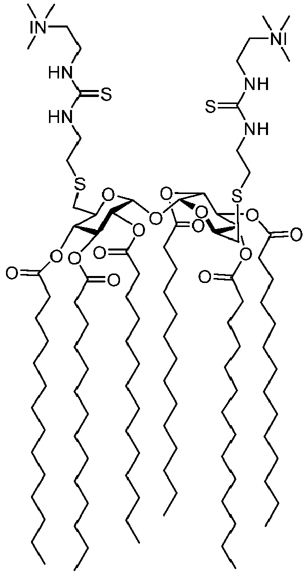 |
| JLF29 | 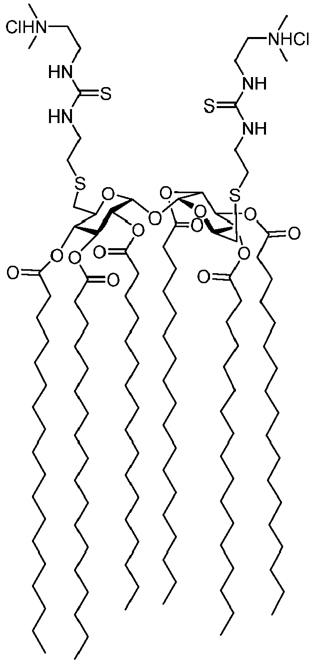 |
| JLF30 | 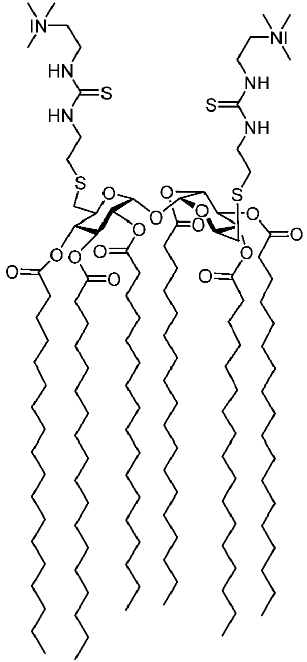 |
| JLF31 | 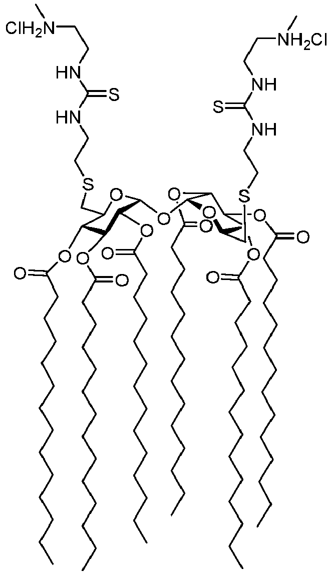 |
| JLF32 | 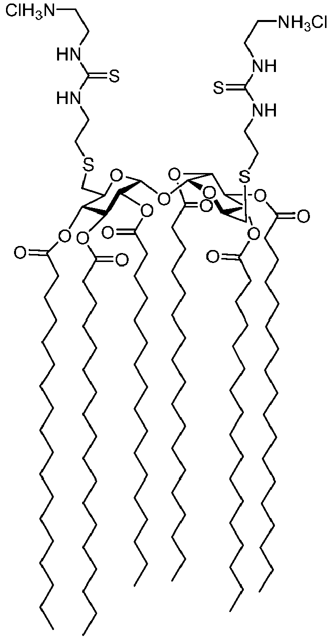 |
| JLF33 | 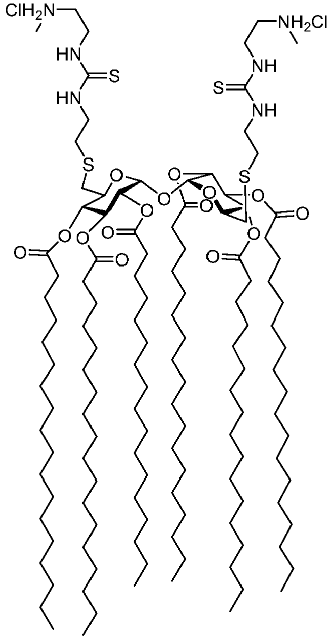 |
| JLF35 | 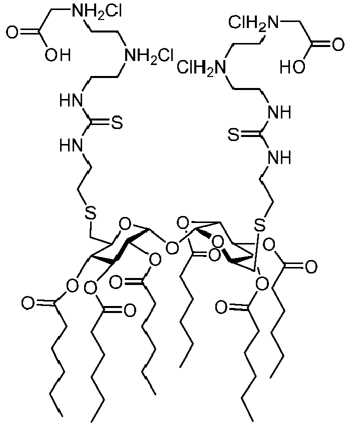 |
| JLF40 | 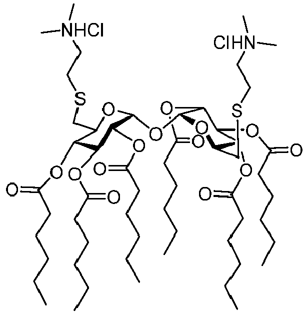 |
| JLF41 | 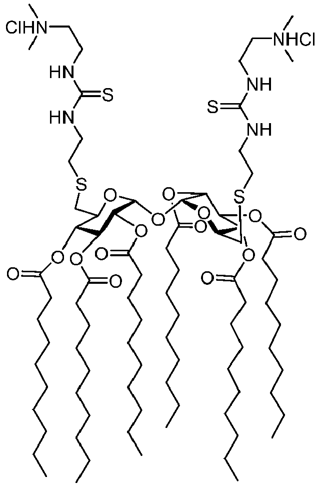 |
| JLF42 | 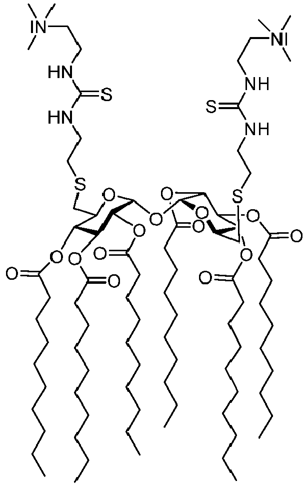 |
| JLF43 | 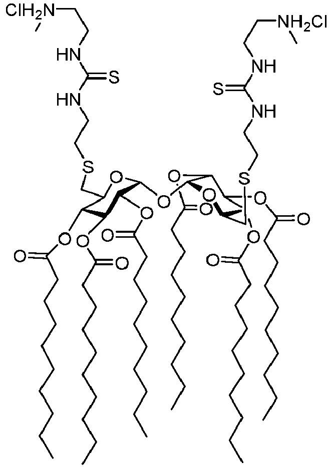 |
| JLF46 | 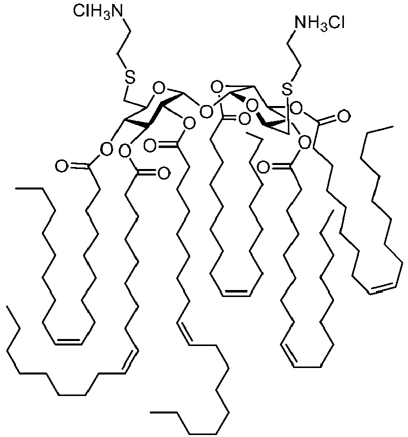 |
| JLF48 | 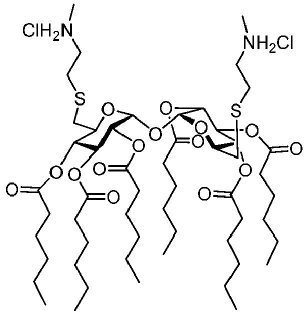 |
| JLF50 | 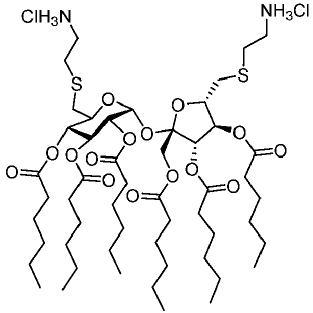 |
| JLF54 | 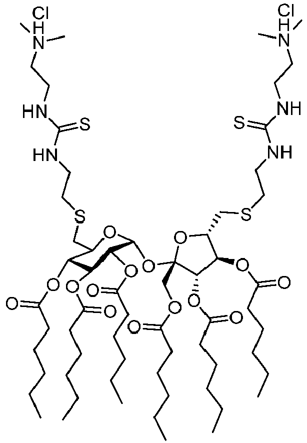 |
| JLF55 | 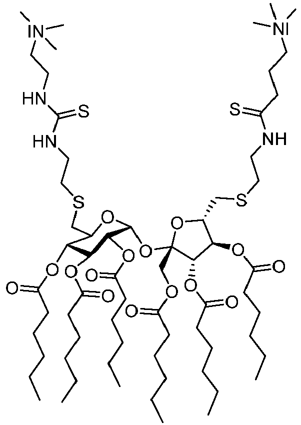 |
| JLF58 | 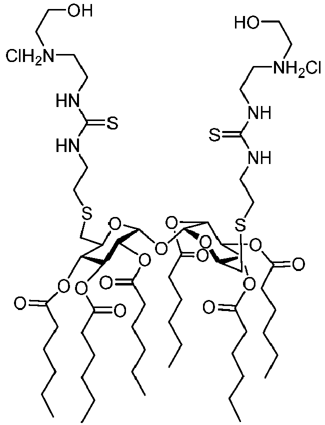 |
| JLF59 | 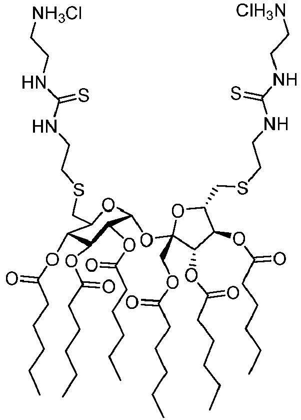 |
| JLF60 | 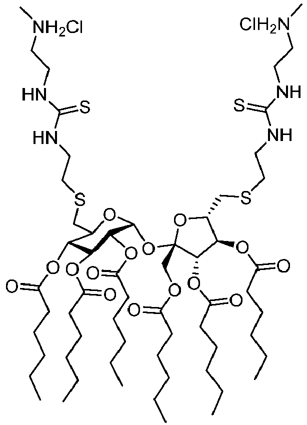 |
| JLF62 | 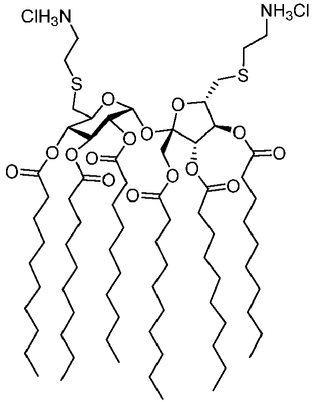 |
| JLF66 | 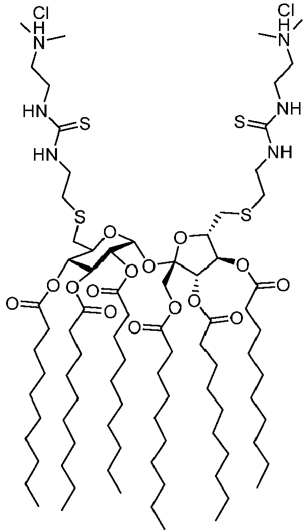 |
| JLF67 | 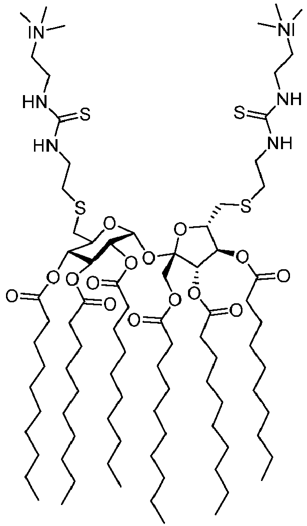 |
| JLF68 | 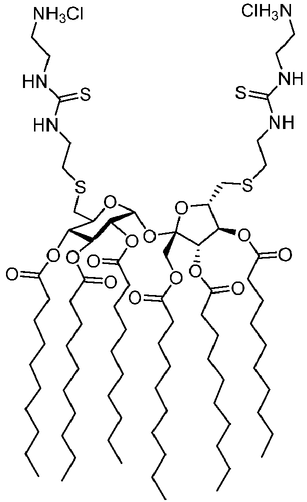 |
| JLF69 | 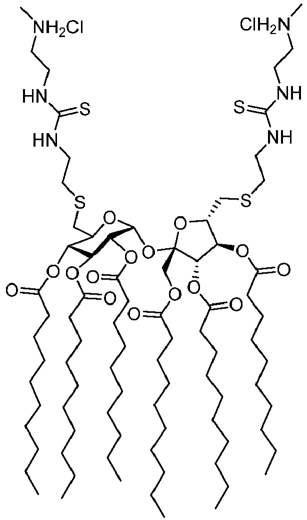 |
| JLF76 | 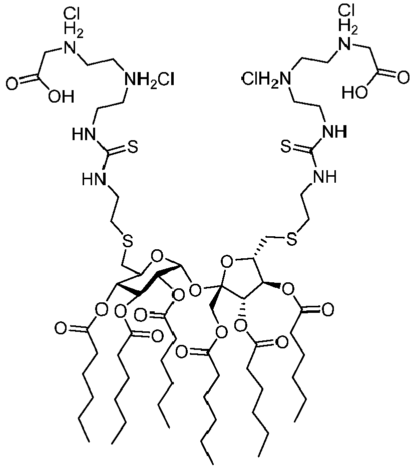 |
| JLF77 | 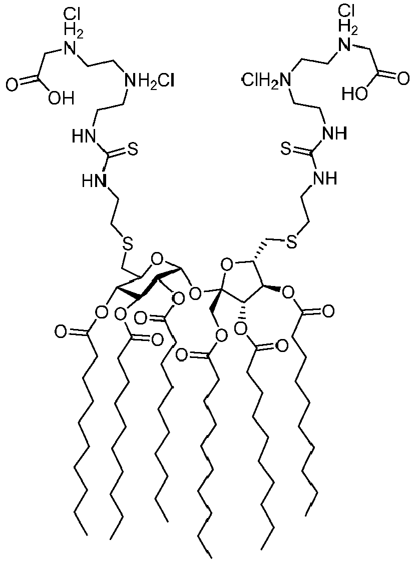 |
| JLF78 | 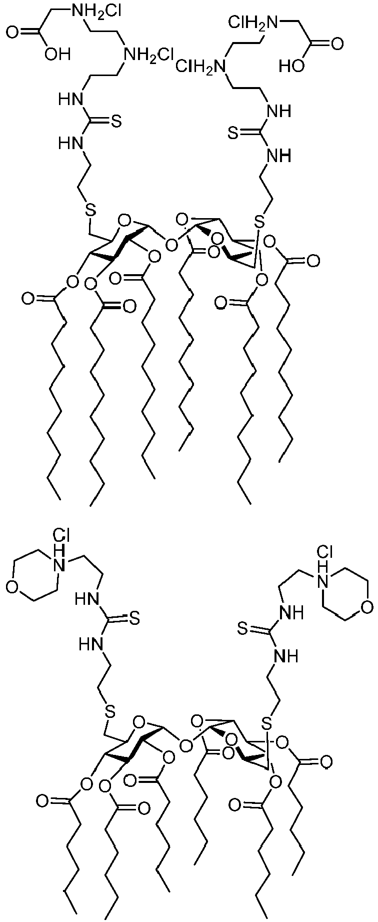 |
| JLF81 | 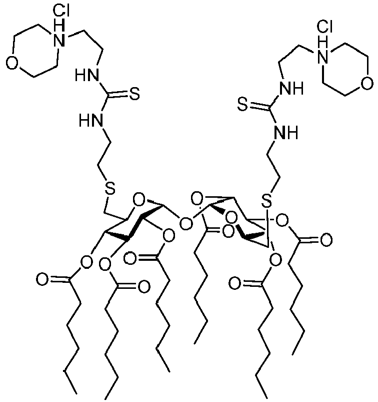 |
| JLF82 | 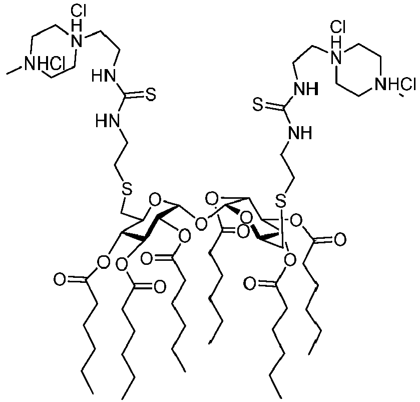 |
| JLF87 | 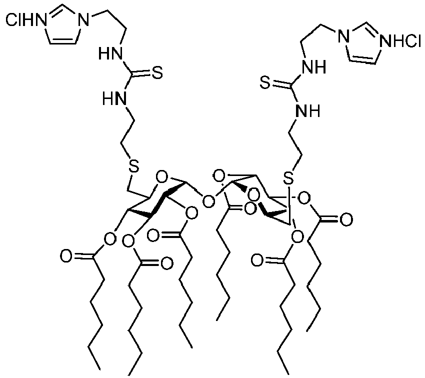 |
| JLF90 | 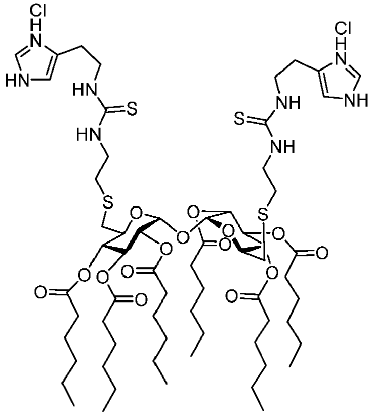 |
| JLF93 | 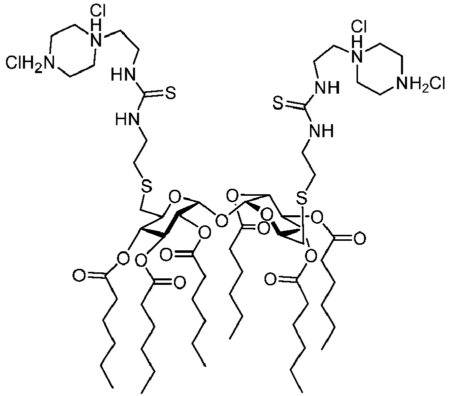 |
| JLF94 | 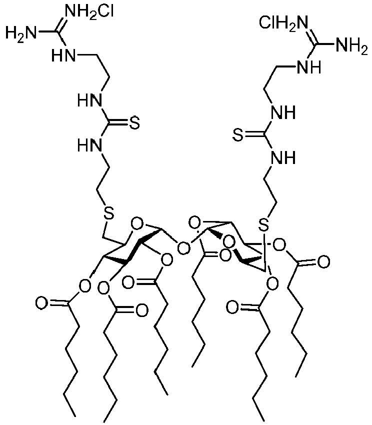 |
| JLF95 | 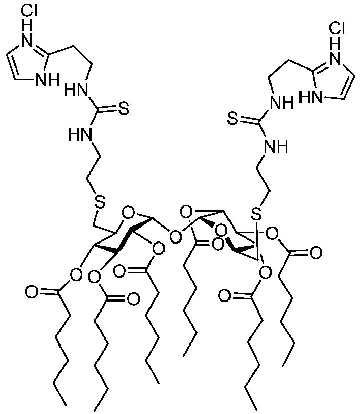 |
| JLF96 | 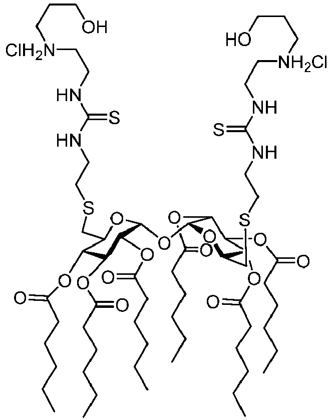 |
| JLF97 | 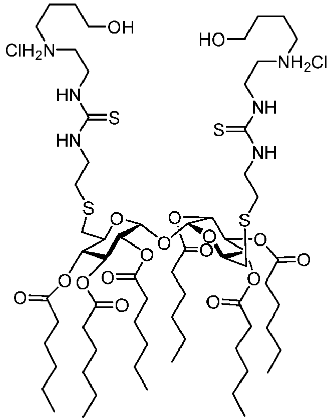 |
| JLF98 | 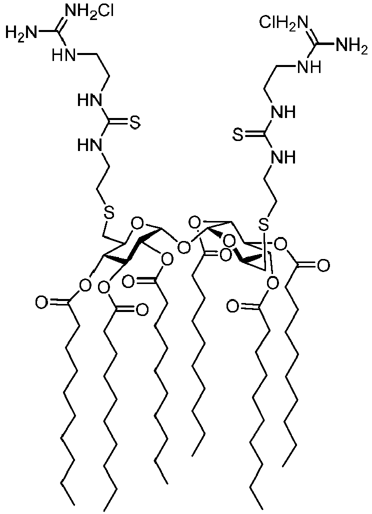 |
| JLF99 | 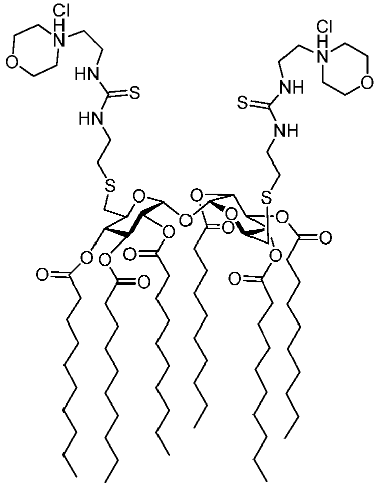 |
| JLF102 | 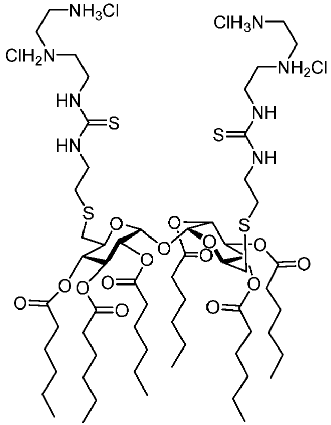 |
| JLF103 | 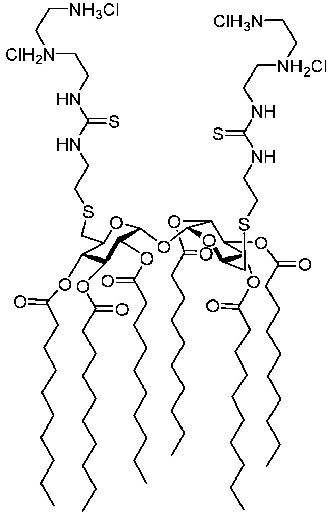 |
| JLF106 | 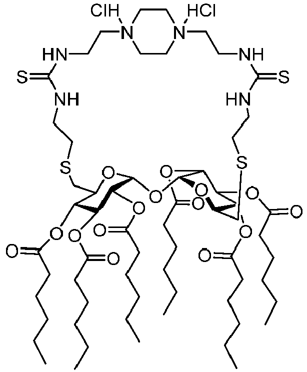 |
| JLF108 | 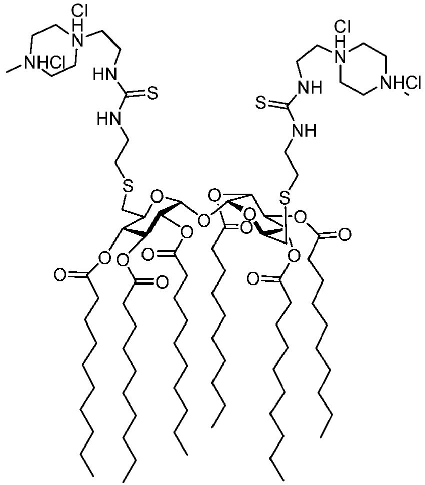 |
| JLF110 | 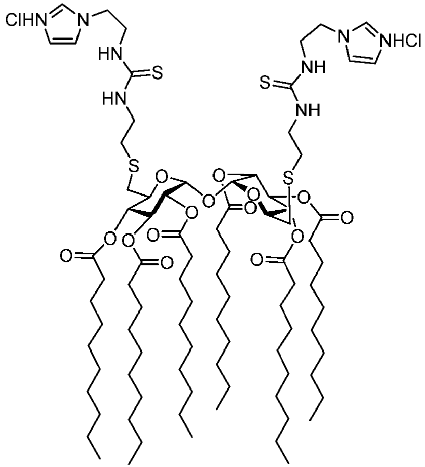 |
| JLF111 | 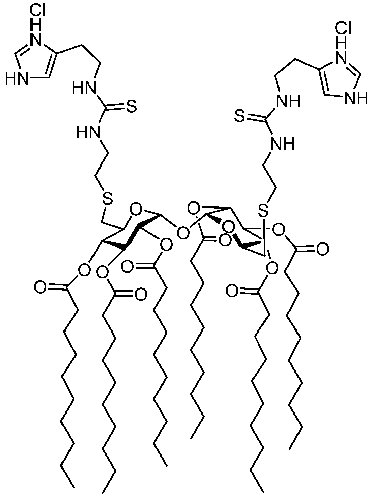 |
| JLF115 | 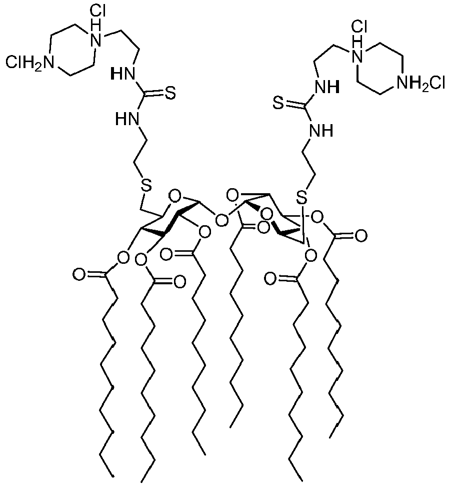 |
| JLF120 | 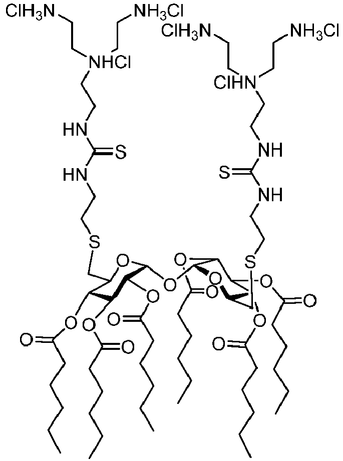 |
| JLF121 | 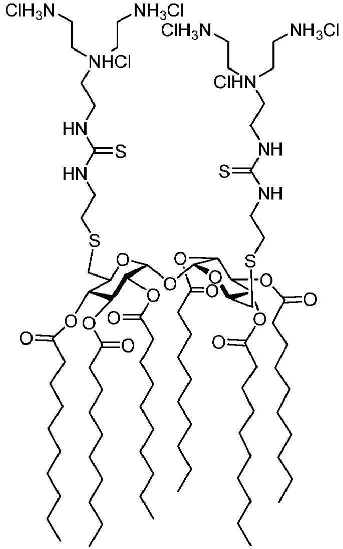 |
| JLF125 | 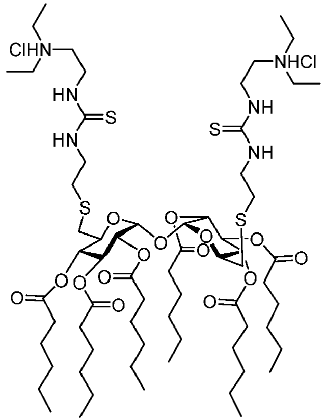 |
| JLF128 | 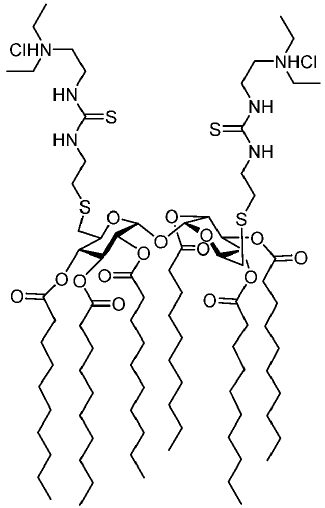 |
| JLF130 | 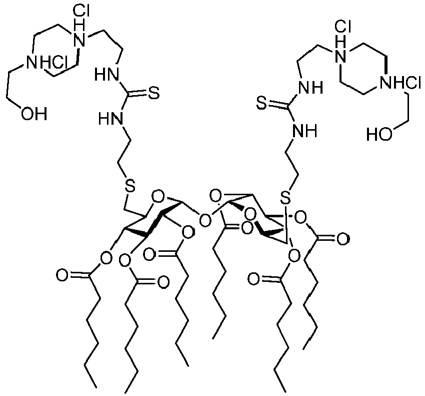 |
| JLF131 | 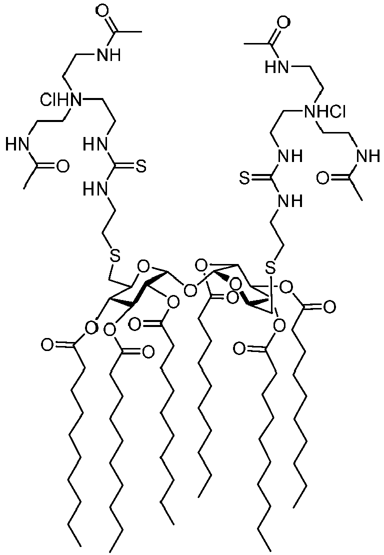 |
| JLF134 | 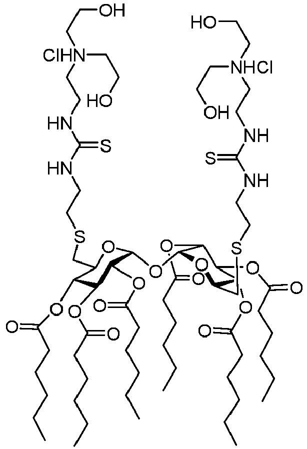 |
| JLF135 | 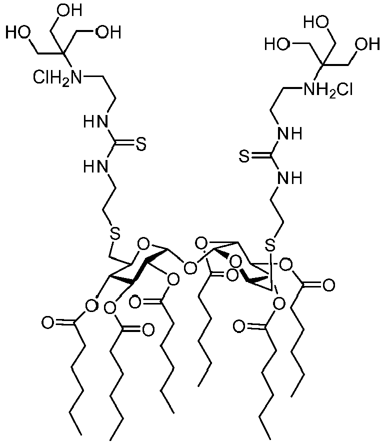 |
| JLF138 | 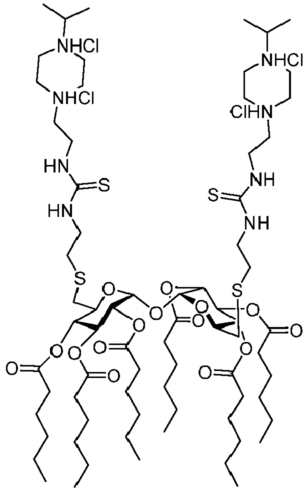 |
| JLF139 | 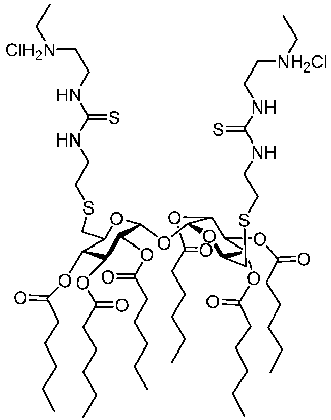 |
| JLF141 | 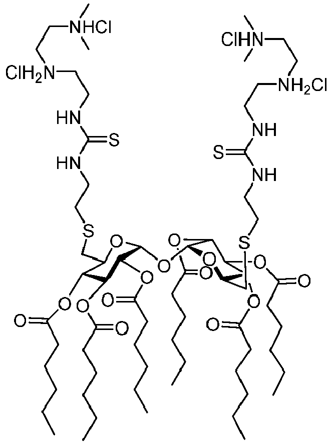 |
| JLF142 | 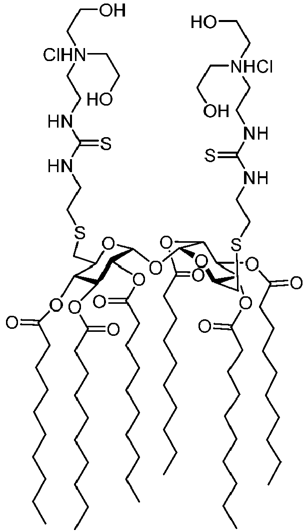 |
| JLJB604 | 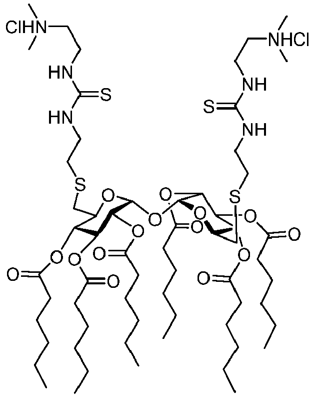 |
| JLJB605 | 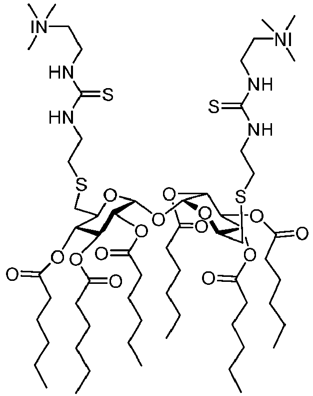 |
| JLJB617 | 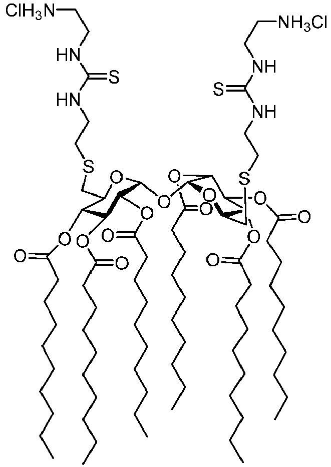 |
| JLJB618 | 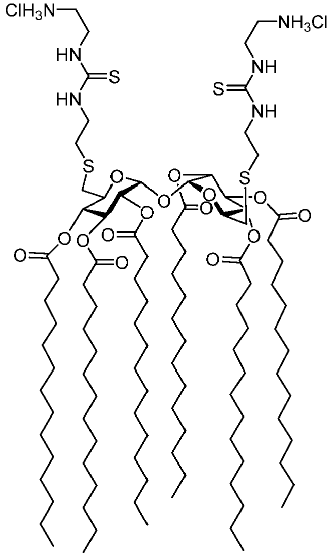 |
| JLJB619 | 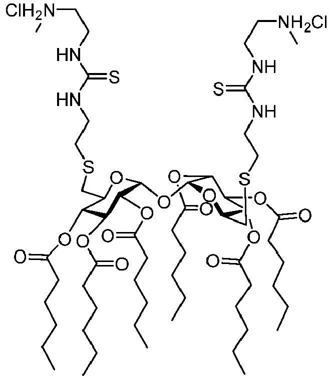 |
| PER20 | 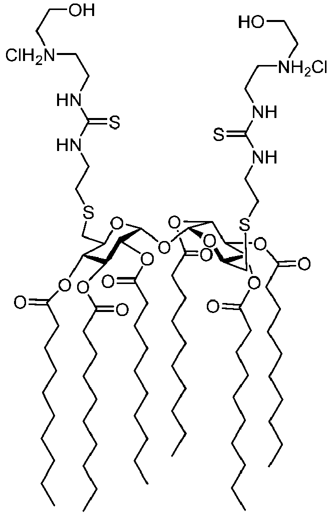 |

In some embodiments, the present disclosure provides a compound selected from:

| **Name** | **Structure** | |
|---|---|---|
| JRL13 | 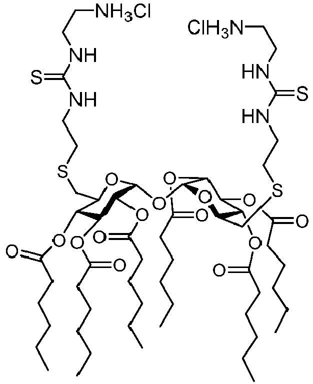 | |

| **Name** | **Structure** | |
|---|---|---|
| JRL45 | 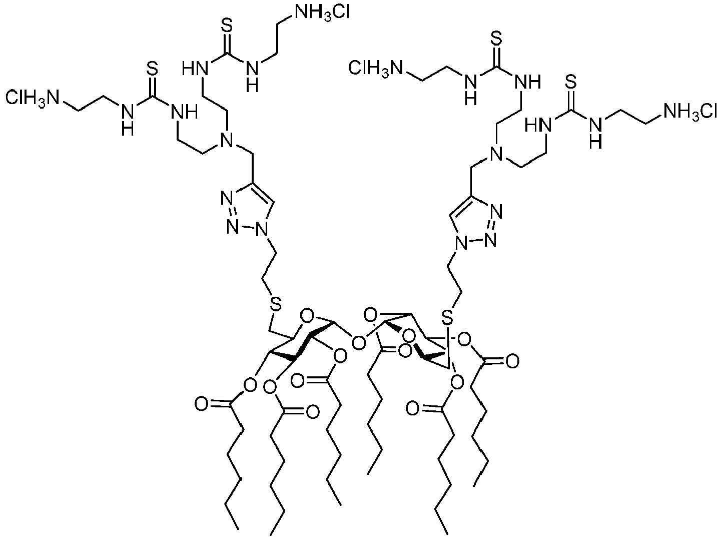 | |

### Complexes and Compositions

The compositions described herein exhibit unexpectedly improved characteristics relative to previously described complexes. Moreover, the present disclosure encompasses an insight that cationic and/or ionizable oligosaccharides are surprisingly useful for the delivery and targeted expression of particular therapeutic agents, e.g., nucleic acids, RNA.

Previous work related to cationic oligosaccharides focused on their use with DNA. In particular, Carbajo-Gordillo, *et al*., reported use of certain oligosaccharides for DNA nanocomplexing and delivery. See Carbajo-Gordillo, et al., Chem. Comm., 55:8227-8230 (2019). Notably, however, the oligosaccharide-DNA complexes reported by Carbajo-Gordillo, *et al*., exhibit surprisingly distinct properties from those reported in the present application, in particular when complexed with RNA. For example, the compositions reported herein, in some embodiments, exhibit different behavior in vivo, such as targeting (i.e., causing increased expression of RNA relative to other parts of the body) different systems in the body, and can be prepared with significantly reduced ratios of oligosaccharide to nucleic acid. For at least these reasons, the presently reported complexes exhibit unexpected benefits over those complexes previously reported.

For example, and solely by way of comparison, and as elaborated upon in Example 2, complexes pDNA with JRL13 provides a complex that predominantly targets the liver. See FIG. 3G. Complexing JRL13 with mRNA, in contrast, provides a complex that predominantly targets the lung. See FIG. 3E. Similarly, complexing pDNA with JRL45 provides a complex that is predominantly expressed in the lung. See FIG. 3G. Complexing JRL45 with mRNA, instead, targets the spleen, with very little complex being expressed in the lung. See FIG. 3E. These results are surprising. There is no indication in previous work that complexing a particular oligosaccharide with DNA would provide a composition for targeting one system, while complexing the same oligosaccharide with RNA would provide a complex targeting a different system. Further, the presently reported compositions exhibit improved ratios of cationic moieties, or groups that are ionizable to cationic groups (on an oligosaccharide) to anionic moieties (on a nucleic acid). For example, as described herein, a complex comprises a ratio of cationic groups to anionic groups, which is referred to as an "N/P ratio." As described herein, an N/P ratio refers to a molar ratio of cationic groups (or ionizable groups that can become cationic, referred to as "N" of N/P) in a composition comprising cationic oligosaccharide compounds relative to anionic groups in a composition comprising mRNA (referred to as "P" of N/P). The presently reported compositions exhibit favorable ratios, thereby providing concentrated complexes for RNA delivery, relative to previously reported complexes with DNA. For example, the complexes of Carbajo-Gordillo requires an N/P of about 20:1 for effective targeting and delivery of pDNA. The present complexes, in contrast, comprise an N/P ratio of less than 20:1.

For example, in some embodiments, an N/P ratio of a complex reported herein is less than 20:1. In some embodiments, an N/P ratio of a complex reported herein is less than or equal to 15:1. In some embodiments, an N/P ratio of a complex reported herein is less than or equal to 12:1. In some embodiments, an N/P ratio of a complex reported herein is less than or equal to 10:1. In some embodiments, an N/P ratio of a complex reported herein is less than or equal to 5:1. In some embodiments, an N/P ratio of a complex reported herein is less than or equal to 2:1. In some embodiments, an N/P ratio of a complex reported herein is less than or equal to 1:1. In some embodiments, an N/P ratio of a complex reported herein is from about 1:1 to about 15:1. In some embodiments, an N/P ratio of a complex reported herein is from about 1:1 to about 12:1. In some embodiments, an N/P ratio of a complex reported herein is from about 1:1 to about 10:1. In some embodiments, an N/P ratio of a complex reported herein is about 1:1, about 2:1, about 3:1, about 4:1, about 5:1, about 6:1, about 7:1, about 8:1, about 9:1, about 10:1, about 11:1, about 12:1, about 13:1, about 14:1, about 15:1, about 16:1, about 17:1, about 18:1, or about 19:1.

In some embodiments, a complex described herein has a diameter of about 30 nm to about 300 nm. In some embodiments a complex described herein has a diameter of about 50 nm to about 200 nm. In some embodiments, a complex described herein has a diameter that is about 50, 60, 70, 80, 90, 100, 110, 120, 130, 140, 150, 160, 170, 180, 190, or 200 nm. In some embodiments, a complex described herein has a diameter of about 30 nm to about 150 nm. In some embodiments, a complex described herein has a diameter of about 30 nm to about 100 nm. In some embodiments, a complex described herein has a diameter of less than 100 nm. In some embodiments, a complex described herein has a diameter of about 30, 35, 40, 45, 50, 55, 60, 65, 70, 75, 80, 85, 90, 95, or 100 nm.

In some embodiments, a composition or complex described herein further comprises a pharmaceutically acceptable surfactant. In some embodiments, a pharmaceutically acceptable surfactant is selected from a polysorbate (e.g., polysorbate 20 (Tween20), polysorbate 40 (Tween40), polysorbate 60 (Tween60), and polysorbate 80 (Tween80)), poloxamers, and an amphiphilic group comprising a moiety selected from polyalkylene glycols (e.g., polyethylene glycol), poly(2-oxazoline), poly(2-methyl-2-oxazoline), polysarcosine, polyvinylpyrrolidone, and poly[N-(2-hydroxypropyl)methacrylamide, wherein the moiety is bound to one or more C₁₂-C₂₀ aliphatic groups.

In some embodiments, a ratio of oligosaccharide to surfactant is about 1:0.0075 to about 1:3. In some embodiments, a ratio of oligosaccharide to surfactant is about 1:0.0075 to about 1:1.5. In some embodiments, a ratio of oligosaccharide to surfactant is about 1:1. In some embodiments, a ratio of oligosaccharide to surfactant is In some embodiments, a ratio of oligosaccharide to surfactant is about 1:0.0075, about 1:0.01, about 1:0.1, about 1:0.5, about 1:1, about 1:1.5, about 1:2, about 1:2.5. or about 1:3.

In some embodiments, a surfactant is a polysorbate. In some embodiments, a ratio of oligosaccharide to polysorbate is 1:0.0075 to 1:3. In some embodiments, a surfactant is a polysorbate. In some embodiments, a ratio of oligosaccharide to polysorbate is 1:0.0075 to 1:1.5. In some embodiments, a surfactant is a polysorbate. In some embodiments, a ratio of oligosaccharide to polysorbate is 1:0.0075 to 1:1. In some embodiments, a ratio of oligosaccharide to polysorbate is about 1:0.0075, about 1:0.01, about 1:0.1, about 1:0.5, about 1:1, about 1:1.5, about 1:2, about 1:2.5. or about 1:3.. In some embodiments, a complex comprising an oligosaccharide and a pharmaceutically acceptable surfactant has a diameter of about 30 nm to about 150 nm. In some embodiments, a complex described herein has a diameter of about 30 nm to about 100 nm. In some embodiments, a complex comprising an oligosaccharide and a pharmaceutically acceptable surfactant has a diameter of less than 100 nm. In some embodiments, a complex comprising an oligosaccharide and a pharmaceutically acceptable surfactant has a diameter of about 30, 35, 40, 45, 50, 55, 60, 65, 70, 75, 80, 85, 90, 95, or 100 nm.

### RNA

In some embodiments, a complex described herein comprises one or more oligosaccharide compositions and a nucleic acid. In some embodiments, a nucleic acid is RNA.

In some embodiments, an RNA amenable to technologies described herein is a single-stranded RNA. In some embodiments, an RNA as disclosed herein is a linear RNA. In some embodiments, a single-stranded RNA is a non-coding RNA in that its nucleotide sequence does not include an open reading frame (or complement thereof). In some embodiments, a single-stranded RNA has a nucleotide sequence that encodes (or is the complement of a sequence that encodes) a polypeptide or a plurality of polypeptides (e.g., epitopes) of the present disclosure.

In some embodiments, an RNA is or comprises an siRNA, an miRNA, or other non-coding RNA.

In many embodiments, a relevant RNA includes at least one open reading frame (ORF) (*e.g.*, is an mRNA); in some embodiments, a relevant RNA includes a single ORF; in some embodiments, a relevant RNA includes more than one ORF.

In some embodiments, an RNA comprises an ORF, *e.g.*, encoding a polypeptide of interest or encoding a plurality of polypeptides of interest. In some embodiments, an RNA produced in accordance with technologies provided herein comprises a plurality of ORFs (*e.g*., encoding a plurality of polypeptides). In some embodiments, an RNA produced in accordance with technologies herein comprises a single ORF that encodes a plurality of polypeptides. In some such embodiments, polypeptides are or comprise antigens or epitopes thereof (*e.g*., relevant antigens).

In some embodiments, an ORF for use in accordance with the present disclosure encodes a polypeptide that includes a signal sequence, *e.g.*, that is functional in mammalian cells, such as an intrinsic signal sequence or a heterologous signal sequence. In some embodiments, a signal sequence directs secretion of an encoded polypeptide, in some embodiments, a signal sequence directs transport of an encoded polypeptide into a defined cellular compartment, preferably the cell surface, the endoplasmic reticulum (ER) or the endosomal-lysosomal compartment.

In some embodiments, an ORF encodes a polypeptide that includes a multimerization element (e.g., an instrinsic or heterologous multimerization element). In some embodiments, an ORF that encodes a surface polypeptide (e.g., that includes a signal sequence directing surface localization) includes a multimerization element.

In some embodiments, an ORF encodes a polypeptide that includes a transmembrane element or domain.

In some embodiments, an ORF is codon-optimized for expression in a cells of a particular host, *e.g*., a mammalian host, *e.g.*, a human.

In some embodiments, an RNA includes unmodified uridine residues; an RNA that includes only unmodified uridine residues may be referred to as a "uRNA". In some embodiments, an RNA includes one or more modified uridine residues; in some embodiments, such an RNA (*e.g*., an RNA including entirely modified uridine residues) is referred to as a "modRNA". In some embodiments, an RNA may be a self-amplifying RNA (saRNA). In some embodiments, an RNA may be a trans-amplifying RNA (*see*, for example, WO2017/162461).

In some embodiments, a relevant RNA includes a polypeptide-encoding portion or a plurality of polypeptide-encoding portions. In some particular embodiments, such a portion or portions may encode a polypeptide or polypeptides that is or comprises a biologically active polypeptide or portion thereof (*e.g*., an enzyme or cytokine or therapeutic protein such as a replacement protein or antibody or portion thereof). In some particular embodiments, such a portion or portions may encode a polypeptide or polypeptides that is or comprises an antigen (or an epitope thereof), a cytokine, an enzyme, etc. In some embodiments, an encoded polypeptide or polypeptides may be or include one or more neoantigens or neoepitopes associated with a tumor. In some embodiments, an encoded polypeptide or polypeptides may be or include one or more antigens (or epitopes thereof) of an infectious agent (e.g., a bacterium, fungus, virus, etc.). In certain embodiments, an encoded polypeptide may be a variant of a wild type polypeptide.

In some embodiments, a single-stranded RNA (*e.g.*, mRNA) may comprise a secretion signal-encoding region (*e.g*., a secretion signal-encoding region that allows an encoded target entity or entities to be secreted upon translation by cells). In some embodiments, such a secretion signal-encoding region may be or comprise a non-human secretion signal. In some embodiments, such a secretion signal-encoding region may be or comprise a human secretion signal.

In some embodiments, a single-stranded RNA (*e.g*., mRNA) may comprise at least one non-coding element (*e.g.*, to enhance RNA stability and/or translation efficiency). Examples of non-coding elements include but are not limited to a 3' untranslated region (UTR), a 5' UTR, a cap structure (*e.g*., in some embodiments, an enzymatically-added cap; in some embodiments, a co-transcriptional cap), a poly adenine (polyA) tail (e.g., that, in some embodiments, may be or comprise 100 A residues or more, and/or in some embodiments may include one or more "interrupting" [*i.e.*, non-A] sequence elements), and any combinations thereof. Exemplary embodiments of such non-coding elements may be found, for example, in WO2011015347, WO2017053297, US 10519189, US 10494399, WO2007024708, WO2007036366, WO2017060314, WO2016005324, WO2005038030, WO2017036889, WO2017162266, and WO2017162461, each of which is incorporated herein by referenced in its entirety.

### Formats

At least four formats useful for RNA pharmaceutical compositions (e.g., immunogenic compositions or vaccines) have been developed, namely non-modified uridine containing mRNA (uRNA), nucleosidemodified mRNA (modRNA), self-amplifying mRNA (saRNA), and trans-amplifying RNAs.

Features of a non-modified uridine platform may include, for example, one or more of intrinsic adjuvant effect, good tolerability and safety, and strong antibody and T cell responses.

Features of modified uridine (e.g., pseudouridine) platform may include reduced adjuvant effect, blunted immune innate immune sensor activating capacity and thus augmented antigen expression, good tolerability and safety, and strong antibody and CD4-T cell responses. As noted herein, the present disclosure provides an insight that such strong antibody and CD4 T cell responses may be particularly useful for vaccination.

Features of self-amplifying platform may include, for example, long duration of polypeptide (*e.g.*, protein) expression, good tolerability and safety, higher likelihood for efficacy with very low vaccine dose.

In some embodiments, a self-amplifying platform (*e.*g., RNA) comprises two nucleic acid molecules, wherein one nucleic acid molecule encodes a replicase (*e.g*., a viral replicase) and the other nucleic acid molecule is capable of being replicated (*e.g*., a replicon) by said replicase *in trans* (trans-replication system). In some embodiments, a self-amplifying platform (*e.g*., RNA) comprises a plurality of nucleic acid molecules, wherein said nucleic acids encode a plurality of replicases and/or replicons.

In some embodiments, a *trans*-replication system comprises the presence of both nucleic acid molecules in a single host cell.

In some such embodiments, a nucleic acid encoding a replicase (*e.g*., a viral replicase) is not capable of self-replication in a target cell and/or target organism. In some such embodiments, a nucleic acid encoding a replicase (*e.g*., a viral replicase) lacks at least one conserved sequence element important for (-) strand synthesis based on a (+) strand template and/or for (+) strand synthesis based on a (-) strand template.

In some embodiments, a self-amplifying RNA comprises a 5'-cap; in some *trans-*replication systems, at least an RNA encoding a replicase is capped. Without wishing to be bound by any one theory, it has been found that a 5'-cap can be important for high level expression of a gene of interest *in trans.*

In some embodiments, a self-amplifying platform does not require propagation of virus particles (*e.g.*, is not associated with undesired virus-particle formation). In some embodiments, a self-amplifying platform is not capable of forming virus particles.

In some embodiments, an RNA may comprise an Internal Ribosomal Entry Site (IRES) element. In some embodiments, an RNA does not comprise an IRES site; in particular, in some embodiments, an saRNA does not comprise an IRES site. In some such embodiments, translation of a gene of interest and/or replicase is not driven by an IRES element. In some embodiments, an IRES element is substituted by a 5'-cap. In some such embodiments, substitution by a 5'-cap does not affect the sequence of a polypeptide encoded by an RNA.

In some embodiments, a complex described herein comprises modRNA or saRNA. In some embodiments, a complex comprises modRNA. In some embodiments, a complex comprises saRNA.

### Methods of Use

Complexes described herein are useful in the treatment and prophylaxis in a subject of diseases, disorders, and conditions described herein. In some embodiments, a disease, disorder, or condition is an infectious disease, cancer, an autoimmune disease, or a rare disease.

In some embodiments, an infectious disease is caused by or associated with a viral pathogen. In some embodiments, a viral pathogen is of a family selected from poxviridae, rhabdoviridae, filoviridae, paramyxoviridae, hepadnaviridae, coronaviridae, caliciviridae, picornaviridae, reoviridae, retroviridae, and orthomyxoviridae. In some embodiments, an infectious disease is a virus selected from SARS-CoV-2, influenza, Crimean-Congo Hemorhhagic Fever (CCHF), Ebola virus, Lassa virus, Marburg virus, HIV, Nipah virus, and MERS-CoV.

In some embodiments, an infectious disease is caused by or associated with a bacterial pathogen. In some embodiments, a bacterial pathogen is of a species selected from Actinomyces israelii, bacillus antracis, Bacteroides fragilis, Bordetella pertussis, Borrelia burgdorferi, Borrelia garinii, Borrelia afzelii, Borrelia recurrentis, Brucella abortus, Brucella canis, Brucella melitensis, Brucella suis, Campolobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium idphteriae, Ehrlichia canis, Ehrlichia chaffeensis, Enterococcus faecalis, Enterococcus faecium, Escherichia coli, Francisella tularensis, Haemophilus influenzae, Helicobacter pylori, Klebsiella pneumoniae, Legionella pneumophila, Leptospira, Listeria monocytogenes, Mycobacterium leprae, Mycobacterium tuberculosis, Mycoplasma pneumoniae, Neisseria gonorrhoeae, Neisseria meningitidis, Pseudomonas aeruginosa, Nocardia asteroids, Rickettsia ricektssii, Salmonella typhi, Salmonella typhimurium, Shigella sonnei, Shigella dysenteriae, Staphylococcus aureus, Staphylococcus epidermidis, Staphylococcus saprophyticus, Streptococcus agalactiae, Streptococcus pneumoniae, Streptococcus pyogenes, Streptococcus viridans, Treponema pallidum, Vibrio cholerae, and Yersinia pestis.

In some embodiments, an infectious disease is caused by or associated with a parasite. In some embodiments, a parasite is of a family selected from Plasmodium, Leishmania, Cryptosporidium, Entamoeba, Trypanosomas, Schistosomes, Ascaris, Echinococcus and Taeniidae.

In some embodiments, a disease, disorder, or condition is a cancer. In some embodiments, a cancer is selected from bladder cancer, breast cancer, colorectal cancer, kidney cancer, lung cancer, lymphoma, melanoma, oral/oropharyngeal cancer, pancreatic cancer, prostate cancer, thyroid cancer, and uterine cancer.

In some embodiments, a disease, disorder, or condition is a genetic disorder. In some embodiments, a genetic disorder is associated with a gain-of-function mutation or a loss-of-function mutation.

In some embodiments, a disease, disorder, or condition is an autoimmune disease. In some embodiments, an autoimmune disease is selected from addison disease, celiac disease, rheumatoid arthritis, lupus, inflammatory bowel disease, dermatomyositis, multiple sclerosis, diabetes, guillain-barre syndrome, chronic inflammatory demyelinating polyneuropathy, psoriasis, pernicious anemia, graves' disease, hashimoto's thyroiditis, myasthenia gravis, and vasculitis sjörgen syndrome.

In some embodiments, a disease, disorder, or condition is a rare disease. As described herein, a rare disease refers to a life-threatening or chronically debilitating diseases which are of such low prevalence (e.g., fewer than 1/2000 people) that special combined efforts are needed to address them.

In some embodiments, the present disclosure provides complexes that can selectively target particular systems within a body. As used herein, reference to "targeting" a particular system refers to causing increased expression of RNA derived from cargo in the complex in the desired system. For example, in some embodiments, complexes described herein can selectively target the lungs, liver, spleen, heart, brain, lymph nodes, bladder, kidneys, and pancreas. As described herein, a complex "selectively targets" an organ when a single target expresses mRNA in an amount that is 65% or greater than expression in other organs post administration (e.g., 65% or more of mRNA throughout the body is expressed from a single organ, with the remaining 35% distributed between one or more different organs). In some embodiments, a complex described herein selectively targets the lungs. In some embodiments, a complex described herein selectively targets the liver. In some embodiments, a complex described herein selectively targets the spleen. In some embodiments, a complex described herein selectively targets the heart.

### Methods of Delivery

The present disclosure provides, among other things, a complex (e.g., a pharmaceutical composition or a pharmaceutical formulation, as referred to herein) to be administered to a subject. For example, in some embodiments, a complex is administered as a monotherapy. In some embodiments, a complex is administered as part of a combination therapy. In some embodiments, a concentration of total RNA (e.g., a total concentration of all of the one or more RNA molecules) in a pharmaceutical composition described herein is of about 0.01 mg/mL to about 0.5 mg/mL, or about 0.05 mg/mL to about 0.1 mg/mL.

Pharmaceutical compositions may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2006; incorporated herein by reference in its entirety) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this disclosure.

In some embodiments, an excipient is approved for use in humans and for veterinary use. In some embodiments, an excipient is approved by the United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical formulations. Excipients such as cocoa butter and suppository waxes, coloring agents, coating agents, sweetening, flavoring, and/or perfuming agents can be present in the composition, according to the judgment of the formulator.

General considerations in the formulation and/or manufacture of pharmaceutical agents may be found, for example, in Remington: The Science and Practice of Pharmacy 21st ed., Lippincott Williams & Wilkins, 2005 (incorporated herein by reference in its entirety).

In some embodiments, pharmaceutical compositions provided herein may be formulated with one or more pharmaceutically acceptable carriers or diluents as well as any other known adjuvants and excipients in accordance with conventional techniques such as those disclosed in Remington: The Science and Practice of Pharmacy 21st ed., Lippincott Williams & Wilkins, 2005 (incorporated herein by reference in its entirety).

Pharmaceutical complexes and compositions described herein can be administered by appropriate methods known in the art. As will be appreciated by a skilled artisan, the route and/or mode of administration may depend on a number of factors, including, *e.g*., but not limited to stability and/or pharmacokinetics and/or pharmacodynamics of pharmaceutical compositions described herein.

In some embodiments, pharmaceutical compositions described herein are formulated for parenteral administration, which includes modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

In some embodiments, pharmaceutical compositions described herein are formulated for intravenous administration. In some embodiments, pharmaceutically acceptable carriers that may be useful for intravenous administration include sterile aqueous solutions or dispersions and sterile powders for preparation of sterile injectable solutions or dispersions.

In some particular embodiments, pharmaceutical compositions described herein are formulated for subcutaneous (s.c) administration. In some particular embodiments, pharmaceutical compositions described herein are formulated for intramuscular (i.m) administration.

Therapeutic compositions typically must be sterile and stable under the conditions of manufacture and storage. The composition can be formulated as a solution, dispersion, powder (e.g., lyophilized powder), microemulsion, lipid nanoparticles, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. In some embodiments, prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration.

In some embodiments, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Examples of suitable aqueous and nonaqueous carriers which may be employed in the pharmaceutical compositions described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the presence of microorganisms may be ensured both by sterilization procedures, and by the inclusion of various antibacterial and antifungal agents, for example, paraben, chlorobutanol, phenol sorbic acid, and the like. It may also be desirable to include isotonic agents, such as sugars, sodium chloride, and the like into pharmaceutical compositions described herein. In addition, prolonged absorption of the injectable pharmaceutical form may be brought about by the inclusion of agents which delay absorption such as aluminum monostearate and gelatin.

Formulations of pharmaceutical compositions described herein may be prepared by any method known or hereafter developed in the art of pharmacology. In general, such preparatory methods include the step of bringing active ingredient(s) into association with a diluent or another excipient and/or one or more other accessory ingredients, and then, if necessary and/or desirable, shaping and/or packaging the product into a desired single- or multi-dose unit.

A pharmaceutical composition in accordance with the present disclosure may be prepared, packaged, and/or sold in bulk, as a single unit dose, and/or as a plurality of single unit doses. As used herein, a "unit dose" is discrete amount of the pharmaceutical composition comprising a predetermined amount of at least one RNA product produced using a system and/or method described herein.

In some embodiments, an active agent that may be included in a pharmaceutical composition described herein is or comprises a therapeutic agent administered in a combination therapy described herein. Pharmaceutical compositions described herein can be administered in combination therapy, i.e., combined with other agents. In some embodiments, such therapeutic agents may include agents leading to depletion or functional inactivation of regulatory T cells. For example, in some embodiments, a combination therapy can include a provided pharmaceutical composition with at least one immune checkpoint inhibitor.

In some embodiments, pharmaceutical composition described herein may be administered in conjunction with radiotherapy and/or autologous peripheral stem cell or bone marrow transplantation.

In some embodiments, a pharmaceutical composition described herein can be frozen to allow long-term storage.

Although the descriptions of pharmaceutical compositions provided herein are principally directed to pharmaceutical compositions that are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation.

### Methods of Preparing Compositions Described Herein

As described herein, the present disclosure provides, among other things, a method of preparing an oligosaccharide of formula II: 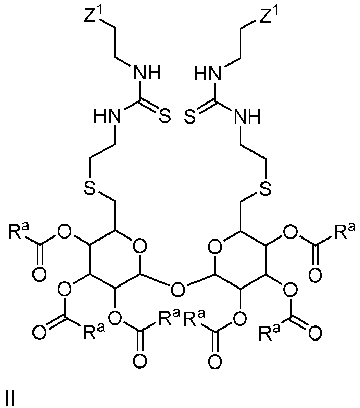 comprising contacting a compound of formula III: 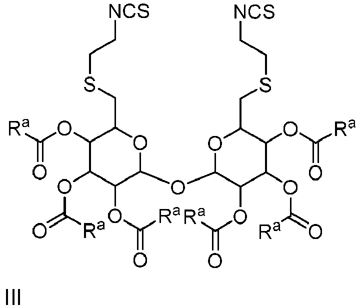 with a compound of formula IV: 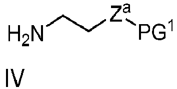
in the presence of an acid,
wherein
each R^{a} is optionally substituted -C₁-C₂₀ aliphatic;
Z¹ is a cationic or ionizable group selected from optionally substituted 5- to 14-membered heterocyclyl ring having 1-3 heteroatoms selected from N, O, and S, - N⁺(M)₃, 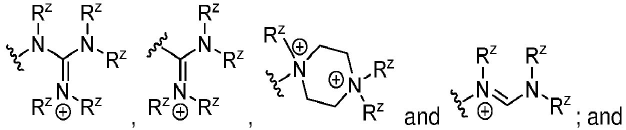
each M is independently ―C₀-C₆ aliphatic-R^{z} or ―C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S;
Z^{a}-PG¹ is Z¹ wherein one R^{z} has been replaced with a PG¹ group; and
PG¹ a suitable nitrogen protecting group.

In some embodiments, a method of preparing a compound of formula III comprises contacting a compound of formula V 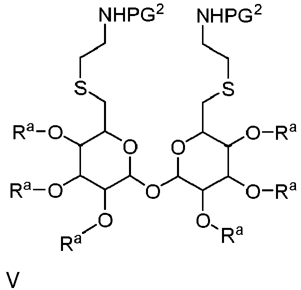 with an acid and then contacting the resulting product with CSCl₂ to provide a compound of formula III; wherein PG² is a suitable acid sensitive nitrogen protecting group.

In some embodiments, a method of preparing a compound of formula V comprises contacting a compound of formula VI 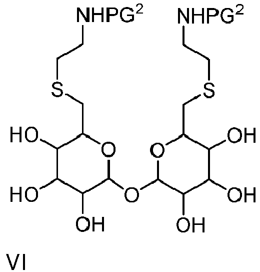 with a compound of formula VII:

R^{a}-COCl VII

to provide a compound of formula V.

In some embodiments, a method of preparing a compound of formula VI comprises contacting a compound of formula VIII: 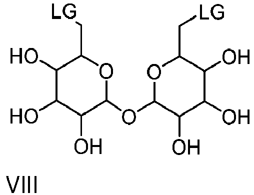 with a compound of formula IX:

HS(CH₂)₂NHPG² IX

to provide a compound of formula VI, wherein LG is a suitable leaving group

In some embodiments, PG¹ and PG² are each independently selected from Boc (tert-butoxycarbonyl), Fmoc (fluorenylmethyloxycarbonyl), Cbz (benzyl chloroformate), Troc (2,2,2-trichloroethoxycarbonyl), Trityl (triphenylmethyl), Phthalimide, Tetrachlorophthalimide, and Trifluoroaceamide.

In some embodiments, LG is selected from iodo, bromo, chloro, methylsulfonate, tricholoromethylsulfonate, and p-toluenesulfonate.

### Exemplary Embodiments

Embodiment 1. A composition comprising an oligosaccharide of formula I: 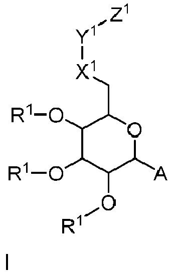 or a pharmaceutically acceptable salt thereof, wherein:
A is A¹, A², or A³: 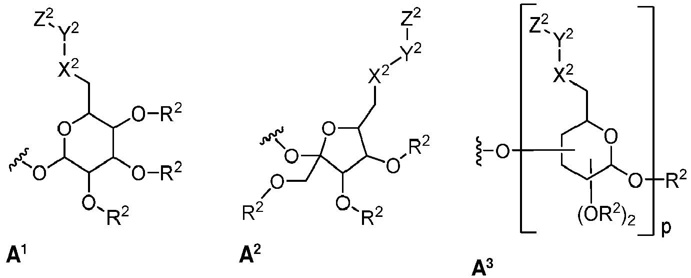
R¹ and R² are each independently selected from H, R^{a}, and -C(O)-R^{a};
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, and -NH-;
Y¹ and Y² are each independently an optionally substituted C₁₋₃₀ aliphatic group wherein one or more carbons are optionally and independently replaced by -Cy-, -NH-, -NHC(O)-, -C(O)NH-, -NHC(O)O-, -OC(O)NH-, -NHC(O)NH-, -NHC(S)NH-, -C(S)NH- - C(O)NHSO₂-, -SO₂NHC(O)-,-OC(O)O-, -O-, -C(O)-, -OC(O)-, -C(O)O-, -SO-, or -SO₂-;
each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃, 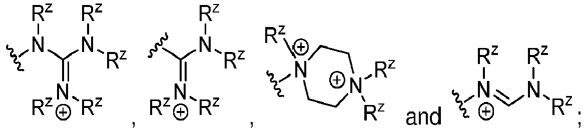
each M is independently ―C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5;
provided that when A is A¹, then:
   (i) each R¹ and R² is not ―CO-(CH₂)₄-CH₃, X¹ and X² are not both -S-, Y¹ and Y² are not both ―(CH₂)₂- and Z¹ and Z² are not both ―N⁺(H)₃,
   (ii) each R¹ and R² is not ―O-(CH₂)₅-CH₃ or ―O-(CH₂)₁₃-CH₃, X¹ and X² are not both -S-, Y¹ and Y² are not both ―(CH₂)₂-, and Z¹ and Z² are not both ―N⁺(H)₃,
   (iii) each R¹ and R² is not ―CO-(CH₂)₄-CH₃, X¹ and X² are not both -S-, Y¹ and Y² are not both ―(CH₂)₂-NH-C(S)-NH-(CH₂)₂-, and Z¹ and Z² are not both ―N+(H)₃, and
   (iv) Cy is not triazolyl.

Embodiment 2. The composition of Embodiment 1, wherein the oligosaccharide is of formula Ia: 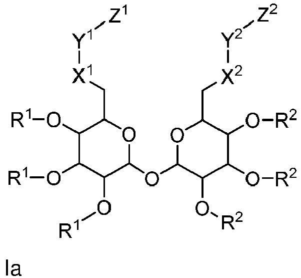 or a pharmaceutically acceptable salt thereof.

Embodiment 3. The composition of Embodiment 1, wherein the oligosaccharide is of formula Ib: 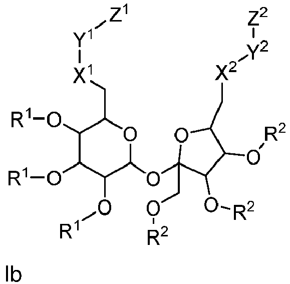 or a pharmaceutically acceptable salt thereof.

Embodiment 4. The composition of Embodiment 1, wherein the oligosaccharide is of formula Ic: 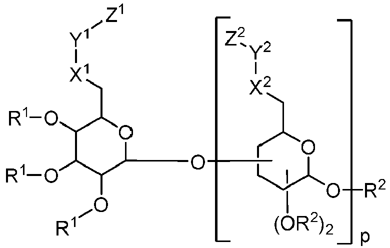 or a pharmaceutically acceptable salt thereof.

Embodiment 5. The composition of any one of Embodiments 1-4, wherein R¹ and R² are each independently selected from R^{a} and -C(O)-R^{a}, and R^{a} is C₁-C₂₀ aliphatic.

Embodiment 6. The composition of any one of Embodiments 1-5, wherein R¹ and R² are each ―C(O)-R^{a}, and R^{a} is C₁-C₁₄ aliphatic.

Embodiment 7. The composition of any one of Embodiments 1-6, wherein each R^{a} is C₅-C₁₀ linear alkyl.

Embodiment 8. The composition of Embodiment 1, wherein R¹ and R² are each ―C(O)-R^{a}, and R^{a} is 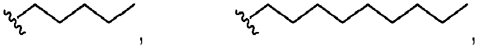 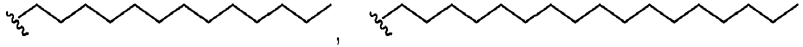 or

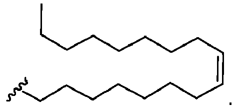

Embodiment 9. The composition of any one of Embodiments 1-8, wherein X¹ and X² are each -S-.

Embodiment 10. The composition of any one of Embodiments 1-9, wherein Y¹ and Y² are each selected from C₁-C₁₀ aliphatic, C₀-C₄-aliphatic-NHC(O)NH-C₀-C₄ aliphatic, Co-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-NHC(O)-C₀-C₄ aliphatic, C₀-C₄-aliphatic-NHSO₂-C₀-C₄ aliphatic, and C₀-C₄-aliphatic-C(O)-C₀-C₄ aliphatic. Embodiment 11. The composition of any one of Embodiments 1-10, wherein Y¹ and Y² are each C₁-C₄ aliphatic-NHC(S)NH-C₁-C₄ aliphatic.

Embodiment 12. The composition of Embodiment 1, wherein Y¹ and Y² are each ―CH₂-CH₂-NHC(S)NH-CH₂-CH₂-.

Embodiment 13. The composition of any one of Embodiments 1-12, wherein a moiety X¹-Y¹-Z¹ is

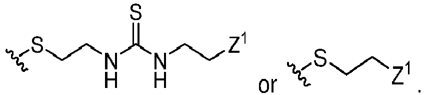

Embodiment 14. The composition of any one of Embodiments 1-13, wherein a moiety X²-Y²-Z² is

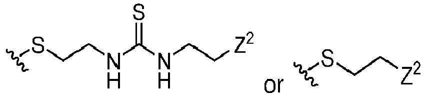

Embodiment 15. The composition of any one of Embodiments 1-14, wherein Z¹ and Z² are each independently selected from: N⁺(M)₃,

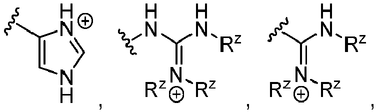

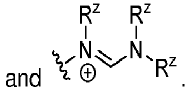

Embodiment 16. The composition of any one of Embodiments 1-14, wherein Z¹ and Z² are each independently selected from:

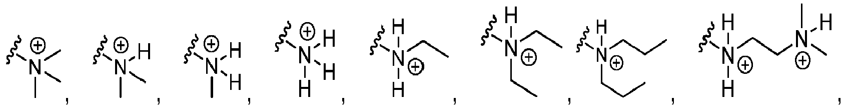

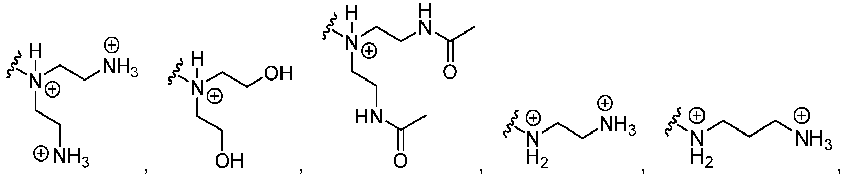

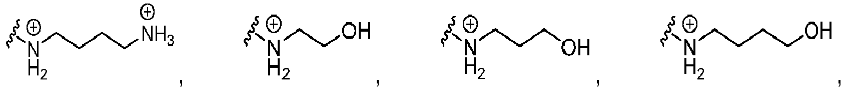

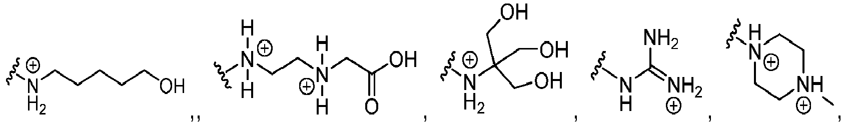

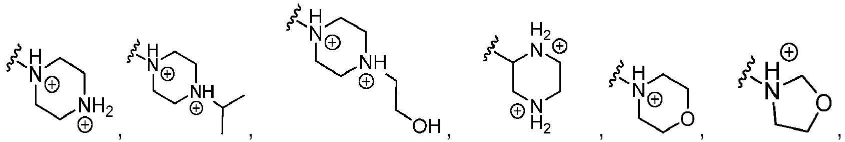

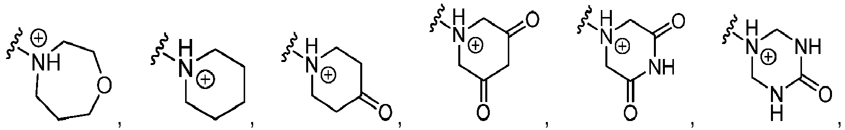

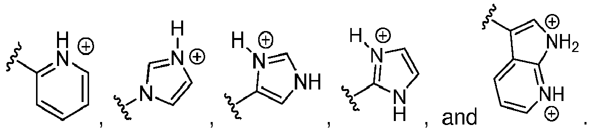

Embodiment 17. The compound of Embodiment 1, wherein the oligosaccharide is a compound of formula Id: 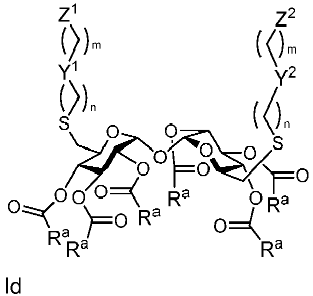 or a pharmaceutically acceptable salt thereof, wherein n and m are each independently selected from 0, 1, 2, 3, 4, 5, or 6.

Embodiment 18. The compound of Embodiment 17, wherein the oligosaccharide is a compound of formula Id-i: 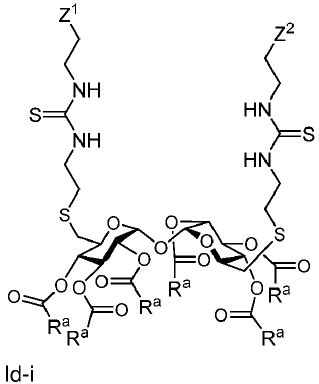 or a pharmaceutically acceptable salt thereof, wherein n and m are each independently selected from 0, 1, 2, 3, 4, 5, or 6.

Embodiment 19. The composition of any one of Embodiments 1-18, further comprising one or more suitable counterions.

Embodiment 20. The composition of Embodiment 1, wherein the oligosaccharide is selected from Table A

Embodiment 21. A complex comprising the composition oligosaccharide of any one of Embodiments 1-20, and a nucleic acid.

Embodiment 22. The complex of Embodiment 21, wherein the nucleic acid is RNA.

Embodiment 23. The complex of Embodiment 22, wherein the RNA is a modRNA or a saRNA.

Embodiment 24. The complex of Embodiments 22 or 23, wherein a ratio of N/P is less than 20:1.

Embodiment 25. The complex of Embodiment 24, wherein a ratio of N/P is equal or less than 12:1.

Embodiment 26. The complex of any one of Embodiments 21-25, wherein the complex has a diameter of about 30 nm to about 300 nm.

Embodiment 27. The complex of any one of Embodiments 21-26, wherein the complex has a diameter of about 50 nm to about 200 nm.

Embodiment 28. The complex of any one of Embodiments 21-27, further comprising a pharmaceutically acceptable surfactant.

Embodiment 29. The complex of Embodiment 28, wherein the pharmaceutically acceptable surfactant is a polysorbate.

Embodiment 30. The complex of Embodiments 28 or 29, wherein a ratio of the oligosaccharide to the pharmaceutically acceptable surfactant is from about 1:0.0075 to about 1:3.

Embodiment 31. A method of increasing or causing increased expression of RNA in a target in a subject comprising administering to the subject the complex of any one of Embodiments 21-30.

Embodiment 32. The method of Embodiment 31, wherein the target is selected from the lungs, liver, spleen, heart, brain, lymph nodes, bladder, kidneys, and pancreas. Embodiment 33. A method of treating a disease, disorder, or condition in a subject comprising administering to the subject a complex of any one of Embodiments 21-30. Embodiment 34. The method of Embodiment 33, wherein the disease, disorder, or condition is an infectious disease, cancer, a genetic disorder, an autoimmune disease, or a rare disease

Embodiment 35. The method of any one of Embodiments 31-34, wherein the complex is administered intramuscularly.

Embodiment 36. The method of Embodiment 35, wherein the complex is of a diameter between 50 nm and 100 nm.

Embodiment 37. The method of any one of Embodiments 31-34, wherein the complex is administered subcutaneously.

Embodiment 38. A method of preparing an oligosaccharide of formula II: 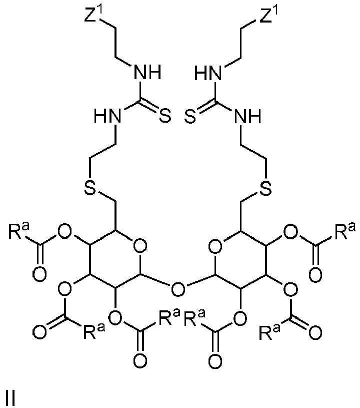 comprising contacting a compound of formula III: 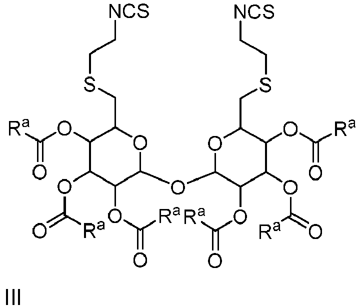 with a compound of formula IV: 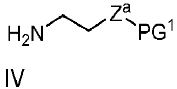
in the presence of an acid,
wherein
each R^{a} is optionally substituted -C₁-C₂₀ aliphatic;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃, 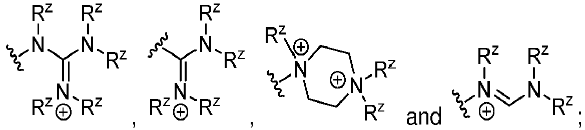 and
each M is independently ―C₀-C₆ aliphatic-R^{z} or ―C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S;
Z^{a}-PG¹ is Z¹ wherein one R^{z} has been replaced with a PG¹ group;
PG¹ a suitable nitrogen protecting group, provided that Z¹ and Z² are not both ―N⁺(H)₃.

Embodiment 39. The method of Embodiment 38, comprising first contacting a compound of formula V 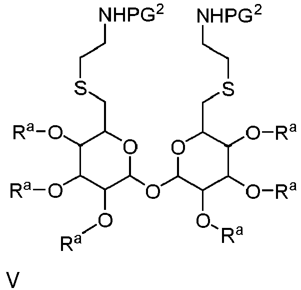 with an acid and then contacting the resulting product with CSCl₂ to provide a compound of formula III; wherein PG² is a suitable acid sensitive nitrogen protecting group.

Embodiment 40. The method of Embodiment 39, comprising contacting a compound of formula VI 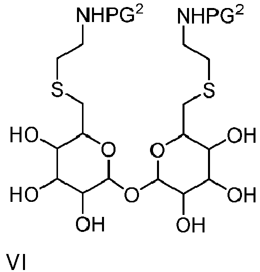 with a compound of formula VII:

R^{a}-COCl VII

to provide a compound of formula V.

Embodiment 41. The method of Embodiment 40, comprising contacting a compound of formula VIII: 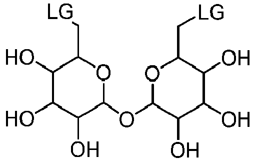 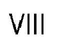 with a compound of formula IX:

HS(CH₂)₂NHPG² IX

to provide a compound of formula VI,
wherein LG is a suitable leaving group.

The foregoing has been a description of certain non-limiting embodiments of the subject matter described within. Accordingly, it is to be understood that the embodiments described in this specification are merely illustrative of the subject matter reported within. Reference to details of the illustrated embodiments is not intended to limit the scope of the claims, which themselves recite those features regarded as essential.

It is contemplated that systems and methods of the claimed subject matter encompass variations and adaptations developed using information from the embodiments described within. Adaptation, modification, or both, of the systems and methods described within may be performed by those of ordinary skill in the relevant art. Throughout the description, where systems are described as having, including, or comprising specific components, or where methods are described as having, including, or comprising specific steps, it is contemplated that, additionally, there are systems encompassed by the present subject matter that consist essentially of, or consist of, the recited components, and that there are methods encompassed by the present subject matter that consist essentially of, or consist of, the recited processing steps.

It should be understood that the order of steps or order for performing certain action is immaterial so long as any embodiment of the subject matter described within remains operable. Moreover, two or more steps or actions may be conducted simultaneously

### Acknowledgements

The Applicant wishes to thank Consejo Superior de Investigaciones Científicas and FIUS/UNIVERSITY OF SEVILLE/ for their support and contribution to the development of this work.

### Exemplification

### Example 1 - Complexes and Methods of Preparing Complexes

The following complexes were prepared using various embodiments described herein:

| **Route*** | **Target/Indication** | **Oligo** | **RNA** | **N/P** |
|---|---|---|---|---|
| Systemic (i.v) | Lung | JRL13 | modRNA | 6-12 |
| | Liver | JLJB604 | modRNA | 3 |
| | Spleen | JLJB604+JLJB619 | modRNA | 6 |
| Local (i.m) | Vaccination | JRL13 | saRNA | 6-12 |
| | | JLF102 | saRNA | |
| | | JLF97 | saRNA | |
| | | JLF94 | saRNA | |
| | | JLF93 | saRNA | |
| Local (i.m/s.c) or Systemic (i.v) | Vaccination, Protein-Replacement | JLF99 | modRNA | |
| Local (s.c.) | Vaccination | JLJB619 | saRNA | 6-12 |
| Local (i.m/s.c) | Vaccination, Protein-Replacement | JLF99 + Tween20 | modRNA | 6 |

| | | | | |
|---|---|---|---|---|
| i.m = intramuscular; s.c. = subcutaneous; and i.v. = intravenous | | | | |

The present Example provides an example of how certain complexes comprising an oligosaccharide and a nucleic acid, here, an RNA. In the provided examples, paOS refers to "polycationic amphiphilic oligosaccharides".

As described herein, a calculation of N/P ratio is determined by finding a ratio of positive charges (e.g., either protonated or protonable- or permanent charges of nitrogen atoms of amines/guanidines (N)) in the cationic oligosaccharides and anionic charges (phosphates) of the RNA (P). An amount of RNA and oligosaccharide in a particular complex is therefore determined by the N/P ratio calculation and the desired final RNA concentration of the formulation. The complexation itself is produced upon asymmetrical volume mixing of the RNA containing solution and the oligosaccharide containing solution. For the RNA solution, the required concentration of RNA is prepared by diluting appropriate volume of ddH20, RNA Stock and buffer (6x). The oligosaccharide containing solution is prepared in a separate tube by diluting the oligosaccharide in DMSO to required concentration. Complexation of RNA will take place by mixing RNA-oligosaccharide phases at a volume ratio of 9:1 (RNA: oligosaccharide). The formulated complexes are incubated 5-10 min at room temperature for stabilization. The buffer used for the formulations in all experiments is MGB (MES buffered Glucose (10mM MES, pH6.1, 5% D-Glucose))

### Example 2 - Intravenous Examples

### Example 2a - Targeting Lung Expression

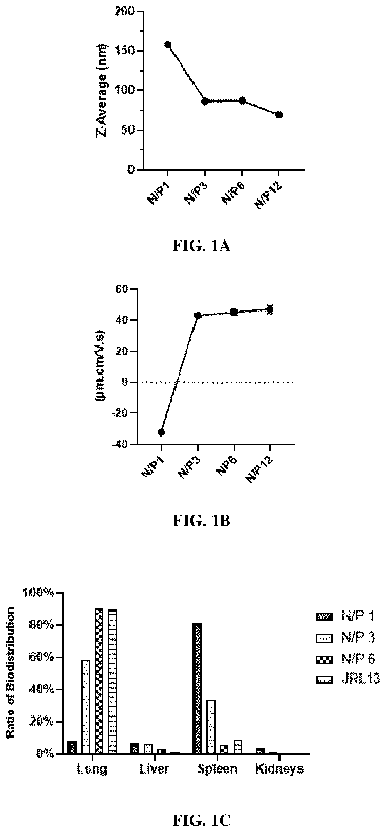

Nine female BALB/c mice are divided into three study groups. All the groups received the same amount of formulated luciferase encoding modRNA (5µg), applied i.v (tail vein). After 6h, ex-vivo luciferase expression from each extracted organ was quantified. All groups received modRNA formulated with JRL13, and the modRNA was formulated at N/P1 or N/P3 or N/P6 or N/P12. All N/P ratios are calculated based on the oligosaccharide-RNA charges ratio. Formulations were complexed in MBG Buffer (final concentration 5% w/v Glucose, 10mM MES, pH 6.1), at RNA final concentration of 0.05mg/ml and the mixing ratio of the RNA containing solution and the oligosaccharide containing solution was 9:1 (v:v) for all the cases. Formulations were characterized prior to injection to confirm hydrodynamic diameter and zeta potential. **FIG. 1A** indicates hydrodynamic diameter of modRNA formulated at different N/P Ratios and different oligosaccharide. **FIG. 1B** indicates zeta potential at different N/P Ratios and different oligosaccharide. **FIG. 1C** indicates quantified ex-vivo luminescence after 6h of injection of modRNA in different organs and at different N/P ratios.

Organ selective expression of modRNA was found to be strongly dependent with the N/P ratio of the JRL13-Formulation. While at N/P1 signal in spleen was observed, with increasing N/P, the ratio of biodistribution shifts completely to lung. At N/P3, there is a mixed expression of lung and spleen, while at N/P 6, >90% of the signal comes from the lungs. In FIG. 1A and in FIG.1B no differences in the surface charge or hydrodynamic diameter can be appreciated. Further, incrementing the N/P above 6 did not alter the biodistribution.

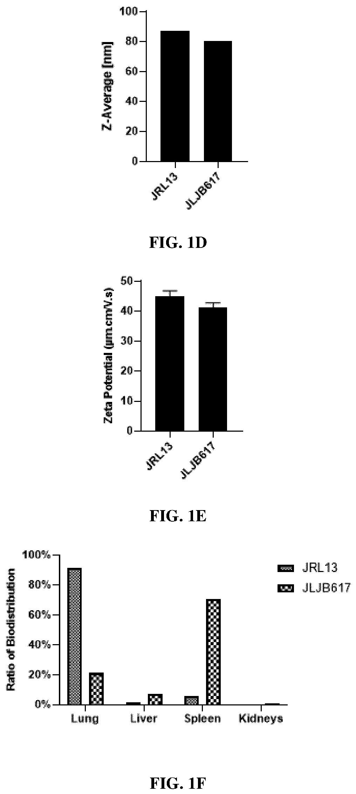

The impact of changing the aliphatic chains in JRL13 was also investigated to elucidate the correlation between this elements in the targeting profile of JRL13. Six female BALB/c mice were divided into two study groups. All the groups received the same amount of formulated luciferase encoding modRNA (5µg), applied i.v (tail vein). After 6h, ex-vivo luciferase expression from each extracted organ was quantified. One group received RNA formulated with JRL13 and the other group received RNA formulated with a further JRL13-derivate where the aliphatic chains have been substituted by longer chains e.g., C10, as observed in JLJB617. All groups received modRNA formulated at N/P6. N/P ratios are calculated based on the oligosaccharide-RNA charges ratio. Formulations were complexed in MBG Buffer (final concentration 5% w/v Glucose, 10mM MES, pH 6.1), at RNA final concentration of 0.05mg/ml and the mixing ratio of the RNA containing solution and the oligosaccharide containing solution was 9:1 (v:v) for all the cases. **FIG. 1D** indicates hydrodynamic diameter of modRNA formulated. **FIG. 1E** indicates zeta potential. **FIG. 1F** indicates quantified ex-vivo luminescence after 6h of injection of modRNA in different organs.

The introduction of longer aliphatic chains (C10, JLJB617) did not affect the overall surface charge or hydrodynamic diameter of the complexes formed by JLJB617 in comparison to JRL13. The biodistribution was, on the other hand, affected by the introduction of a longer aliphatic chain (see FIG. 1F). While JLR13 showed >90% of modRNA expression coming from the lung, this was reduced to only 21% coming from the lung with JLJB617 and in parallel, the spleen expression was increased from 5% in JRL13 to 70% in JLJB617.

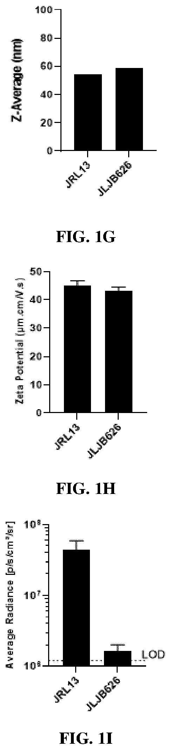

Finally, the impact of the disaccharide scaffold in the targeting profile of JRL13 was investigated. Six female BALB/c mice are divided into two study groups. All the groups received the same amount of formulated luciferase encoding modRNA (5µg), applied i.v (tail vein). After 6h, ex-vivo luciferase expression from each extracted organ was quantified. One group received RNA formulated with JRL13 and the group received RNA formulated with a analogue of JRL13, where the trehalose has been substituted by sucrose-JLJB626-. All groups received modRNA formulated at N/P3. Formulations were complexed in MBG Buffer (final concentration 5% w/v Glucose, 10mM MES, pH 6.1), at RNA final concentration of 0.05mg/ml and the mixing ratio of the RNA containing solution and the oligosaccharide containing solution was 9:1 (v:v) for all the cases. **FIG. 1G** indicates hydrodynamic diameter of modRNA formulated. **FIG. 1H** indicates zeta potential. **FIG. 1I** indicates quantified ex-vivo luminescence after 6h of injection of modRNA in spleen.

### Example 2b ― Targeting Live or Spleen

In the present Example, JLJB604 was formulated with modRNA at two different N/Ps: 3 and 6. The biodistribution was analyzed based on the organ expression of the modRNA encoding luciferase. Six female BALB/c mice are divided into two study groups. All the groups received the same amount of formulated luciferase encoding modRNA (5µg), applied i.v (tail vein). After 6h, ex-vivo luciferase expression from each extracted organ was quantified. All groups received modRNA formulated with JLJB604, where the modRNA was formulated at N/P3 or N/P6. Formulations were complexed in MBG Buffer (final concentration 5% w/v Glucose, 10 mM MES, pH 6.1), at RNA final concentration of 0.05 mg/ml and the mixing ratio of the RNA containing solution and the oligosaccharide containing solution was 9:1 (v:v) for all the cases. Formulations were characterized prior to injection to confirm hydrodynamic diameter and zeta potential. **FIG. 2A** indicates hydrodynamic diameter of modRNA formulated at different N/P Ratios and different oligosaccharides. **FIG. 2B** indicates zeta potential at different N/P Ratios and different oligosaccharides. **FIG. 2C** indicates quantified ex-vivo luminescence after 6h of injection of modRNA in different organs and at different N/P ratios.

### Example 2c ― Targeting Spleen Expression

In the present Example, the combination of JLJB619 and JLJB604 was used at N/P6 to directly formulate modRNA in comparison to a formulation (F12) currently in clinical development and that has been widely published for mRNA expression in spleen. The biodistribution was analyzed based on the organ expression of the modRNA encoding luciferase. Six female BALB/c mice are divided into two study groups. All the groups received the same amount of formulated luciferase encoding modRNA (5µg), applied i.v (tail vein). After 6h, ex-vivo luciferase expression from each extracted organ was quantified. The first group received modRNA formulated with an equimolar mixture of JLJB619 and JLJB604, whereas the modRNA was formulated to a final N/P6. This formulation was prepared in MBG Buffer (final concentration 5% w/v Glucose, 10mM MES, pH 6.1), at RNA final concentration of 0.05mg/ml and the mixing ratio of the RNA containing solution and the oligosaccharide solution was 9:1 (v:v). The second group received modRNA formulated with F12 (a formulation consisting of 3:1 Ratio of DOTMA:DOPE lipid at a final N/P ratio of 0.65), where the N/P ratio is calculated based on the cationic:anionic charges between DOTMA:RNA. F12 was prepared in ddH₂O with a final concentration of 0.112mM of NaCl at a RNA final concentration of 0.05mg/ml. All formulations were characterized prior to injection to confirm hydrodynamic diameter and zeta potential. **FIG. 2D** indicates hydrodynamic diameter of modRNA formulated with the two different formulations. **FIG. 2E** indicates zeta potential of the two different formulations. **FIG. 2F** indicates quantified ex-vivo luminescence after 6h of injection of modRNA in spleen. **FIG. 2G** indicates quantified ex-vivo luminescence after 6h of injection of modRNA in different organs.

### Example 2d ― modRNA Delivery vs. DNA Delivery

Six female BALB/c mice are divided into two study groups. All the groups received the same amount of formulated luciferase encoding modRNA (10µg), applied i.v (tail vein). After 6h, ex-vivo luciferase expression from each extracted organ was quantified. All groups received modRNA formulated at N/P 3, either with JRL13 or JRL45. Furthermore, modRNA was formulated at different N/P ratios with either JRL13 or JRL45 to investigate more extensively the binding properties to modRNA and the zeta-potential of the different formulations with modRNA. Formulations were complexed in MBG Buffer (final concentration 5% w/v Glucose, 10mM MES, pH 6.1), at RNA final concentration of 0.05mg/ml and the mixing ratio of the RNA containing solution and the oligosaccharide containing solution was 9:1 (v:v) for all the cases. Formulations were characterized prior to injection to confirm hydrodynamic diameter and zeta potential. **FIG. 3A** indicates percentage of free RNA quantified by gel electrophoresis of formulated modRNA at different N/P ratios between 0-3 for either JRL13 or JRL45. **FIG. 3B** indicates zeta potential after formulation of modRNA at different N/P ratios with either JRL13 or JRL45. **FIG. 3C** indicates hydrodynamic diameter for formulated modRNA at different N/P ratios with either JRL13 or JRL45. **FIG. 3D** indicates expression of luciferase-enconding modRNA after 24 of transfection of C2C12 cells, N/P 6 formulated modRNA with either JRL13 or JRL45, at 25 ng of RNA/well. Data here represents 3 independent experiments (n=3) with technical triplicates. **FIG. 3E** indicates quantified ex-vivo luminescence in different organs after 6h of injection of 10 µg of modRNA formulated at N/P 3 with either JRL13 or JRL45. **FIG. 3F** indicates quantified ex-vivo luminescence in different organs after 6h of the total flux [p/s] ratio from the JRL13 group to JRL45 group.

JRL45 and JRL13 oligosaccharides are two molecules from the library described in the literature (Garcia Fernández et al. 2019, DOI 10.1039/c9cc04489b) for delivery of pDNA after intra-venous injection. JRL45 is referred to as compound 10 and JRL13 is referred to as compound 8. In the complexes reported by Garcia Fernandez, complexes of oligosaccharide to pDNA had an N/P ratio of 20.

The formulations of Garcia Fernández were prepared in Hepes Buffer (20mM) at pH 7.4, comprising diluting the pDNA in a buffer to desired concentration and diluting oligosaccharide by simple dispersion and mixture. The expression of pDNA is mostly in the liver for JRL13 (compound 8) and lung for JRL45 (compound 10). See FIG. 3G. The present complexes, when they comprise RNA, as seen in FIG. 3E, JRL13 was mostly expressed in spleen and lung while JRL45 had significantly reduced expression (FIG. 3F), and was only observed in spleen. The results obtained here not only contradict the results published, but also highly the impact that appropriate formulation parameters that can lead for molecules previously discarded, to be evaluated as very potent for RNA.

### Example 3 - Intramuscular Delivery

### Example 3a - modRNA and saRNA Delivery and N/P Ratios

Eighteen female BALB/c mice are divided into eleven study groups. All groups received the same amount of formulated luciferase encoding modRNA or saRNA (1µg), applied i.m to each leg. At seven time points (6h, 24h, 72h, 6d, 8d, 20d) in-vivo luciferase expression in applied tissue was measured. Three modRNA groups were formulated with JRL13 at three different N/P Ratios (1, 6, or 12) and three saRNA groups were formulated with JRL13 at three different N/P Ratios (either 1, 6 or 12). N/P ratio is calculated based on the JRL13-RNA charges ratio. Formulations were complexed in MBG Buffer (final concentration 5% w/v Glucose, 10mM MES, pH 6.1), at RNA final concentration of 0.05mg/ml and the mixing ratio of the RNA containing solution and the oligosaccharides containing solution was 9:1 (v:v) for all the cases. Formulations were characterized prior to injection to confirm hydrodynamic diameter and zeta potential. **FIG. 4A** indicates hydrodynamic diameter of complexes formulated at different N/P Ratios and RNA. **FIG. 4B** indicates zeta potential at different N/P Ratios and RNA. **FIG. 4C** indicates quantified bioluminescence over the whole duration of the experiment, per injected N/P ratio of modRNA-JRL13 formulations. **FIG. 4D** indicates quantified bioluminescence over the whole duration of the experiment, per injected N/P ratio of saRNA-JRL13 formulations.

After i.m application of modRNA, only expression could be observed at the negatively charged N/P Ratio (N/P1) while increasing the overall N/P ratio of the formulation lead to total loss of the expression of modRNA. JRL13 formulations comprising modRNA appear to show little difference for complexes size (FIG. 4A) with increasing N/P, although the surface charge was shifted from negative to positive (FIG. 4B). The saRNA formulations, in contrast, showed that size was affected by the N/P ratio. For example, at lower N/P ratios (e.g., N/P 1) the complexes were ~170nm and the size decreased to 90nm nanometers than with higher N/Ps (e.g., N/P of 6-12). saRNA formulations, at the given dose, had the highest expression at N/P 12, opposite to modRNA where with N/P 1, highest modRNA expression was given (FIG. 4C - FIG. 4D).

### Example 3b - Cationic Oligosaccharide Screening

The present example examines different cationic heads to determine impact on intramuscular delivery of saRNA. Forty-eight female BALB/c mice were divided into sixteen study groups. All the groups received the same amount of formulated luciferase encoding saRNA (2µg), applied i.m to each leg. At six time points (6h, 24h, 72h, 6d, 9d, 20d) in-vivo luciferase expression in applied tissue was measured. All groups received saRNA formulated at N/P 6. Each group received saRNA formulated with one of the following oligosaccharides: JLJB619, JLF41, JLF42, JRL13, JLF102, JLF58, JLF120, JLF94, JLF96, JLF97, JLF135, JLF90, JLF130, JLF82, JLF93, JLF99. Formulations were complexed in MBG Buffer (final concentration 5% w/v Glucose, 10mM MES, pH 6.1), at RNA final concentration of 0.1mg/ml and the mixing ratio of the RNA containing solution and the oligosaccharide containing solution was 9:1 (v:v) for all the cases. Formulations were characterized prior to injection to confirm hydrodynamic diameter. **FIG. 5A** indicates hydrodynamic diameter of complexes formulated at N/P6 Ratios with saRNA and different oligosaccharides. **FIG. 5B** indicates quantified bioluminescence over the whole duration of the experiment and normalized to the JRL13-saRNA group. **FIG. 5C** indicates quantified bioluminescence at each measured time point of certain formulations.

While most of the tested formulations with saRNA at N/P 6 had a hydrodynamic diameter that ranged within 50 to 100nm (FIG. 5A). A formulation comprising JLF99 was about 150 nm in diameter. Among the different tested oligosaccharides, at least 4 were identified to be comparable to JRL13: JLF102, JLF94, JLF97 and JLF93 (FIG. 5B). Further, certain combinations of primary amine and secondary amines (e.g., JLF102) demonstrate a positive effect that translated in two-fold signal increment compared to JRL13.

### Example 3c - Screening N/P Ratios for Selected Oligosaccharides

Based on previous observations (e.g., as seen in FIG. 4C), further cationic derivatives were tested with different N/P ratios in order to identify suitable amine types for delivery of modRNA. Oligosaccharides having different type of amines were tested, for example primary amines (JRL13), secondary amine (JLJB619), secondary amines-butanol (JLF58), tertiary amines (JLF41) and quaternary ammonium (JLF42).

Thirty female BALB/c mice are divided into ten study groups. All the groups received the same amount of formulated luciferase encoding modRNA (2µg), applied i.m to each leg. At five time points (6h, 1d, 2d, 3d, 6d) in-vivo luciferase expression in applied tissue was measured. Half of the groups received modRNA formulated at N/P 1 while other half received the modRNA formulated at N/P 6. Two groups received the modRNA formulated with JRL13, two groups with JLJB619, two groups with JLF58, two groups with JLF41 and two groups with JLF42. Formulations were complexed in MBG Buffer (final concentration 5% w/v Glucose, 10mM MES, pH 6.1), at RNA final concentration of 0.1mg/ml and the mixing ratio of the RNA containing solution and the oligosaccharide containing solution was 9:1 (v:v) for all the cases. Formulations were characterized prior to injection to confirm hydrodynamic diameter. **FIG. 6A** indicates hydrodynamic diameter of complexes formulated at different N/P Ratios with modRNA and different oligosaccharides. **FIG. 6B** indicates quantified bioluminescence over the whole duration of the experiment.

In all tested formulations, the lower N/P ratio tested (1) lead to the formation of significantly larger nanoparticles (FIG. 6A). There was a greater difference in particle size between N/P 1 and 6 for JLJB619, JLF58, and JLF41 while the difference between N/P 1 and N/P 6 was smaller in the JRL13 and JLF42 formulations. Without being bound by theory, it is believed that because JRL13 possesses a primary amine which is virtually charged at any physiological pH, and JLF42 has a quaternary ammonium which is in fact a permanent cation, Both groups interact with RNA and have a suitable condensation capability. JLJB619, JLF58, and JLF41 have amines with a pKa within a physiological range. JLF58 and JLF41 comprise amines which are spatially hindered. The bioluminescence, at both tested N/P, was highest in the JLF141 (FIG. 6B). The N/P 6 group was 1.5-fold higher than the N/P 1 group. JRL13 and JLF58 showed no significant difference in the expression of modRNA between both tested N/Ps.

### Example 3d - Screening of Additional Oligosaccharides for modRNA Delivery

Forty-eight female BALB/c mice are divided into sixteen study groups. All groups received the same amount of formulated luciferase encoding saRNA (2µg), applied i.m to each leg. At six time points (6h, d1, d2, d3, d6) in-vivo luciferase expression in applied tissue was measured. All groups received modRNA formulated at N/P 6. Each group received modRNA formulated with one of the following oligosaccharides: JRL13, JLJB619, JLF42, JLF41, JLF128, JLF142, JLF131, JLF58, JLF96, JLF96, JLF97, JLF135, JLF94, JLF90, JLF99, JLF82, JLF82, JLF138. Formulations were complexed in MBG Buffer (final concentration 5% w/v Glucose, 10mM MES, pH 6.1), at RNA final concentration of 0.1mg/ml and the mixing ratio of the RNA containing solution and the oligosaccharides containing solution was 9:1 (v:v) for all the cases. Formulations were characterized prior to injection to confirm hydrodynamic diameter. **FIG. 7A** indicates hydrodynamic diameter of complexes formulated at N/P 6 ratios with modRNA and different oligosaccharides. **FIG. 7B** indicates quantified bioluminescence over the whole duration of the experiment and normalized to the JLF41-modRNA group.

JLF99 shows promising modRNA expression (FIG. 7B). Certain oligosaccharides (JLF142, JLF131, JLF99) formed complexes with modRNA over 100nm.

### Example 3e - Screening Cationic Heads at Different N/P Ratios for modRNA Delivery

Twenty-four female BALB/c mice were divided into eight study groups. All groups received the same amount of formulated luciferase encoding modRNA (5µg), applied s.c right flank. At five time points (6h, 24h, 48h, 72h, 6d) in-vivo luciferase expression in applied tissue was measured. Two groups received modRNA formulated with JRL13, one at N/P 1 and one at N/P 6. Two groups received modRNA formulated with JLJB619, one at N/P 1 and one at N/P 6. Two groups received modRNA formulated with JLJB604, one at N/P 1 and one at N/P 6. The last two groups received modRNA formulated with JLJB605, one at N/P 1 and one at N/P 6. Formulations were complexed in MBG Buffer (final concentration 5% w/v Glucose, 10mM MES, pH 6.1), at RNA final concentration of 0.05mg/ml and the mixing ratio of the RNA containing solution and the oligosaccharide containing solution was 9:1 (v:v) for all the cases. Formulations were characterized prior to injection to confirm hydrodynamic diameter and zeta potential. **FIG. 8A** indicates hydrodynamic diameter of complexes formulated at different N/P Ratios. **FIG. 8B** indicates zeta potential at different N/P Ratios of injected modRNA formulations. **FIG. 8C** indicates quantified bioluminescence over the whole duration of the experiment, per injected oligosaccharide formulation.

### Example 3f ― Screening Aliphatic Chain Length for modRNA Delivery

Thirty-six female BALB/c mice are divided into twelve study groups. All the groups received the same amount of formulated luciferase encoding modRNA (5µg), applied s.c right flank. At seven time points (6h, 24h, 72h, 6d, 9d, 20d) in-vivo luciferase expression in applied tissue was measured. Three groups received oligosaccharide bearing primary amines with different aliphatic chains lengths, i.e., C₆ (JRL13), C₁₀ (JLJB617), C₁₄ (JLJB618). Three groups received oligosaccharides bearing secondary amines with different aliphatic chains lengths, i.e C₆ (JLJB619)- C₁₀ (JLF43)- C₁₄ (JLF31). Three groups received oligosaccharides bearing tertiary amines with different aliphatic chains lengths, i.e C₆ (JLJB604)- C₁₀ (JLF41)- C₁₄ (JFL25). Three groups received oligosaccharides bearing quaternary amines with different aliphatic chains lengths, i.e., C₆ (JLJB605), C₁₀ (JLF42), C₁₄ (JLF26). All formulations were tested at N/P ratio 6. Formulations were complexed in MBG Buffer (final concentration 5% w/v Glucose, 10mM MES, pH 6.1), at RNA final concentration of 0.05mg/ml and the mixing ratio of the RNA containing solution and the oligosaccharides containing solution was 9:1 (v:v) for all the cases. Formulations were characterized prior to injection to confirm hydrodynamic diameter and zeta potential. **FIG. 9A** indicates hydrodynamic diameter of complexes formulated. **FIG. 9B** indicates quantified bioluminescence over the whole duration of the experiment, per injected oligosaccharides formulation.

### Example 3g - Screening Aliphatic Chain Length for saRNA Delivery

Thirty-six female BALB/c mice are divided into twelve study groups. All the groups received the same amount of formulated luciferase encoding saRNA (5µg), applied s.c right flank. At seven time points (6h, 24h, 72h, 6d, 8d, 20d) in-vivo luciferase expression in applied tissue was measured. Three groups received oligosaccharides bearing primary amines with different aliphatic chains lengths, i.e., C₆ (JRL13), C₁₀ (JLJB617), C₁₄ (JLJB618). Three groups received oligosaccharides bearing secondary amines with different aliphatic chains lengths, i.e., C₆ (JLJB619), C₁₀ (JLF43), C₁₄ (JLF31). Three groups received oligosaccharides bearing tertiary amines with different aliphatic chains lengths, i.e., C₆ (JLJB604), C₁₀ (JLF41), C₁₄ (JFL25). Three groups received oligosaccharides bearing quaternary amines with different aliphatic chains lengths, i.e., C₆ (JLJB605), C₁₀ (JLF42), C₁₄ (JLF26). All formulations were tested at N/P ratio 6. Formulations were complexed in MBG Buffer (final concentration 5% w/v Glucose, 10mM MES, pH 6.1), at RNA final concentration of 0.05mg/ml and the mixing ratio of the RNA containing solution and the oligosaccharides containing solution was 9:1 (v:v) for all the cases. Formulations were characterized prior to injection to confirm hydrodynamic diameter and zeta potential. **FIG. 10A** indicates hydrodynamic diameter of complexes formulated. **FIG. 10B** indicates quantified bioluminescence over the whole duration of the experiment, per injected oligosaccharides formulation. **FIG. 10C** indicates quantified response of CD8 positive T cells from spleen samples of treated mice 20 days after s.c application. The CD8 response to luciferase peptides presented by murine MHC is quantified via IFN release in an ELISPOT. An irrelevant peptide serves as control to show that the CD8 answer to the luciferase peptide is specific.

### Example 3h - N/P Ratio Effect for Delivery of modRNA and saRNA

Eighteen female BALB/c mice are divided into six study groups. All the groups received the same amount of formulated luciferase encoding saRNA (5µg) or modRNA (5µg), applied s.c right flank. At seven time points (6h, 24h, 72h, 6d, 8d, 20d) in-vivo luciferase expression in applied tissue was measured. Three groups received modRNA formulated with JLJB619 at three different N/Ps (3, 6, and 12) and three further groups received saRNA formulated with JLJB619 at three different N/Ps (3, 6, and 12). Three groups received oligosaccharides bearing secondary amines with different aliphatic chains lengths, i.e C6 (JLJB619)- C10 (JLF43)- C14(JLF31). Three groups received oligosaccharides bearing tertiary amines with different aliphatic chains lengths, i.e C6 (JLJB604)- C10 (JLF41)- C14(JFL25). All N/P ratios are calculated based on the oligosaccharides-RNA charges ratio. Formulations were complexed in MBG Buffer (final concentration 5% w/v Glucose, 10mM MES, pH 6.1), at RNA final concentration of 0.05mg/ml and the mixing ratio of the RNA containing solution and the JLJB619 containing solution was 9:1 (v:v) for all the cases. Formulations were characterized prior to injection to confirm hydrodynamic diameter and zeta potential. **FIG. 11A** indicates hydrodynamic diameter of complexes formulated at different N/P Ratios and RNA. **FIG. 11B** indicates zeta potential at different N/P Ratios and RNA. **FIG. 11C** indicates quantified bioluminescence over the whole duration of the experiment for modRNA groups. **FIG. 11D** indicates quantified bioluminescence over the whole duration of the experiment for saRNA groups.

### Example 3i -Immunization against H1N1/Cal07/HA after saRNA Delivery

Fifteen female BALB/C mice were divided into 3 groups. One group received only buffer as a negative control and two further groups received 2µg of formulated saRNA coding for Hemagglutinin (HA) protein of the influenza strain California/7/2009 complexed with JLJB619. The remaining mice received 2µg of formulated saRNA coding for Hemagglutinin (HA) protein of the influenza strain California/7/2009 complexed with LNP (DODMA, Cholesterol, DOPE and PEG200-ceramide C16 at a molar ratio of 40:48:10:2). All saRNA formulations were applied subcutaneously. The saRNA with JLJB619 was formulated at N/P 6. The saRNA formulated with the LNP was prepared at N/P 4. The N/P ratio for the LNP is determined by the DODMA/RNA ratio.

While the JLJB619 formulations was complexed in MBG Buffer (final concentration 5% w/v Glucose, 10mM MES, pH 6.1), at RNA final concentration of 0.05mg/ml and the mixing ratio of the RNA containing solution and the paOs containing solution was 9:1 (v:v); the LNP formulation was produced in PBS following standard LNP production method widely described in the literature. See Nogueira, et al., ACS Appl. Nano. Mater., 3(11):10634-10645 (2020). The LNP is produced at a mixing ratio of lipid mixture in ethanol mixed at a 3:1 ratio with saRNA in a citrate buffer (pH 4.5) and at a speed of 12mUmin. The LNP was then dialyzed in PBS for 3 hours. The animals were subjected to non-invasive serological surveillance over 49 days at different time points (d0, d14, d21, d28, d49) and the spleen was extracted at the end of experiment for quantification of CD4/CD8 T-Cell response to Influenza HA peptides. Anti-Influenza HA antibodies in serum were quantified by enzyme-linked immunosorbent assay. CD4/CD8 T-Cell response was quantified by via IFN -ELISPOT of Splenocytes. **FIG. 12A** indicates hydrodynamic diameter of complexes formulated at different N/P Ratios and RNA. **FIG. 12B** shows serological levels of specific IgG against HA during the curse of experiment at a serum dilution of 1:300. **FIG. 12C** shows end-point titration of the serological levels of specific IgG against HA. **FIG. 12D** indicates the CD8/CD4 response against HA-peptide pools.

### Example 3j - Cationic Oligosaccharide Combination Complexes

The present example considers complexes comprising JLF99 and modRNA at N/P 6 in combination with a polysorbate (e.g., Tween20 and Tween40). Nine female BALB/c mice are divided into 3 study groups. All the groups received the same amount of formulated luciferase encoding modRNA (2µg), applied i.m to each leg. At five time points (6h, 1d, 2d, 3d, 6d) in-vivo luciferase expression in applied tissue was measured. The first group received modRNA formulated exclusively with JLF99, the second group received modRNA formulated with a mixture of JLF99+Tween20 (1:0.5 molar ratio) and the third group received modRNA formulated with a mixture of JLF99+ Tween20 (1:1 molar ratio). All groups received modRNA formulated at N/P6. Formulations were complexed in MBG Buffer (final concentration 5% w/v glucose, 10mM MES, pH 6.1), at an RNA final concentration of 0.1mg/ml and the mixing ratio of the RNA containing solution and the oligosaccharide/Tween20 containing solution was 9:1 (v:v) for all the cases. Formulations were characterized prior to injection to confirm hydrodynamic diameter. **FIG. 13A** illustrates hydrodynamic diameter of complexes formulated at N/P 6 but with different surfactants and with a surfactant-to-JLF99 ratio between 0:1 and 0.5:1 (where C14-pMeOx45 is C₁₄ alkyl-poly(2-methyl-2-oxazoline)₄₅, C14pSar23 is C14 alkyl-poly(sarcosine)₂₃, and Tween20 is polysorbate 20). **FIG. 13B** illustrates hydrodynamic diameter of complexes formulated at N/P 6 with either Tween20 or Tween40 and within a surfactant-to-JLF99 ratio between 0 and 1.5. **FIG. 13C** illustrates expression of luciferase-enconding modRNA after 24 of transfection of C2C12 cells, N/P6 formulated modRNA with either Tween20 or Tween40 at different surfactant-to-JLF99 ratio, at 100ng of RNA/well. Data here represents 3 independent experiments (n=3) with technical triplicates. **FIG. 13D** illustrates expression of luciferase-encoding modRNA after 24 of transfection of HepG2 cells, N/P6 formulated modRNA with either Tween20 or Tween40 at different surfactant-to-JLF99 ratio, at 100ng of RNA/well. Data here represents 3 independent experiments (n=3) with technical triplicates. **FIG. 13E** illustrates hydrodynamic diameter of complexes formulated at different N/P6 (where T20 refers to Tween20). **FIG. 13F** illustrates quantified bioluminescence over the whole duration of the experiment represented as area under the curve (where T20 refers to Tween20). **FIG. 13G** illustrates quantified bioluminescence over the whole duration of the experiment (where T20 refers to Tween20). **FIG. 13H** shows bioluminescence of on the ventral axis after 6hours of injection of modRNA into each group (where T20 refers to Tween20).

### Example 3k - Intramuscular Vaccination with Cationic Oligosaccharides and saRNA

Forty female BALB/c mice are divided into six groups. One group receives only buffer as a negative control and five groups receive 1µg of formulated sarNA condig for Hemagglutinin (HA) protein of the influenza strain California/7/2009. All saRNA formulations are applied i.m in one of the legs. All saRNA groups were formulated at N/P12 but with different oligosaccharides (JRL13, JLF102, JLF97, JLF94) or with linear polyethylenimine 22.5kDa (AVROXA, Ghent, Belgium) as a benchmark. The N/P ratio for the PEI formulated saRNA is determined by a secondary amines:RNA charge ratio. Formulations are complexed in MBG Buffer (final concentration 5% w/v Glucose, 10mM MES, pH 6.1), at RNA final concentration of 0.05mg/ml and the mixing ratio of the RNA containing solution and the cationic oligosaccharide containing solution was 9:1 (v:v) for all the cases. The animals will be subjected to non-invasive serological surveillance over 49 days at different time points (d0, d14, d21, d28, d49) and spleen will be extracted for quantification of CD4/CD8 T-Cell response to Influenza HA peptides. Anti-Influenza HA antibodies in serum will be quantified by enzyme-linked immunosorbent assay. Virus neutralizing titers will be quantified by VNT Assay. CD4/CD8 T-Cell response was quantified by via IFN - ELISPOT of Splenocytes.

### Example 4 - Synthetic Examples

All reagents and solvents used in the preparations disclosed in the following examples were purchased from commercial sources and used without further purification, unless otherwise specified. Thin-layer chromatography (TLC) was carried out on aluminum sheets coated with Silica gel 60 F₂₅₄ Merck with visualization by UV light (λ 254 nm) and by charring with 10% ethanolic H₂SO₄, 0.1% ethanolic ninhydrin and heating at 100 °C. Column chromatography was carried out on Silice 60 A.C.C. Chromagel (SDS 70-200 and 35-70 µm). The structure of all compounds described herein, including synthetic intermediates and precursors, was confirmed by ¹H and ¹³C nuclear magnetic resonance (NMR) and mass spectrometry (MS). NMR experiments were performed at 300 (75.5), and 500 (125.7, 202) MHz with Bruker 300 ADVANCE and 500 DRX. 1D TOCSY, 2D COSY, HMQC and HSQC experiments were used to assist on NMR assignments. Mass spectra (High-resolution mass spectra) were carried out on a Bruker Daltonics Esquire6000^{™} (LTQ-Orbitrap XL ETD). The samples were introduced via solid probe heated from 30 to 280 °C. ESI as ionization source (Electrospray Ionization) was used to which methanol was used as solvent. The samples were introduced via direct injection using a Cole-Parmer syringe at a flow rate of 2 µl/min. Ions were scanned between 300 and 3000 Da with a scan speed of 13000 Da/s at unit resolution using resonance ejection at the multipole resonance of one-third of the radio frequency (Ω = 781.25 kHz).

### Methods

Scheme 1 depicts synthesis of a compound of formula I wherein A is A¹, R¹ and R² are both -C(O)-R^{a}, X¹ and X² are both -S-, Y¹ and Y² are both ―(CH₂)₂- and Z¹ and Z² are NH₃⁺ (compounds **4T** in Scheme 1). Scheme 1 also shows the general routes (routes A1, A2, B1 and B2) implemented for the syntheses of a compound of formula I wherein A is A¹, R¹ and R² are both ―C(O)-R^{a}, X¹ and X² are both -S-, Y¹ and Y² are both - (CH₂)₂-NHC(S)NH-(CH₂)₂― and Z¹ and Z² are identical (compounds **6T** in Scheme 1):

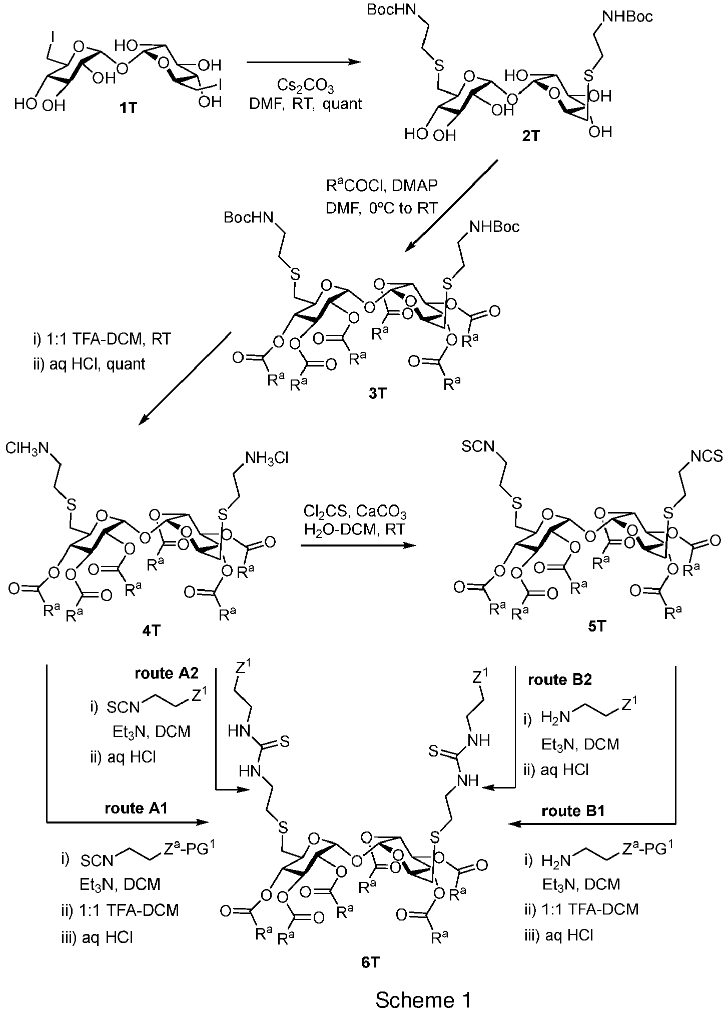

Scheme 2 depicts synthesis of a compound of formula I wherein A is A², R¹ and R² are both ―C(O)--R^{a}, X¹ and X² are both -S-, Y¹ and Y² are both ―(CH₂)₂- and Z¹ and Z² are NH₃⁺ (compounds **4S** in Scheme 2). Scheme 2 also shows the general routes (routes A1 and A2) implemented for the syntheses of a compound of formula I wherein A is A², R¹ and R² are both ―(C=O)R^{a}, X¹ and X² are both -S- and Y¹ and Y² are both ―(CH₂)₂-NHC(S)NH-(CH₂)₂― and Z¹ and Z² are identical:

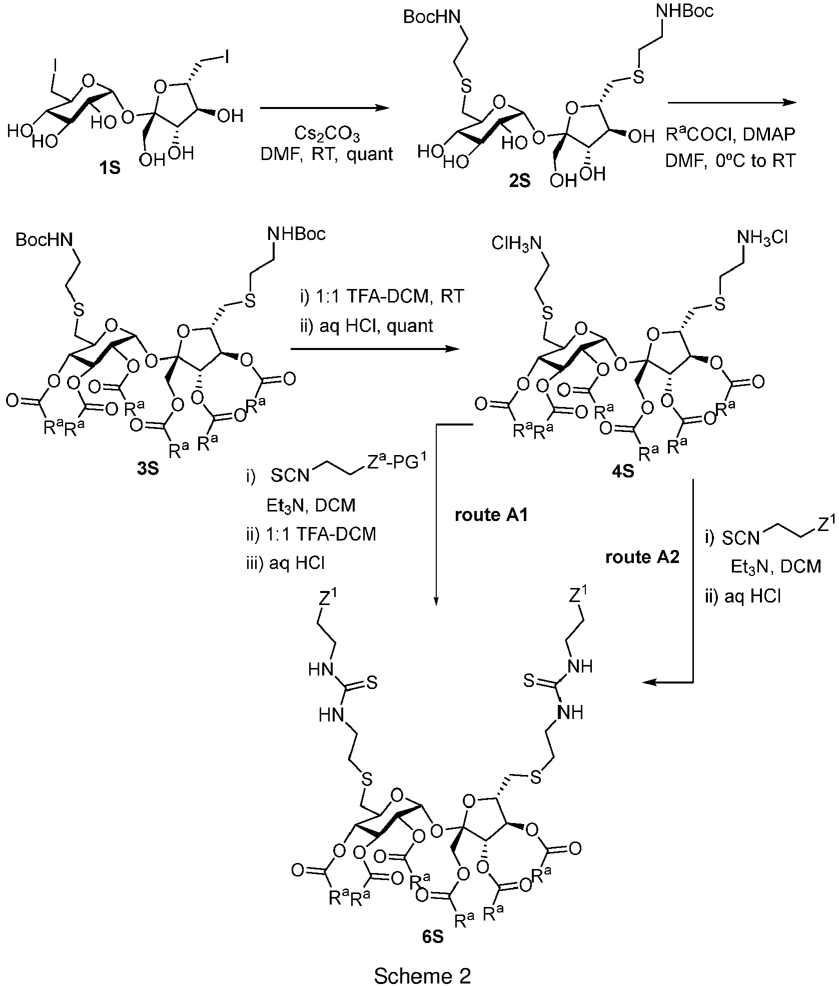

Scheme 3 depicts the synthesis of a compound of formula I wherein A is A¹, R¹ and R² are both ―C(O)R^{a}, X¹ and X² are both -S-, Y¹ and Y² are both ―(CH₂)₂- and Z¹ and Z² are -N(CH₃)H₂⁺ (compounds **8T** in Scheme 3) or -N(CH₃)₂H⁺ (compounds **9T** in Scheme 3):

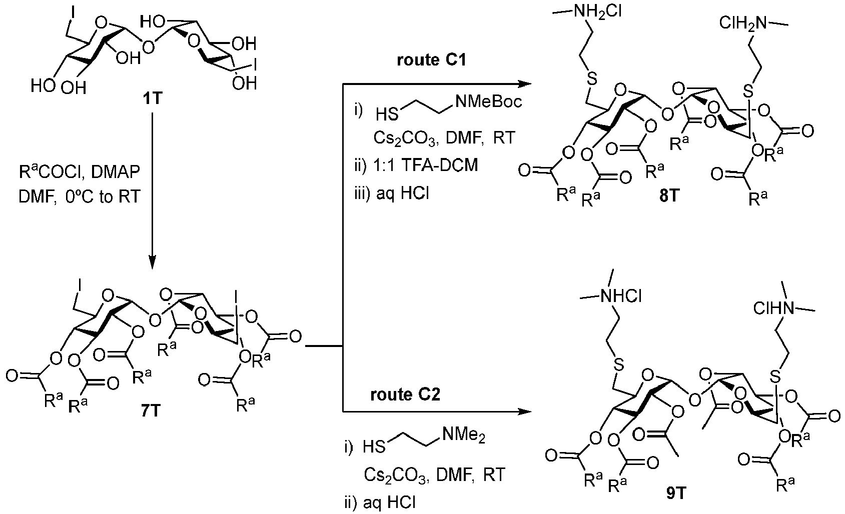

In some embodiments, synthesis of a compound of formula I wherein R¹ and R² are both ―C(O)-R^{a} comprises the following lipid reagents:

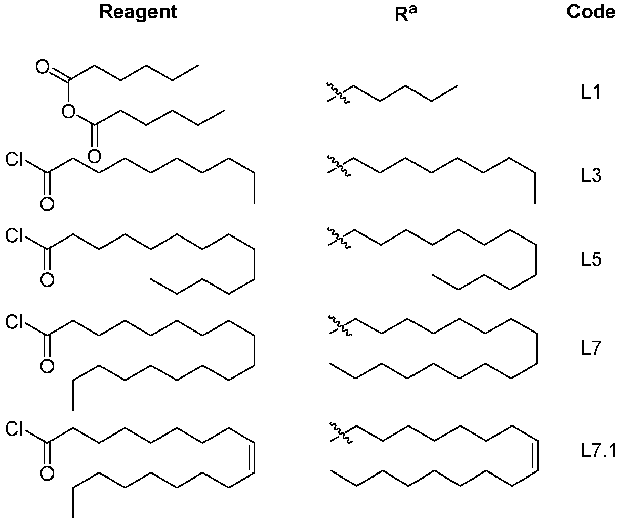

In exemplary embodiments, the synthesis of the compounds of formula I according to Schemes 1 or 2 involved the following isothiocyanate or amine reagents in the reaction step leading to the installation of the cationic heads Z¹ and Z²:

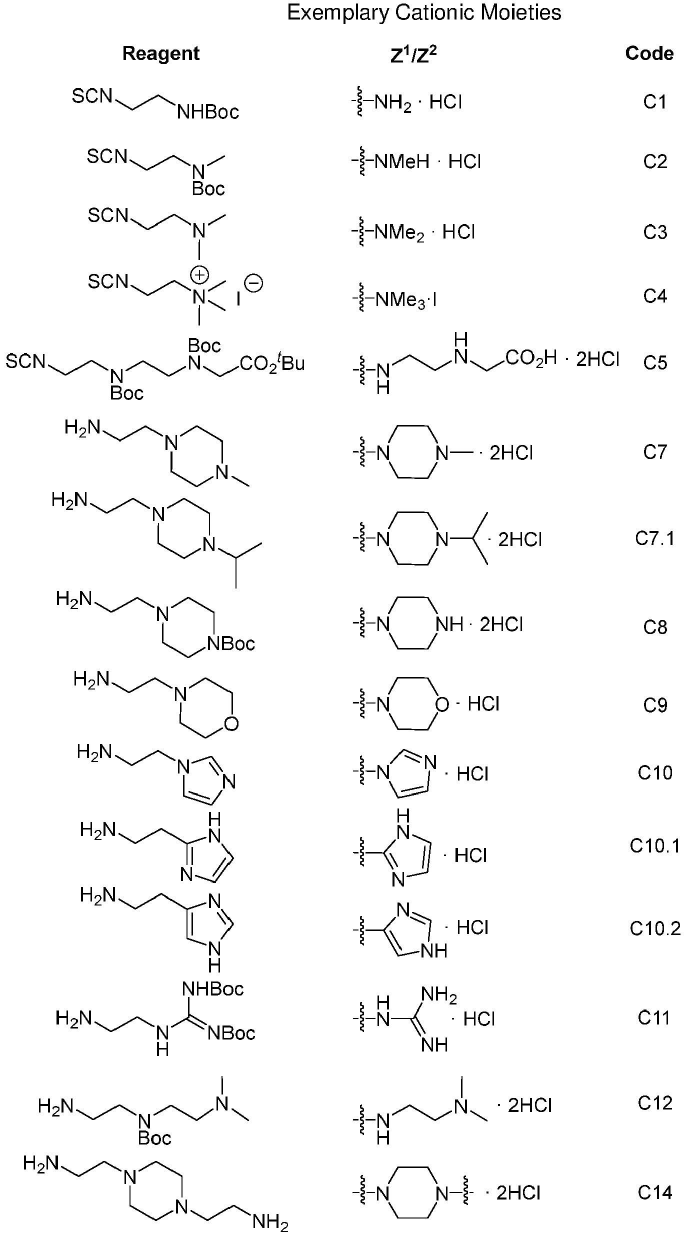

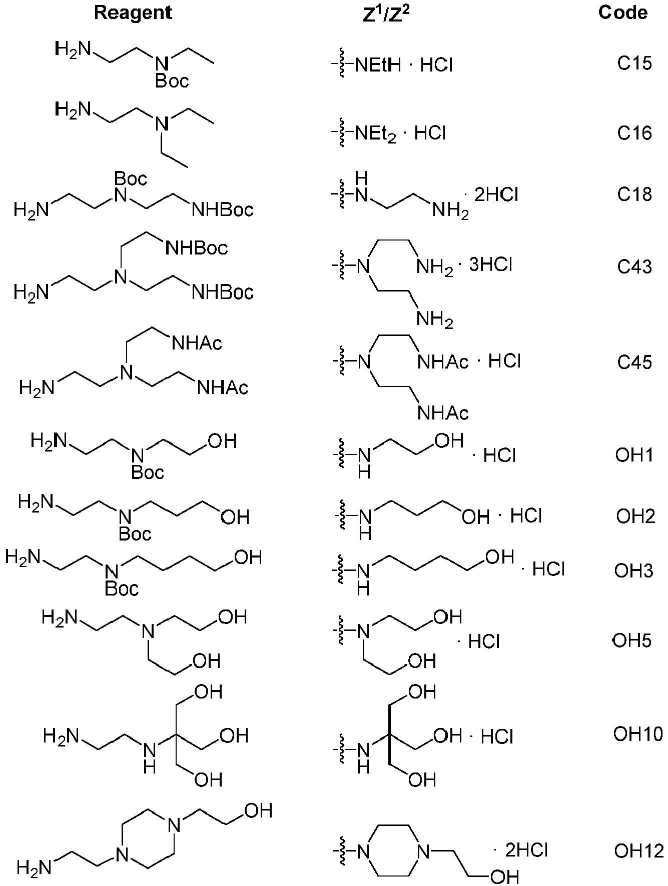

### Materials

In some exemplary embodiments, the following precursors used in the synthesis of specific compounds described herein were prepared according to known methods: 6,6'-Diiodo-6,6'-dideoxy-α,α'-trehalose (1T) was prepared following the procedure described in the literature. J. M. Garcia Fernandez, C. Ortiz Mellet, J. L. Jiménez Blanco, J. Fuentes Mota, A. Gadelle, A. Coste-Sarguet, J. Defaye, Carbohydr. Res. 1995, 268, 57-71.

6,6'-Diiodo-6,6'-dideoxy-sucrose **(1S)** was prepared according to the procedure described in the literature. *See* J. M. Garcia Fernandez, A. Gadelle, J. Defaye, Carbohydr. Res. 1994, 265, 249-269.

As used herein, an arrow followed by a code between parentheses, e.g., "(→- **code**)" indicates the lipophilic tails (R¹ and R² substituents in a compound of formula I) or cationic heads (Z¹ and Z² substituents in a compound of formula I) that the preceding reagent provided during the synthesis. E.g., "hexanoic anhydride (→- **L1**)" is intended to refer to moiety L1, as described in the tables above, was bonded to a compound through use of hexanoic anhydride.

Hexanoic anhydride (→- **L1**) and decanoyl (→- **L3**), myristoyl (→- **L5**), stearoyl (→- **L7**) and oleoyl (→- **L7.1**) chlorides were purchased from commercial sources. 2-(*N*-*tert*-Butoxycarbonylamino)ethanethiol **(C1SH)**, *2-*(*N,N-*dimethylamino)ethylisothiocyanate (→- **C3**), 2-(4-methylpiperazin-1-yl)ethanamine (→-**C7),** 2-(4-isopropylpiperazin-1-yl)ethanamine (→- **C7.1**), *tert*-butyl 4-(2-aminoethyl)piperazine-1-carboxylate (→- **C8**), 2-morpholinoethanamine (→- **C9**), 2-(1*H-*imidazol-1-yl)ethanamine (→- **C10**), 2-(1*H*-imidazol-2-yl)ethanamine (→- **C10.1**)**,** 2-(1*H-*imidazol-4-yl)ethan-1-amine (→- **C10.2),** 2,2'-(piperazine-1,4-diyl)diethanamine (→-**C14),** *N*,*N*-diethylethylenediamine (→- **C16),** *N*,*N*-bis(2-hydroxyethyl)ethylenediamine (→- **OH5),** 2-((2-aminoethyl)amino)-2-(hydroxymethyl)propane-1,3-diol (→- **OH10),** 2-(4-(2-aminoethyl)piperazin-1-yl)ethanol (→ **OH12),** 2*-*[*N*-methyl*-N-*(*tert-*butoxycarbonyl)amino]ethanethiol **(C2SH),** and 2-(*N*,*N*-dimethylamino)ethanethiol **(C3SH)** were purchased from commercial sources.

2-(*N*-*tert*-Butoxycarbonylamino)ethylisothiocyanate (→ **C1**), D. M. Kneeland, K. Ariga, V. M. Lynch, C. Y. Huang, E. V. Anslyn, J. Am. Chem. Soc. 1993, 115, 10042-10055. 2-(*N*-methyl-*N*-*tert*-butoxycarbonylamino)ethylisothiocyanate (→ **C2),** T. Kim, Y.-J. Kim, I.-H. Han, D. Lee, J. Ham, K. S. Kan, J. W. Lee, Bioorg. Med. Chem. Lett. 2015, 25, 62-66. 2-(*N,N,N*-trimethylamino)ethylisothiocyanate (→ **C4**), Y.-J. Ghang, J. J. Lloyd, M. P. Moehlig, J. K. Arguelles, M. Mettry, X. Zhang, R. R. Julian, Q. Cheng, R. J. Hooley, Langmuir 2014, 30, 10160-10166. 2-(2-aminoethyl)-1,3-di-(*tert-*butoxycarbonyl)guanidine (→- **C11**), S. M. Hickey, T. D. Ashton, J. M. White, J. Li, R. L. Nation, H. Y. Yu, A. G. Elliott, M. S. Butler, J. X. Huang, M. A. Cooper, F. M. Pfeffer, RSC Adv. 2015, 5, 28582-28596. and *N*,*N*-bis(2-acetamidoethyl)ethylenediamine (→-**C45)** were prepared according to the described procedures. L. Liu, M. Rozenman, R. Breslow, J. Am. Chem. Soc. 2002, 124, 12660-12661.

*N*^{I},*N*^{I}-dimethyl-*N*^{II}-*tert*-butoxycarbonyl-diethylenetriamine (→- **C12**), *N*^{I}*-*ethyl-*N*^{I}*-*(*tert-*butoxycarbonyl)ethylenediamine (→ **C15**), *N*^{I}*,N*^{II}*-*bis(*tert-*butoxycarbonyl)diethylenetriamine (→ **C18**), *N*-(2-hydroxyethyl)-*N*-(*tert-*butoxycarbonyl)ethylenediamine (→ **OH1**), *N*-(3-hydroxypropyl)-*N*-(*tert-*butoxycarbonyl)ethylenediamine (→- **OH2)** and *N*-(4-hydroxybutyl)-*N*-(*tert-*butoxycarbonyl)ethylenediamine (→- **OH3)** were synthesized by sequential primary amine trifluoroacetylation with ethyl trifluoroacetate, carbamoylation of the remaining amino groups with Boc₂O and trifluoroacetamide hydrolysis from commercially available *N*^{I},*N*^{I}-dimethyl-diethylenetriamine, *N*-ethyl-ethylenediamine, diethylenetriamine, *N*-(2-hydroxyethyl)ethylenediamine, N-(3-hydroxypropyl)ethylenediamine and *N*-(4-hydroxybutyl)ethylenediamine, respectively. *N*,*N*-bis[2-(*tert*-butoxycarbonylamino)ethyl]ethylenediamine (→- **C43)** was prepared by sequential primary amine tritylation with trityl chloride, carbamoylation of the remaining amino groups with Boc₂O and trityl hydrolysis from commercially available tris(2-aminoethyl)amine. *N*^{I}-(*tert*-Butoxycarbonylmethyl)-*N*^{II}-(2-isothiocyanatoethyl)-ethylenediamine (→- **C5)** was synthesized according to the following scheme (Scheme 4) from commercially available diethylenetriamine.

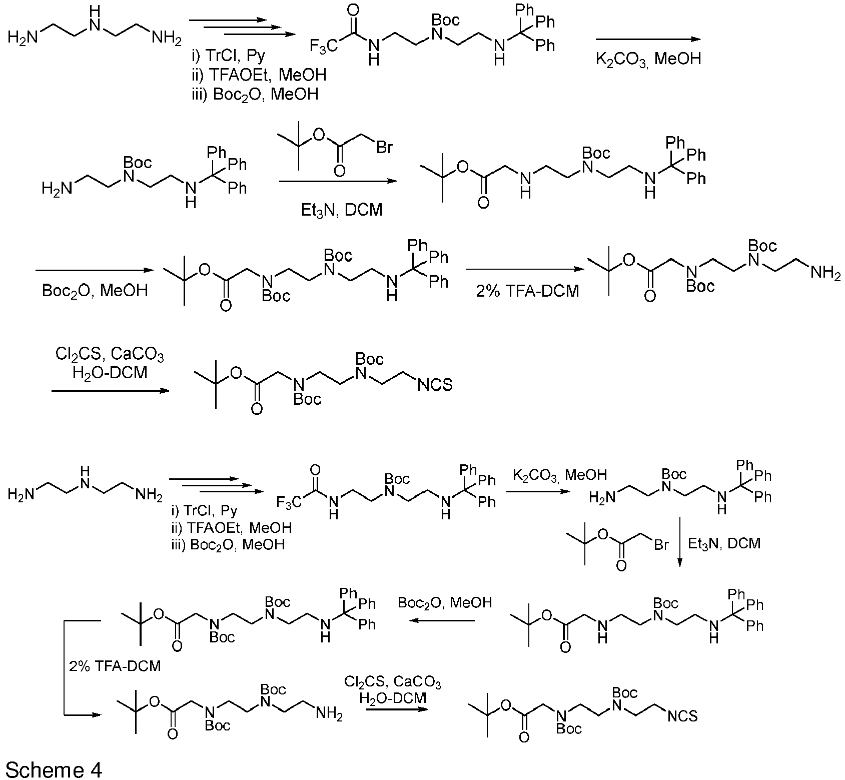

The synthetic intermediates **2T, 2S, 3T** and **3S** in the reaction sequences depicted in Schemes 1 and 2 were prepared as described below:
**6,6'-Bis[2-(*tert-*butoxycarbonylamino)ethylthio)-α,α'-trehalose (2T).** To a mixture of diiodo-trehalose **1T** (14.3 g, 25.44 mmol) and Cs₂CO₃ (23.45 g, 71.23 mmol) in anhydrous DMF (150 mL) under N₂ atmosphere, a solution of 2*-*(*N-tert-*butoxycarbonylamino)ethanethiol **(C1SH**, 13.6 mL, 76.32 mmol) in anhydrous DMF (100 mL) under N₂ was added. The reaction mixture was stirred at RT overnight and the solid remaining in suspension was decanted and filtrated off. The solvent was then removed under reduced pressure and the resulting residue was triturated with Et₂O (500 mL), upon which a sticky solid evolved. The residue was filtrated and triturated with EtOH (250 mL) and the remaining solid again filtrated off. The solvents were finally evaporated to dryness to furnish analytically pure compound **2T** in virtually quantitative yield (16.8 g). Chemical Formula: C₂₆H₄₈N₂O₁₃S₂. Molecular Weight: 660.26. ESI-MS (*mlz*) 705.51 ([M + HCO₂]⁻).

6,6'-Bis[2-(tert-butoxycarbonylamino)ethylthio)sucrose (2S). To a mixture of diiodo-sucrose 1S (1.07 g, 1.9 mmol) and Cs2CO3 (1.75 g, 5.33 mmol) in anhydrous DMF (30 mL) under N2 atmosphere, 2-(tert-butoxycarbonylamino)ethylmercaptane (1.0 mL, 5.7 mmol) was added. The reaction mixture was stirred at RT overnight. The solvent was then removed under reduced pressure and the resulting residue was triturated with Et2O (100 mL) upon which a sticky solid evolved. The residue was filtrated and triturated with EtOH (50 mL) and the remaining solid again filtrated off. The solvents were finally evaporated to dryness to furnish analytically pure compound 2S in virtually quantitative yield (1.25 g). Chemical Formula: C26H48N2O13S2. Molecular Weight: 660.26. ESI-MS (m/z) 705.51 ([M + HCO2]-).

**General procedure for exhaustive hydroxyl acylation of 2T and 2S to give reaction intermediates 3T and 3S.** To a solution of the corresponding acylating agent (→- **L1** to **L7.1,** 46 mmol) in anhydrous DMF (20 mL) under N₂ inert atmosphere at 0 ºC, a solution of **2T** or **2S** (3.37 g, 5.1 mmol) and DMAP (11.3 g, 92 mmol) in anhydrous DMF (100 mL) was dropwise added over a period of 10 min. The reaction mixture was allowed to warm to RT over 2 h and further stirred overnight. The reaction was then diluted in a 1:1 water-DCM mixture (400 mL). The organic layer was decanted and further washed with water (100 mL), 2 N H₂SO₄ (2 x 100 mL), saturated NaHCO₃ (100 mL) and water (100 mL), dried over MgSO₄ or Na₂SO₄, filtrated and evaporated under reduced pressure. The resulting syrupy residue was purified by column chromatography using the eluent indicated in each case to give the corresponding **3T** or **3S** reaction intermediate with R^{a} substituents as defined by the codes **L1** to **L7.1** in the above lipid reagent list.

| | | |
|---|---|---|
| Compound **3T-L1** | eluent 1:4 EtOAc-petroleum ether | yield 82% |
| Compound **3T-L3** | eluent 1:10→1:3 EtOAc-petroleum ether | yield 54% |
| Compound **3T-L5** | eluent 1:10→1:5 EtOAc-petroleum ether | yield 63% |
| Compound **3T-L7** | eluent 1:10→1:5 EtOAc-petroleum ether | yield 34% |
| Compound **3T-L7.1** | eluent 1:10→1:5 EtOAc-petroleum ether | yield 39% |
| Compound **3S-L1** | eluent 1:4 EtOAc-petroleum ether | yield 72% |
| Compound **3S-L3** | eluent 1:10→1:3 EtOAc-petroleum ether | yield 17% |

**General procedure for *tert*-butoxycarbonyl group hydrolysis in reaction intermediates 3T (→- 4T) and 3S** (→- **4S).** To a solution of **3T** or **3S** (1 mmol) in DCM (10 mL), at RT, trifluoroacetic acid (TFA, 10 mL) was added. The reaction mixture was stirred at RT for 1 h, then the solvents were evaporated under reduced pressure and the traces of acid were eliminated by repeated coevaporation with water. The resulting residue was lyophilized from 10-mM HCI to furnish the corresponding diamines **4T** and **4S,** as hydrochloride salts, in quantitative yield.

In some preferred embodiments, the following compounds of formula I featuring structure **4T** were synthesized according to Scheme 1 following the above procedure: JLF19, JLF20, JLF23, JLF46.

In some preferred embodiments, the following compounds of formula I featuring structure **4S** were synthesized according to Scheme 1 following the above procedure: JLF50, JLF62.

**General procedure for the synthesis of diisothiocyanate reaction intermediates 5T in Scheme 1.** To a solution of **4T** (1 mmol) in a heterogeneous mixture of water and DCM (1:1, mL), CaCO₃ (0.48 g, 4.8 mmol) and Cl₂CS (0.22 ml, 2.4 mmol) were sequentially added. The mixture was vigorously stirred at RT for 1 h and then transferred to a decantation funnel and diluted with water and DCM (1:1, 100 mL). The organic layer was decanted and washed with water (50 mL) and saturated aq NaHCO₃ (50 mL), dried over MgSO₄ or Na₂SO₄, filtrated and evaporated under reduced pressure to furnish the target diisothiocyanate reaction intermediates **5T**, with R^{a} substituents as defined by the codes **L1** or **L3** in the **Lipid reagent list:**
Compound **5T-L1:** Yield 99%. Chemical Formula: C₅₄H₈₈N₂O₁₅S₄. MW: 1133.54. ESI-MS *(m*/*z)* 1155.58 ([M + Na]⁺).
Compound **5T-L3):** Yield 90%. Chemical Formula: C₇₈H₁₃₆N₂O₁₅S₄. MW: 1470.19. ESI-MS (*m*/*z*) 1514.87 ([M + HCO₂]⁻).

General procedure for the synthesis of a compound of formula I according to Schemes 1 and 2, routes A1, A2, B1 and B2.

**Route A1 (schemes 1 or 2):** To a solution of the corresponding diamine dihydrochloride **(4T** or **4S,** 0.1 mmol) in DCM (10 mL) and triethylamine (Et₃N, 0.3 mmol, 41 µL), a solution of the corresponding isothiocyanate reagent SCN-(CH₂)₂-Z^{a}-PG¹ (0.3 mmol) in DCM (5 mL) were sequentially added. The reaction mixture was stirred at RT for 16 h while monitoring pH to ensure it remains basic (In case pH is found below neutrality, it should be readjusted to slightly basic (pH ca. 8) by addition of aliquots of Et₃N). Upon reaction completion (TLC revealed the complete disappearance of the starting material **4T** or **4S** and the formation of a novel spot at a higher *R*_{f}), the reaction mixture was concentrated to dryness and the crude material purified by column chromatography using the eluent indicated in each case. The resulting intermediate with Boc-protected amino groups was subsequently treated with a 1:1 DCM-TFA mixture (10 mL) at RT for 1 h followed by evaporation of the solvents under reduced pressure. Traces of TFA were eliminated by coevaporation with toluene (3 x 10 mL). Lyophilization from 10 mM HCI furnished the target products **6T** or **6S** in the yield indicated in each case.

In some embodiments, the following compounds of formula I featuring structure **6T** were synthesized according to Scheme 1, route A1: JLF20, JLF32, JLF33, JLF35, JLF43, JLF58, JLF78, JLJB617, JLJB618, JLJB619.

In some embodiments, the following compounds of formula I featuring structure **6S** were synthesized according to Scheme 2, route A1: JLF59, JLF60, JLF68, JLF69, JLF76, JLF77

**Route A2 (schemes 1 or 2):** Similarly, to route A1, route A2 implies the coupling reaction of a compound of structure **4T** or **4S** with an isothiocyanate reagent SCN-(CH₂)₂-Z¹, using an analogous protocol. Upon reaction completion, the reaction mixture was transferred to a decantation funnel and partitioned between DCM and 0.1 N aq HCI (1:1, 20 mL). The organic layer was decanted, dried over MgSO₄ or Na₂SO₄, filtrated and evaporated under reduced pressure. Column chromatography purification was conducted in these cases. The resulting product was lyophilized from 10 mM HCI to furnish the target product **6T** or **6S** in the yield indicated in each case.

In some embodiments, the following compounds of formula I featuring structure **6T** were synthesized according to Scheme 1, route A2: JLF25, JLF26, JLF29, JLF30, JLF41, JLF42, JLJB604, JLJB605.

In some embodiments, the following compounds of formula I featuring structure **6S** were synthesized according to Scheme 2, route A2: JLF54, JLF55, JLF66, JLF67,

**Route B1 (scheme 1):** To a solution of the corresponding diisothiocyanate reaction intermediate **5T** (1 mmol) in DCM (10 mL) a solution the corresponding amine reagent H₂N-(CH₂)₂-Z^{a}-PG¹ (0.3 mmol) in DCM (5 mL) was added. The reaction mixture was stirred at RT for 16 h while monitoring pH to ensure it remains basic. Upon reaction completion (TLC revealed the complete disappearance of the starting material **5T** and the formation of a novel spot at a lower *R*_{f}), the reaction mixture was concentrated to dryness and the crude material purified by column chromatography using the indicated eluent. The resulting intermediate was further treated with a 1:1 DCM-TFA mixture (10 mL) at RT for 1 h followed by evaporation of the solvents under reduced pressure, coevaporation of the acidic traces with toluene (3 x 10 mL) and lyophilization from 10 mM HCI to furnish the target products **6T** in the yield indicated in each case.

In some embodiments, the following compounds of formula I featuring structure **6T** were synthesized according to Scheme 1, route B1: JLF94, JLF96, JLF97, JLF98, JLF102, JLF103, JLF111, JLF115, JLF120, JLF121, JLF139, JLF141, PER20.

**Route B2:** Similarly, to route B1, route B2 implies the coupling reaction of a compound of structure **5T** with an amine reagent H₂N-(CH₂)₂-Z¹, using an analogous protocol. Upon reaction completion, the reaction mixture was transferred to a decantation funnel and partitioned between DCM and 0.1 N aq HCI (1:1, 20 mL). The organic layer was decanted, dried over MgSO₄ or Na₂SO₄, filtrated and evaporated under reduced pressure. Column chromatography purification is unnecessary in these cases. The resulting product was lyophilized from 10 mM HCI to furnish the target products **6T** in the indicated yields.

In some embodiments, the following compounds of formula I featuring structure 6T were synthesized according to Scheme 1, route B1: JLF94, JLF96, JLF97, JLF98, JLF102, JLF103, JLF111, JLF115, JLF120, JLF121, JLF139, JLF141, PER20. General procedure for the synthesis of a compound of formula I according to Scheme 3, routes C1 and C2.

The diiodo trehalose derivative **1T** was per-*O*-acylated following a procedure analogous to the **General procedure for exhaustive hydroxyl acylation of 2T and 2S to give reaction intermediates 3T and 3S** described above. The resulting crude product was subjected to column chromatography purification (1:10 EtOAc-petroleum ether) to furnish reaction intermediate **7T** in 67% yield.

In a preferred embodiment, the diiodo reaction intermediate 7T wherein the R^{a} substituents are defined by the code **L1 (7T-L1),** as indicated in the **Lipid reagent list,** was prepared according to this procedure.

**Route C1:** To a mixture of **7T** ( 0.1 mmol) and Cs₂CO₃ (91 mg, 0.28 mmol) in anhydrous DMF (3 mL) under N₂ atmosphere, 2-[*N*-methyl-*N-*(*tert-*butoxycarbonyl)amino]ethanethiol **(C2SH,** 52 µL, 0.3 mmol) was added and the reaction mixture was stirred at RT overnight. The solvent was then removed under reduced pressure and the resulting residue was purified by column chromatography (1:6 EtOAc-petroleum ether). The resulting intermediate was treated with a 1:1 DCM-TFA mixture (4 mL) at RT for 1 h. The solvents were evaporated under reduced pressure, the traces of acid were repeatedly coevaporated with water and the residue was lyophilized from 10-mM HCI to furnish **8T** in 60% yield.

In a preferred embodiment, the following compound of formula I featuring structure **8T** was synthesized according to Scheme 3, route C1: JLF48.

**Route C2:** Similarly to route C1, route C2 implies the coupling reaction between **7T** and 2-(*N*,*N*-dimethylamino)ethanethiol **(C3SH),** following an analogous protocol. Upon reaction completion, the reaction mixture was transferred to a decantation funnel and partitioned between DCM and 0.1N aq HCI (1:1, 20 mL). The organic layer was decanted, dried over MgSO₄ or Na₂SO₄, filtrated and evaporated under reduced pressure. Column chromatography purification is unnecessary in this case. The resulting product was lyophilized from 10 mM HCI to furnish compound **8T** in 82% yield.

In a preferred embodiment, the following compound of formula I featuring structure **8T** was synthesized according to Scheme 3, route C1: JLF40.

### Example 4a - Compound JLF19

Synthesized from compound **3T-L7** according to Scheme 1 **(3T** to **4T** step) in quantitative yield. Chemical Formula: C₁₂₄H₂₃₈Cl₂N₂O₁₅S₂. Molecular Weight: 2132.29 ESI-MS (*m*/*z*) 1031.08 ([M - 2Cl]²⁺).

### Example 4b - Compound JLF20

Synthesized from compound **3T-L5** according to Scheme 1 **(3T** to **4T** step) in quantitative yield. Chemical Formula: C₁₀₀H₁₉₀Cl₂N₂O₁₅S₂. Molecular Weight: 1795.64 ESI-MS (*m*/*z*) 1723.23 ([M - 2HCl + H]+), 862.20 ([M - 2Cl]²⁺).

### Example 4c - Compound JLF23

Synthesized from compound **3T-L3** according to Scheme 1 **(3T** to **4T** step) in quantitative yield. Chemical Formula: C₇₆H₁₄₂Cl₂N₂O₁₅S₂. Molecular Weight: 1458.99 ESI-MS (*m*/*z*) 1385.93 ([M - 2HCl + H]+), 693.98 ([M - 2Cl]²⁺).

### Example 4d - Compound JLF25

Synthesized from compound **JLF20** and the corresponding isothiocyanate reagent (code **C3)** according to Scheme 1, Route A2 **(4T** to **6T** step). Yield 72%. Chemical Formula: C₁₁₀H₂₁₀Cl₂N₆O₁₅S₄. Molecular Weight: 2056.06 ESI-MS *(m*/*z)* 992.56 ([M - 2Cl]²⁺).

### Example 4e - Compound JLF26

Synthesized from compound **JLF20** and the corresponding isothiocyanate reagent (code **C4)** according to Scheme 1, Route A2 **(4T** to **6T** step). Yield 76%. Chemical Formula: C₁₁₂H₂₁₄I₂N₆O₁₅S₄. Molecular Weight: 2267.02 ESI-MS *(m*/*z)* 1006.49 ([M - 21]²⁺).

### Example 4f -Compound JLF29

Synthesized from compound **JLF19** and the corresponding isothiocyanate reagent (code **C3)** according to Scheme 1, Route A2 **(4T** to **6T** step). Yield 77%. Chemical Formula: C₁₃₂H₂₅₄Cl₂N₆O₁₅S₄. Molecular Weight: 2264.65 ESI-MS (*m*/*z*) 1160.74 ([M - 2Cl]²⁺).

### Example 4g - Compound JLF30

Synthesized from compound **JLF19** and the corresponding isothiocyanate reagent (code **C4)** according to Scheme 1, Route A2 **(4T** to **6T** step). Yield 87%. Chemical Formula: C₁₃₄H₂₅₈I₂N₆O₁₅S₄. Molecular Weight: 2575.61 ESI-MS *(m*/*z)* 1174.68 ([M - 2I]²⁺).

### Example 4h - Compound JLF31

Synthesized from compound **JLF20** and the corresponding isothiocyanate reagent (code **C2)** according to Scheme 1, Route A1 **(4T** to **6T** step). Yield 91%. Chemical Formula: C₁₀₈H₂₀₆Cl₂N₆O₁₅S₄. Molecular Weight: 2028.00 ESI-MS (*m*/*z*) 978.06 ([M - 2Cl]²⁺.

### Example 4i - Compound JLF32

Synthesized from compound **JLF19** and the corresponding isothiocyanate reagent (code **C1)** according to Scheme 1, Route A1 **(4T** to **6T** step). Yield 66%. Chemical Formula: C₁₂₈H₂₄₆Cl₂N₆O₁₅S₄. Molecular Weight: 2308.54 ESI-MS (*m*/*z*) 1132.20 ([M - 2Cl]²⁺).

### Example 4j - Compound JLF33

Synthesized from compound **JLF19** and the corresponding isothiocyanate reagent (code **C2)** according to Scheme 1, Route A1 **(4T** to **6T** step). Yield 86%. Chemical Formula: C₁₃₀H₂₅₀Cl₂N₆O₁₅S₄. Molecular Weight: 2336.60 ESI-MS (*m*/*z*) 1146.25 ([M - 2Cl]²⁺).

### Example 4k - Compound JLF35

Synthesized from compound **JRL9** and the corresponding isothiocyanate reagent (code **C5)** according to Scheme 1, Route A1 **(4T** to **6T** step). Yield 89%. Chemical Formula: C₆₆H₁₂₂Cl₄N₈O₁₉S₄. Molecular Weight: 1601.78 ESI-MS *(m*/*z)* 728.30 ([M - 2HCl - 2Cl]²⁺).

### Example 4l - Compound JLF40

Synthesized from precursor **7T-L1** and HS-(CH₂)₂NMe₂ **(C3SH)** according to Scheme 3, route C2 **(7T** to **8T** step). Yield 82%. Chemical Formula: C₅₆H₁₀₂Cl₂N₂O₁₅S₂. Molecular Weight: 1178.45 ESI-MS (*m*/*z*) 1105.68 ([M - HCI - Cl]⁺), 553.47 ([M - 2Cl]²⁺).

### Example 4m - Compound JLF41

Synthesized from compound **JLF23** and the corresponding isothiocyanate reagent (code **C3)** according to Scheme 1, Route A2 **(4T** to **6T** step). Yield 55%. Chemical Formula: C₈₆H₁₆₂Cl₂N₆O₁₅S₄. Molecular Weight: 1719.41 ESI-MS *(m*/*z)* 1645.95 ([M - HCI - Cl]⁺), 824.06 ([M - 2Cl]²⁺).

### Example 4n - Compound JLF42

Synthesized from compound **JLF23** and the corresponding isothiocyanate reagent (code **C4)** according to Scheme 1, Route A2 **(4T** to **6T** step). Yield 57%. Chemical Formula: C₈₈H₁₆₆I₂N₆O₁₅S₄. Molecular Weight: 1930.37 ESI-MS (*m*/*z*) 838.06 ([M - 2I]²⁺).

### Example 4o - Compound JLF43

Synthesized from compound **JLF23** and the corresponding isothiocyanate reagent (code **C2)** according to Scheme 1, Route A1 **(4T** to **6T** step). Yield 52%. Chemical Formula: C₈₄H₁₅₈Cl₂N₆O₁₅S₄. Molecular Weight: 1691.36 ESI-MS *(m*/*z)* 810.09 ([M - 2Cl]²⁺).

### Example 4p - Compound JLF46

Synthesized from compound **3T-L7.1** according to Scheme 1 **(3T** to **4T** step) in quantitative yield. Chemical Formula: C₁₂₄H₂₂₆Cl₂N₂O₁₅S₂. Molecular Weight: 2021.19 ESI-MS (*m*/*z*) 1024.38 ([M - 2Cl]²⁺).

### Example 4q - Compound JLF48

Synthesized from precursor **7T-L1** and HS-(CH₂)₂N(Boc)Me **(C2SH)** according to Scheme 3, route C1 (**7T** to **8T** step). Yield 60%. Chemical Formula: C₅₄H₉₈Cl₂N₂O₁₅S₂. Molecular Weight: 1150.40 ESI-MS (*m*/*z*) 1077.63 ([M - HCI - Cl]⁺), 536.44 ([M - 2Cl]²⁺).

### Example 4r - Compound JLF50

Synthesized from compound **3S-L1** according to Scheme 2 **(3S** to **4S** step) in quantitative yield. Chemical Formula: C₅₂H₉₄Cl₂N₂O₁₅S₂. Molecular Weight: 1122.34 ESI-MS (*m*/*z*) 1049.63 ([M - HCI - Cl]⁺), 525.44 ([M - 2Cl]²⁺).

### Example 4s ― Compound JLF54

Synthesized from compound **JLF50** and the corresponding isothiocyanate reagent (code **C3)** according to Scheme 2, Route A2 **(4S** to **6S** step). Yield 59%. Chemical Formula: C₆₂H₁₁₄Cl₂N₆O₁₅S₄. Molecular Weight: 1382.76 ESI-MS *(m*/*z)* 1309.71 ([M - HCI - Cl]⁺), 655.57 ([M - 2Cl]²⁺).

### Example 4t ― Compound JLF55

Synthesized from compound **JLF50** and the corresponding isothiocyanate reagent (code **C4)** according to Scheme 2, Route A2 **(4S** to **6S** step). Yield 59%. Chemical Formula: C₆₄H₁₁₈I₂N₆O₁₅S₄. Molecular Weight: 1593.72 ESI-MS *(m*/*z)* 1338.63 ([M - HI - I]⁺), 1338.63 ([M - HI - Me₃NI]⁺), 669.80 ([M - 2I]²⁺), 639.99 ([M - I - Me₃NI]²⁺).

### Example 4u - Compound JLF58

Synthesized from compound **JLF50** and the corresponding isothiocyanate reagent (code **OH1)** according to Scheme 1, Route A1 **(4T** to **6T** step).Yield 69%. Chemical Formula: C₆₂H₁₁₄Cl₂N₆O₁₇S₄. Molecular Weight: 1414.76 ESI-MS *(m*/*z)* 1341.70 ([M - HCI - Cl]⁺), 671.61 ([M - 2Cl]²⁺).

### Example 4v - Compound JLF59

Synthesized from compound **JLF50** and the corresponding isothiocyanate reagent (code **C1)** according to Scheme 2, Route A1 **(4S** to **6S** step).Yield 62%. Chemical Formula: C₅₈H₁₀₆Cl₂N₆O₁₅S₄. Molecular Weight: 1326.65 ESI-MS *(m*/*z)* 1253.69 ([M - HCI - Cl]⁺), 627.59 ([M - 2Cl]²⁺).

### Example 4w - Compound JLF60

Synthesized from compound **JLF50** and the corresponding isothiocyanate reagent (code **C2)** according to Scheme 2, Route A1 **(4S** to **6S** step).Yield 49%. Chemical Formula: C₆₀H₁₁₀Cl₂N₆O₁₅S₄. Molecular Weight: 1354.71 ESI-MS *(m*/*z)* 1284.69 ([M - HCI - Cl]⁺), 641.59 ([M - 2Cl]²⁺).

### Example 4x - Compound JLF62

Synthesized from compound **3S-L3** according to Scheme 2 **(3S** to **4S** step) in quantitative yield. Chemical Formula: C₇₆H₁₄₂Cl₂N₂O₁₅S₂. Molecular Weight: 1458.99 ESI-MS (*m*/*z*) 1385.98 ([M - 2HCl + H]+), 693.95 ([M - 2Cl]²⁺).

### Example 4y - Compound JLF66

Synthesized from compound **JLF62** and the corresponding isothiocyanate reagent (code **C3)** according to Scheme 2, Route A2 **(4S** to **6S** step). Yield 81%. Chemical Formula: C₈₆H₁₆₂Cl₂N₆O₁₅S₄. Molecular Weight: 1719.41 ESI-MS *(m*/*z)* 1646.99 ([M - HCI - Cl]⁺), 824.14 ([M - 2Cl]²⁺).

### Example 4z - Compound JLF67

Synthesized from compound **JLF62** and the corresponding isothiocyanate reagent (code **C4)** according to Scheme 2, Route A2 **(4S** to **6S** step). Yield 85%. Chemical Formula: C₈₈H₁₆₆I₂N₆O₁₅S₄. Molecular Weight: 1930.37 ESI-MS *(m*/*z)* 1615.91 ([M - HI - I]⁺), 838.21 ([M - 2I]²⁺).

### Example 4aa ― Compound JLF68

Synthesized from compound **JLF62** and the corresponding isothiocyanate reagent (code **C1)** according to Scheme 2, Route A1 **(4S** to **6S** step). Yield 57%. Chemical Formula: C₈₂H₁₅₄Cl₂N₆O₁₅S₄. Molecular Weight: 1663.30 ESI-MS *(m*/*z)* 1590.90 ([M - HCI - Cl]⁺), 796.06 ([M - 2Cl]²⁺).

### Example 4ab - Compound JLF69

Synthesized from compound **JLF62** and the corresponding isothiocyanate reagent (code **C2)** according to Scheme 2, Route A1 **(4S** to **6S** step). Yield 73%. Synthesized by Route A1 by coupling of compounds **4S-L3** and **C2.** Overall yield (2-steps) 73% from **4S-L3.** Chemical Formula: C₈₄H₁₅₈Cl₂N₆O₁₅S₄. Molecular Weight: 1691.36 ESI-MS (*m*/*z*) 1618.92 ([M - HCI - Cl]⁺), 810.09 ([M - 2Cl]²⁺).

### Example 4ac - Compound JLF76

Synthesized from compound **JLF50** and the corresponding isothiocyanate reagent (code **C5)** according to Scheme 2, Route A1 **(4S** to **6S** step). Yield 66%. Chemical Formula: C₆₆H₁₂₂Cl₄N₈O₁₉S₄. Molecular Weight: 1601.78 ESI-MS *(m*/*z)* 1454.66 ([M - 4HCl - H]⁻).

### Example 4ad - Compound JLF77

Synthesized from compound **JLF62** and the corresponding isothiocyanate reagent (code **C5)** according to Scheme 2, Route A1 **(4S** to **6S** step). Yield 62%. Chemical Formula: C₉₀H₁₇₀Cl₄N₈O₁₉S₄. Molecular Weight: 1938.43 ESI-MS *(m*/*z)* 897.20 ([M - 2HCl - 2Cl]²⁺).

### Example 4ae - Compound JLF78

Synthesized from compound **JLF23** and the corresponding isothiocyanate reagent (code **C5)** according to Scheme 1, Route A1 **(4T** to **6T** step). Yield 68%. Chemical Formula: C₉₀H₁₇₀Cl₄N₈O₁₉S₄. Molecular Weight: 1938.43 ESI-MS *(m*/*z)* 897.20 ([M - 2HCl - 2Cl]²⁺).

### Example 4af ― Compound JLF81

Synthesized from the diisothiocyanate precursor **5T-L1** and the corresponding amine reagent (code **C9)** according to Scheme 1, Route B2 **(5T** to **6T** step). Yield 86%. Chemical Formula: C₆₆H₁₁₈Cl₂N₆O₁₇S₄. Molecular Weight: 1466.84 ESI-MS *(m*/*z)* 1393.77 ([M - HCl - Cl]⁺), 697.87 ([M - 2Cl]²⁺).

### Example 4ag - Compound JLF82

Synthesized from the diisothiocyanate precursor **5T-L1** and the corresponding amine reagent (code **C7)** according to Scheme 1, Route B2 **(5T** to **6T** step). Yield 66%. Chemical Formula: C₆₈H₁₂₆Cl₄N₈O₁₅S₄. Molecular Weight: 1565.84 ESI-MS *(m*/*z)* 1419.81 ([M - 3HCl - Cl]⁺), 710.90 ([M - 2HCl - 2Cl]²⁺), 1418.96 ([M - 4HCl - H]⁻).

### Example 4ah - Compound JLF87

Synthesized from the diisothiocyanate precursor **5T-L1** and the corresponding amine reagent (code **C10)** according to Scheme 1, Route B2 **(5T** to **6T** step). Yield 89%. Chemical Formula: C₆₄H₁₀₈Cl₂N₈O₁₅S₄. Molecular Weight: 1428.75 ESI-MS *(m*/*z)* 1355.67 ([M - HCl- Cl]⁺), 678.77 ([M - 2Cl]²⁺).

### Example 4ai - Compound JLF90

Synthesized from the diisothiocyanate precursor **5T-L1** and the corresponding amine reagent (code **C10.2)** according to Scheme 1, Route B2 **(5T** to **6T** step). Yield 73%. Chemical Formula: C₆₄H₁₀₈Cl₂N₈O₁₅S₄. Molecular Weight: 1428.75 ESI-MS *(m*/*z)* 1355.68 ([M - HCl - Cl]⁺), 678.77 ([M - 2Cl]²⁺), 1354.61 ([M - 2HCl - H]⁻).

### Example 4aj - Compound JLF93

Synthesized from the diisothiocyanate precursor **5T-L1** and the corresponding amine reagent (code **C8)** according to Scheme 1, Route B2 **(5T** to **6T** step). Yield 81%. Chemical Formula: C₆₆H₁₂₂C₁₄N₈O₁₅S₄. Molecular Weight: 1537.78 ESI-MS *(m*/*z)* 1391.74 ([M - 3HCl - Cl]⁺), 696.90 ([M - 2Cl]²⁺), 647.65 ([M - hexanoyl - 2Cl]²⁺), 1390.84 ([M - 4HCl - H]⁻).

### Example 4ak - Compound JLF94

Synthesized from the diisothiocyanate precursor **5T-L1** and the corresponding amine reagent (code **C11)** according to Scheme 1, Route B1 **(5T** to **6T** step). Yield 95%. Chemical Formula: C₆₀H₁₁₀Cl₂N₁₀O₁₅S₄. Molecular Weight: 1410.74 ESI-MS *(m*/*z)* 1337.68 ([M - HCl - Cl]⁺), 669.73 ([M - 2Cl]²⁺), 1336.62 ([M - 2HCl - H]⁻).

### Example 4al ― Compound JLF95

Synthesized from the diisothiocyanate precursor **5T-L1** and the corresponding amine reagent (code **C10.1)** according to Scheme 1, Route B2 **(5T** to **6T** step). Yield 42%. Chemical Formula: C₆₄H₁₀₈Cl₂N₈O₁₅S₄. Molecular Weight: 1428.75 ESI-MS (*m*/*z*) 1355.65 ([M - HCl - Cl]⁺), 678.79 ([M - 2Cl]²⁺), 1354.62 ([M - 2HCl - H]⁻).

### Example 4am - Compound JLF96

Synthesized from the diisothiocyanate precursor **5T-L1** and the corresponding amine reagent (code **OH2)** according to Scheme 1, Route B1 **(5T** to **6T** step). Yield 97%. Chemical Formula: C₆₄H₁₁₈Cl₂N₆O₁₇S₄. Molecular Weight: 1442.81 ESI-MS (*m*/*z*) 1369.76 ([M - HCl - Cl]⁺), 685.79 ([M - 2Cl]²⁺).

### Example 4an - Compound JLF97

Synthesized from the diisothiocyanate precursor **5T-L1** and the corresponding amine reagent (code **OH3)** according to Scheme 1, Route B1 **(5T** to **6T** step). Yield 34%. Chemical Formula: C₆₆H₁₂₂Cl₂N₆O₁₇S₄. Molecular Weight: 1470.87 ESI-MS (*m*/*z*) 1397.78 ([M - HCl- Cl]⁺), 699.81 ([M - 2Cl]²⁺).

### Example 4ao - Compound JLF98

Synthesized from the diisothiocyanate precursor **5T-L3** and the corresponding amine reagent (code **C11)** according to Scheme 1, Route B1 **(5T** to **6T** step). Yield 99%. Chemical Formula: C₈₄H₁₅₈Cl₂N₁₀O₁₅S₄. Molecular Weight: 1747.38 ESI-MS (*m*/*z*) 838.07 ([M - 2Cl]²⁺).

### Example 4ap - Compound JLF99

Synthesized from the diisothiocyanate precursor **5T-L3** and the corresponding amine mine reagent (code **C9)** according to Scheme 1, Route B2 **(5T** to **6T** step). Yield 45%. Chemical Formula: C₉₀H₁₆₆Cl₂N₆O₁₇S₄. Molecular Weight: 1803.48 ESI-MS (*m*/*z*) 1731.03 ([M - HCl - Cl]⁺), 866.19 ([M - 2Cl]²⁺).

### Example 4aq ― Compound JLF102

Synthesized from the diisothiocyanate precursor **5T-L1** and the corresponding amine reagent (code **C18)** according to Scheme 1, Route B1 **(5T** to **6T** step). Yield 94%. Chemical Formula: C₆₂H₁₁₈Cl₄N₈O₁₅S₄. Molecular Weight: 1485.71 ESI-MS (*m*/*z*) 1339.69 ([M - HCl - Cl]⁺), 670.54 ([M - 2Cl]²⁺), 1338.93 ([M - 2HCl - H]⁻).

### Example 4ar - Compound JLF103

Synthesized from the diisothiocyanate precursor **5T-L3** and the corresponding amine reagent (code **C18)** according to Scheme 1, Route B1 **(5T** to **6T** step). Yield 62%. Chemical Formula: C₈₆H₁₆₆Cl₄N₈O₁₅S₄. Molecular Weight: 1822.36 ESI-MS *(m*/*z)* 1677.15 ([M - 3HCl - Cl]⁺), 839.10 ([M - 2HCl - 2Cl]²⁺).

### Example 4as - Compound JLF106

Synthesized from the diisothiocyanate precursor **5T-L1** and the corresponding amine reagent (code **C14)** according to Scheme 1, Route B2 **(5T** to **6T** step). Yield 94%. Chemical Formula: C₆₂H₁₁₀Cl₂N₆O₁₅S₄. Molecular Weight: 1378.73 ESI-MS (*m*/*z*) 1305.94 ([M - HCl - Cl]⁺), 653.86 ([M - 2Cl]²⁺), 1350.66 ([M - 2HCl + HCO₂]⁻).

### Example 4at - Compound JLF108

Synthesized from the diisothiocyanate precursor **5T-L3** and the corresponding amine reagent (code **C7)** according to Scheme 1, Route B2 **(5T** to **6T** step). Yield 78%. Chemical Formula: C₉₂H₁₇₄Cl₄N₈O₁₅S₄. Molecular Weight: 1902.49 ESI-MS (*m*/*z*) 1757.06 ([M - 3HCl - Cl]⁺), 879.22 ([M - 2HCl - 2Cl]²⁺), 1755.25 ([M - 4HCl - H]⁻).

### Example 4au - Compound JLF110

Synthesized from the diisothiocyanate precursor **5T-L3** and the corresponding amine reagent (code **C10)** according to Scheme 1, Route B2 **(5T** to **6T** step). Yield 72%. Chemical Formula: C₈₈H₁₅₆Cl₂N₈O₁₅S₄. Molecular Weight: 1765.40 ESI-MS (*m*/*z*) 1691.98 ([M - HCl - Cl]⁺), 847.16 ([M - 2Cl]²⁺).

### Example 4av - Compound JLF11

Synthesized from the diisothiocyanate precursor **5T-L3** and the corresponding amine reagent (code **C10.2)** according to Scheme 1, Route B1 **(5T** to **6T** step). Yield 49%. Synthesized by Route B1 by coupling of compounds **5T-L3** and **C10.2.** Overall yield (2-steps) 49% from **5T-L3.** Chemical Formula: C₈₈H₁₅₆Cl₂N₈O₁₅S₄. Molecular Weight: 1765.70 ESI-MS (*m*/*z*) 1692.93 ([M - HCI - Cl]⁺), 847.13 ([M - 2Cl]²⁺), 1691.14 ([M - 2HCl - H]⁻).

### Example 4aw - Compound JLF115

Synthesized from the diisothiocyanate precursor **5T-L3** and the corresponding amine reagent (code **C8)** according to Scheme 1, Route B1 **(5T** to **6T** step). Yield 68%. Chemical Formula: C₉₀H₁₇₀Cl₄N₈O₁₅S₄. Molecular Weight: 1874.43 ESI-MS (*m*/*z*) 1571.05 ([M - 3HCl - Cl]⁺), 865.23 ([M - 2HCl - 2Cl]²⁺).

### Example 4ax - Compound JLF120

Synthesized from the diisothiocyanate precursor **5T-L1** and the corresponding amine reagent (code **C43)** according to Scheme 1, Route B1 **(5T** to **6T** step). Yield 88%. Chemical Formula: C₆₆H₁₃₀Cl₆N₁₀O₁₅S₁₀. Molecular Weight: 1644.76 ESI-MS *(m*/*z)* 1426.74 ([M - 5HCl - Cl]⁺), 713.56 ([M - 4HCl - 2Cl]²⁺), 1424.65 ([M - 6HCl - H]⁻).

### Example 4ay - Compound JLF121.

Synthesized from the diisothiocyanate precursor **5T-L3** and the corresponding amine reagent (code **C43)** according to Scheme 1, Route B1 **(5T** to **6T** step). Yield 55%. Synthesized by Route B1 by coupling of compounds **5T-L3** and **C43.** Overall yield (2-steps) 55% from **5T-L3.** Chemical Formula: C₉₀H₁₇₈Cl₆N₁₀O₁₅S₁₀. Molecular Weight: 1981.41 ESI-MS (*m*/*z*) 1763.11 ([M - 5HCl - Cl]⁺), 882.20 ([M - 4HCl - 2Cl]²⁺), 1761.32 ([M - 6HCl - H]⁻).

### Example 4az - Compound JLF125

Synthesized from the diisothiocyanate precursor **5T-L1** and the corresponding amine reagent (code **C16)** according to Scheme 1, Route B2 **(5T** to **6T** step). Yield 60%. Chemical Formula: C₆₆H₁₂₂Cl₂N₆O₁₅S₄. Molecular Weight: 1438.87 ESI-MS (*m*/*z*) 1365.77 ([M - HCl- Cl]⁺), 683.99 ([M - 2Cl]²⁺), 1364.81 ([M - 2HCl - H]⁻).

### Example 4ba - Compound JLF128

Synthesized from the diisothiocyanate precursor **5T-L3** and the corresponding amine reagent (code **C18)** according to Scheme 1, Route B2 **(5T** to **6T** step). Yield 76%. Synthesized by Route B2 by coupling of compounds **5T-L3** and **C18.** Yield 76% from **5T-L3.** Chemical Formula: C₉₀H₁₇₀Cl₂N₆O₁₅S₄. Molecular Weight: 1775.52 ESI-MS (*m*/*z*) 1703.07 ([M - HCI - Cl]⁺), 852.21 ([M - 2Cl]²⁺), 1701.28 ([M - 2HCl - H]⁻).

### Example 4bb - Compound JLF130

Synthesized from the diisothiocyanate precursor **5T-L1** and the corresponding amine reagent (code **OH12)** according to Scheme 1, Route B2 **(5T** to **6T** step). Yield 71%. Chemical Formula: C₇₀H₁₃₀Cl₄N₈O₁₇S₄. Molecular Weight: 1625.89

ESI-MS (*m*/*z*) 1479.81 ([M - 3HCl - Cl]⁺), 740.95 ([M - 2HCl - 2Cl]²⁺), 1478.81 ([M - 4HCl - H]⁻).

### Example 4bc - Compound JLF131

Synthesized from the diisothiocyanate precursor **5T-L3** and the corresponding amine reagent (code **C46)** according to Scheme 1, Route B2 **(5T** to **6T** step). Yield 40%. Chemical Formula: C₉₈H₁₈₂Cl₂N₁₀O₁₉S₄. Molecular Weight: 2003.73 ESI-MS (*m*/*z*) 1931.17 ([M - HCI - Cl]⁺), 966.35 ([M - 2Cl]²⁺), 1929.40 ([M - 2HCl - H]⁻), 1965.40 ([M - HCI - H]⁻).

### Example 4bd - Compound JLF134

Synthesized from the diisothiocyanate precursor **5T-L1** and the corresponding amine reagent (code **OH5)** according to Scheme 1, Route B2 **(5T** to **6T** step). Yield 74%. Chemical Formula: C₆₆H₁₂₂Cl₂N₆O₁₉S₄. Molecular Weight: 1502.87 ESI-MS (*m*/*z*) 1429.97 ([M - HCl- Cl]⁺), 715.89 ([M - 2Cl]²⁺), 1428.72 ([M - 2HCl - H]⁻).

### Example 4be - Compound JLF135

Synthesized from the diisothiocyanate precursor **5T-L1** and the corresponding amine reagent (code **OH10)** according to Scheme 1, Route B2 **(5T** to **6T** step). Yield 61%. Chemical Formula: C₆₆H₁₂₂Cl₂N₆O₂₁S₄. Molecular Weight: 1534.86 ESI-MS (*m*/*z*) 1461.79 ([M - HCl - Cl]⁺), 731.90 ([M - 2Cl]²⁺), 1460.71 ([M - 2HCl - H]⁻).

### Example 4bf ― Compound JLF138

Synthesized from the diisothiocyanate precursor **5T-L1** and the corresponding amine reagent (code **C7.1)** according to Scheme 1, Route B2 **(5T** to **6T** step). Yield 90%. Chemical Formula: C₇₂H₁₃₄Cl₄N₆O₁₅S₄. Molecular Weight: 1621.95 ESI-MS (*m*/*z*) 1475.94 ([M - 3HCl - Cl]⁺), 739.04 ([M - 2HCl - 2Cl]²⁺), 1475.01 ([M - 4HCl - H]⁻).

### Example 4bg - Compound JLF139

Synthesized from the diisothiocyanate precursor **5T-L1** and the corresponding amine reagent (code **C15)** according to Scheme 1, Route B1 **(5T** to **6T** step). Yield 99%. Chemical Formula: C₆₂H₁₁₄Cl₂N₆O₁₅S₄. Molecular Weight: 1382.76 ESI-MS (*m*/*z*) 1309.84 ([M - HCl - Cl]⁺), 655.88 ([M - 2Cl]²⁺), 1308.96 ([M - 2HCl - H]⁻).

### Example 4bh - Compound JLF141

Synthesized from the diisothiocyanate precursor **5T-L1** and the corresponding amine reagent (code **C12)** according to Scheme 1, Route B1 **(5T** to **6T** step). Yield 78%. Chemical Formula: C₆₆H₁₂₆Cl₄N₈O₁₅S₄. Molecular Weight: 1541.82 ESI-MS (*m*/*z*) 1395.90 ([M - 3HCl - Cl]⁺), 698.68 ([M - 2HCl - 2Cl]²⁺), 1394.92 ([M - 4HCl - H]⁻).

### Example 4bi - Compound JLF142

Synthesized from the diisothiocyanate precursor **5T-L3** and the corresponding amine reagent (code **OH5)** according to Scheme 1, Route B2 **(5T** to **6T** step). Yield 39%. Chemical Formula: C₆₆H₁₂₆Cl₄N₈O₁₅S₄. Molecular Weight: 1541.82 ESI-MS (*m*/*z*) 1767.15 ([M - 3HCl - Cl]⁺), 884.24 ([M - 2HCl - 2Cl]²⁺), 1765.37 ([M - 4HCl - H]⁻).

### Example 4bj - Compound JLJB604

Synthesized from compound **JRL9** and the corresponding isothiocyanate reagent (code **C3)** according to Scheme 1, Route A2 **(4T** to **6T** step). Yield 69%. Chemical Formula: C₆₂H₁₁₄Cl₂N₆O₁₅S₄. Molecular Weight: 1382.76 ESI-MS (*m*/*z*) 1309.62 ([M - HCI - Cl]⁺), 655.53 ([M - 2Cl]²⁺).

### Example 4bk - Compound JLJB605

Synthesized from compound **JRL9** and the corresponding isothiocyanate reagent (code **C4)** according to Scheme 1, Route A2 **(4T** to **6T** step). Yield 94%. Chemical Formula: C₆₄H₁₁₈I₂N₆O₁₅S₄. Molecular Weight: 1593.72 ESI-MS (*m*/*z*) 1278.58 ([M - I - Me₃NI]⁺), 669.80 ([M - 2I]²⁺), 1382.20 ([M - 2HI + Cl]⁻), 1323.49 ([M - HI - Me₃NI + HCO₂]⁺).

### Example 4bl ― Compound JLJB617

Synthesized from compound **JLF23** and the corresponding isothiocyanate reagent (code **C1)** according to Scheme 1, Route A1 **(4T** to **6T** step). Yield 21%. Chemical Formula: C₈₂H₁₅₄Cl₂N₆O₁₅S₄. Molecular Weight: 1663.30 ESI-MS (*m*/*z*) 795.97 ([M - 2Cl]²⁺).

### Example 4bm - Compound JLJB618

Synthesized from compound **JLF20** and the corresponding isothiocyanate reagent (code **C1)** according to Scheme 1, Route A1 **(4T** to **6T** step). Yield 50%. Chemical Formula: C₁₀₆H₂₀₂Cl₂N₆O₁₅S₄. Molecular Weight: 1999.95 ESI-MS (*m*/*z*) 1618.92 ([M - HCI - Cl]⁺), 810.09 ([M - 2Cl]²⁺).

### Example 4bn - Compound JLJB619

Synthesized from compound **JRL9** and the corresponding isothiocyanate reagent (code **C2)** according to Scheme 1, Route A2 **(4T** to **6T** step). Yield 52%. Chemical Formula: C₆₀H₁₁₀Cl₂N₆O₁₅S₄. Molecular Weight: 1354.71 ESI-MS (*m*/*z*) 1281.40 ([M - HCI - Cl]⁺), 641.22 ([M - 2Cl]²⁺).

### Example 4bo - Compound PER20

Synthesized from the diisothiocyanate precursor **5T-L3** and the corresponding amine reagent (code **OH1)** according to Scheme 1, Route B1 **(5T** to **6T** step). Yield 59%. Chemical Formula: C₈₆H₁₆₂Cl₂N₆O₁₇S₄. Molecular Weight: 1751.41 ESI-MS (*m*/*z*) 840.14 ([M - 2Cl]²⁺).

## Claims

1. A composition comprising an oligosaccharide of formula I: 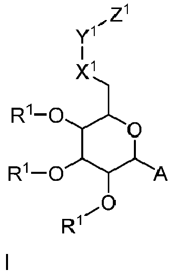 or a pharmaceutically acceptable salt thereof, wherein:
A is A¹, A², or A³: 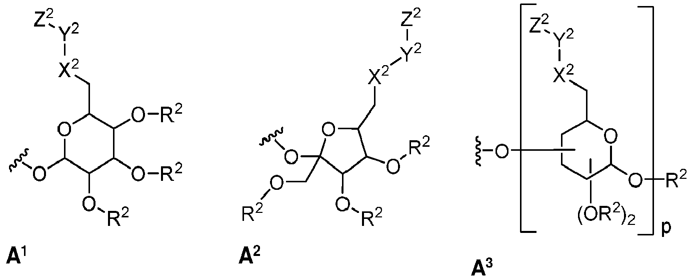
R¹ and R² are each independently selected from H, R^{a}, and -C(O)-R^{a};
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, and -NH-;
Y¹ and Y² are each independently an optionally substituted C₁₋₃₀ aliphatic group wherein one or more carbons are optionally and independently replaced by -Cy-, -NH-, -NHC(O)-, -C(O)NH-, -NHC(O)O-, -OC(O)NH-, -NHC(O)NH-, -NHC(S)NH-, -C(S)NH- - C(O)NHSO₂-, -SO₂NHC(O)-,-OC(O)O-, -O-, -C(O)-, -OC(O)-, -C(O)O-, -SO-, or -SO₂-;
each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N+(M)₃, 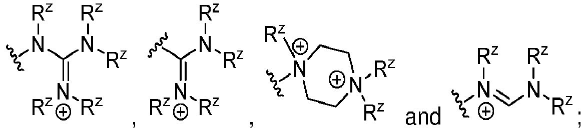
each M is independently ―C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5;
provided that when A is A¹, then:
(i) each R¹ and R² is not ―CO-(CH₂)₄-CH₃, X¹ and X² are not both -S-, Y¹ and Y² are not both ―(CH₂)₂- and Z¹ and Z² are not both ―N+(H)₃,
(ii) each R¹ and R² is not ―O-(CH₂)₅-CH₃ or ―O-(CH₂)₁₃-CH₃, X¹ and X² are not both -S-, Y¹ and Y² are not both ―(CH₂)₂-, and Z¹ and Z² are not both ―N⁺(H)₃,
(iii) each R¹ and R² is not ―CO-(CH₂)₄-CH₃, X¹ and X² are not both -S-, Y¹ and Y² are not both ―(CH₂)₂-NH-C(S)-NH-(CH₂)₂-, and Z¹ and Z² are not both ―N+(H)₃, and
(iv) Cy is not triazolyl.

2. The composition of claim 1, wherein the oligosaccharide is of formula la: 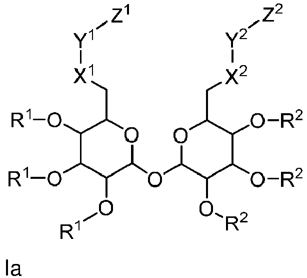 or a pharmaceutically acceptable salt thereof.

3. The composition of claim 1, wherein the oligosaccharide is of formula Ib: 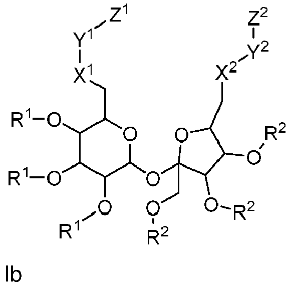 or a pharmaceutically acceptable salt thereof.

4. The composition of claim 1, wherein the oligosaccharide is of formula Ic: 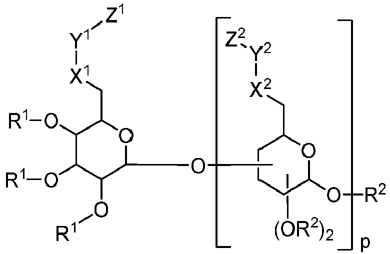 or a pharmaceutically acceptable salt thereof.

5. The composition of any one of claims 1-4, wherein R¹ and R² are each independently selected from R^{a} and -C(O)-R^{a}, and R^{a} is C₁-C₂₀ aliphatic.

6. The composition of any one of claims 1-5, wherein R¹ and R² are each ―C(O)-R^{a}, and R^{a} is C₁-C₁₄ aliphatic.

7. The composition of any one of claims 1-6, wherein each R^{a} is C₅-C₁₀ linear alkyl.

8. The composition of claim 1, wherein R¹ and R² are each ―C(O)-R^{a}, and R^{a} is

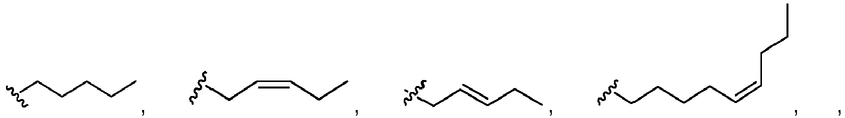

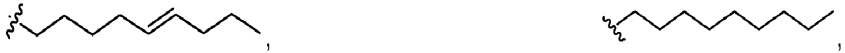

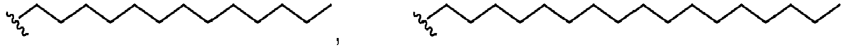

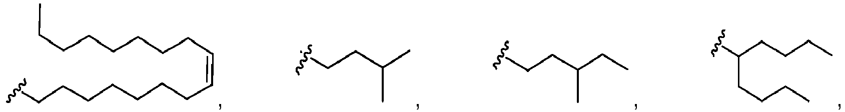

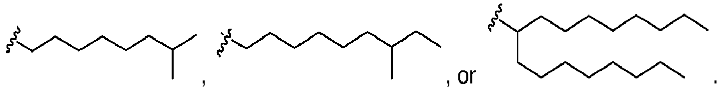

9. The composition of any one of claims 1-8, wherein X¹ and X² are each -S-.

10. The composition of any one of claims 1-9, wherein Y¹ and Y² are each selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-NHC(O)-C₀-C₄ aliphatic, C₀-C₄-aliphatic-NHSO₂-C₀-C₄ aliphatic, and C₀-C₄-aliphatic-C(O)-C₀-C₄ aliphatic.

11. The composition of any one of claims 1-10, wherein Y¹ and Y² are each C₁-C₄ aliphatic-NHC(S)NH-C₁-C₄ aliphatic.

12. The composition of claim 1, wherein Y¹ and Y² are each -CH₂-CH₂-NHC(S)NH-CH₂-CH₂-.

13. The composition of any one of claims 1-12, wherein a moiety X¹-Y¹-Z¹ is

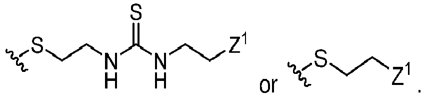

14. The composition of any one of claims 1-13, wherein a moiety X²-Y²-Z² is

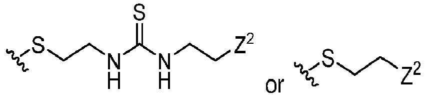

15. The composition of any one of claims 1-14, wherein Z¹ and Z² are each independently selected from: N⁺(M)₃, 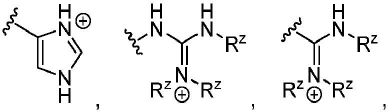 and

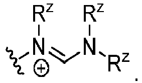

16. The composition of any one of claims 1-14, wherein Z¹ and Z² are each independently selected from:

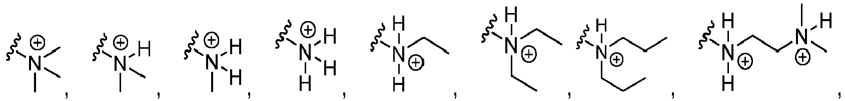

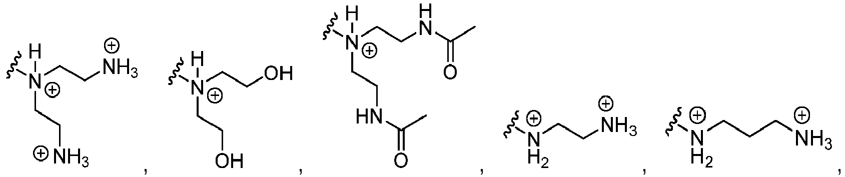

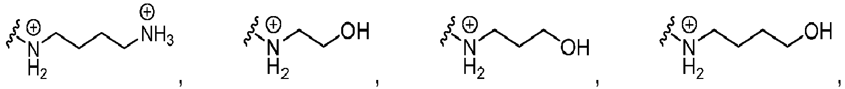

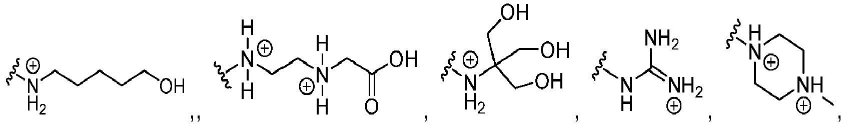

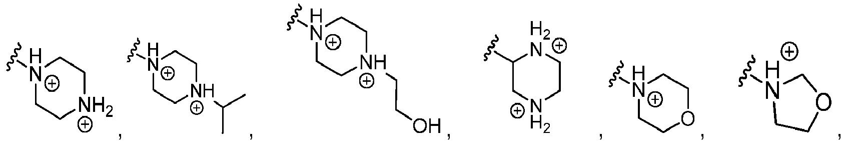

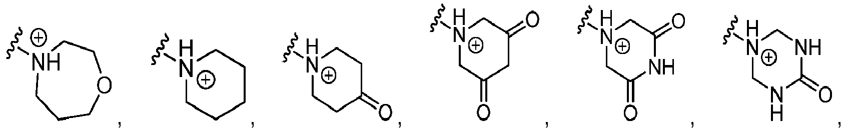

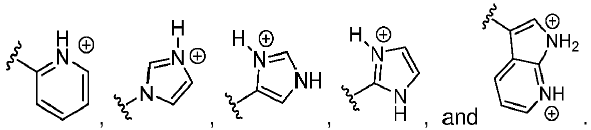

17. The compound of claim 1, wherein the oligosaccharide is a compound of formula Id: 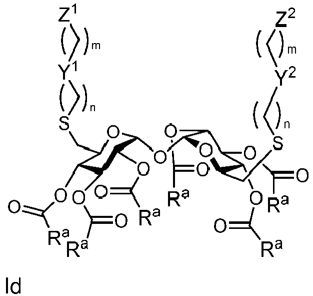 or a pharmaceutically acceptable salt thereof, wherein n and m are each independently selected from 0, 1, 2, 3, 4, 5, or 6.

18. The compound of claim 17, wherein the oligosaccharide is a compound of formula Id-i: 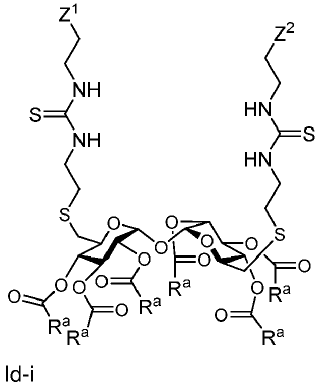 or a pharmaceutically acceptable salt thereof, wherein n and m are each independently selected from 0, 1, 2, 3, 4, 5, or 6.

19. The composition of any one of claims 1-18, further comprising one or more suitable counterions.

20. The composition of claim 1, wherein the oligosaccharide is selected from:
| **Name** | **Structure** |
|---|---|
| JLF19 | 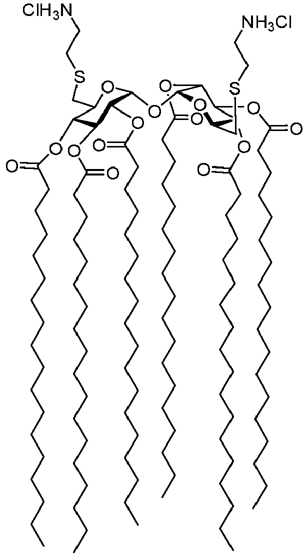 |
| JLF20 | 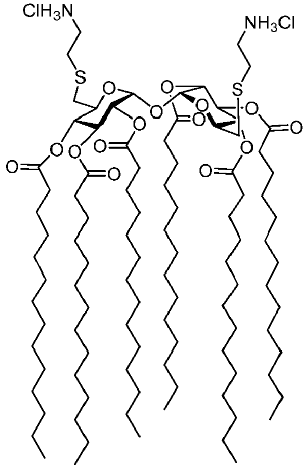 |
| JLF23 | 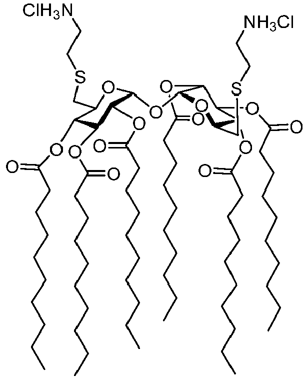 |
| JLF25 | 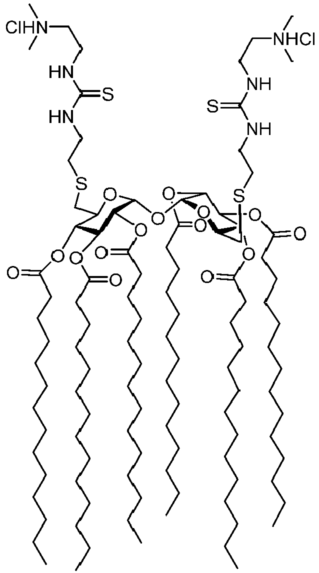 |
| JLF26 | 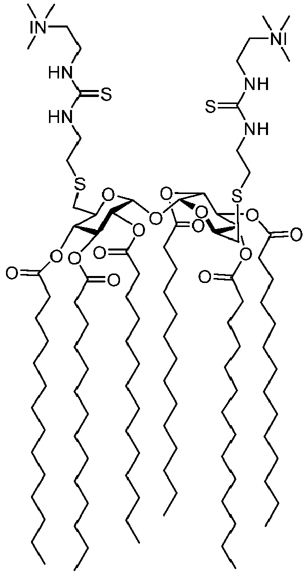 |
| JLF29 | 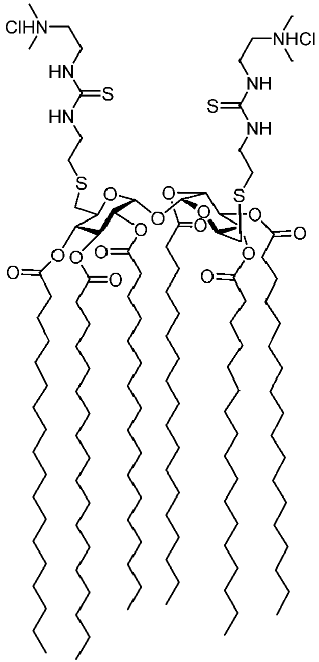 |
| JLF30 | 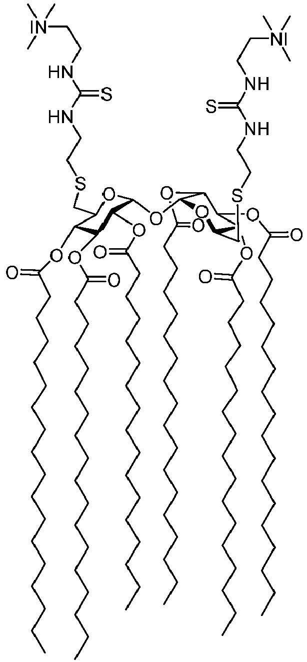 |
| JLF31 | 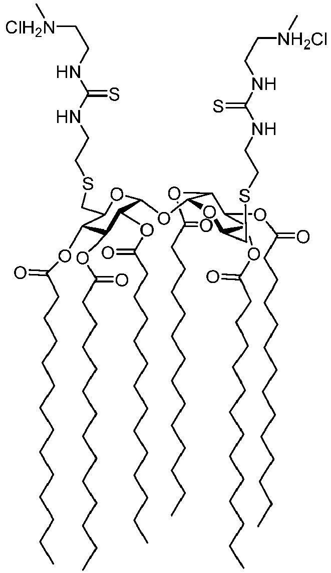 |
| JLF32 | 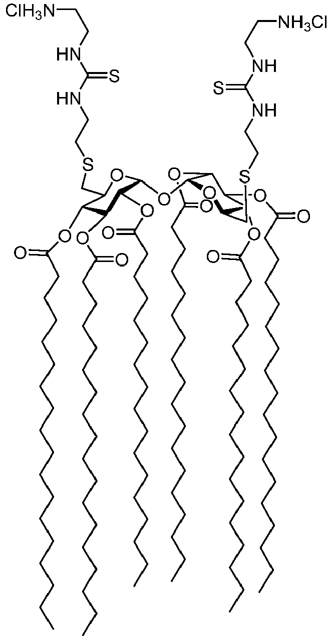 |
| JLF33 | 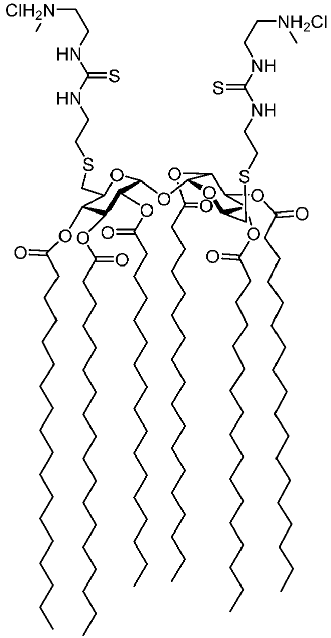 |
| JLF35 | 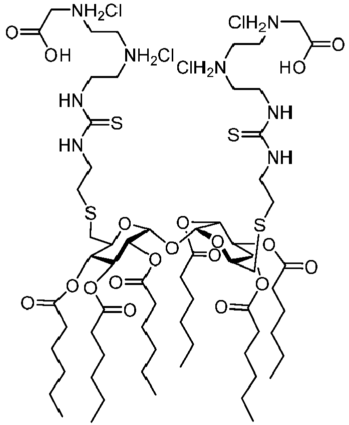 |
| JLF40 | 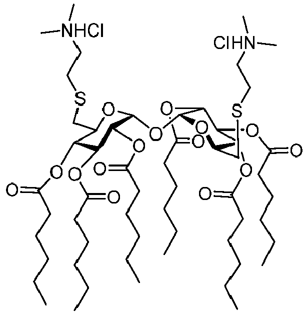 |
| JLF41 | 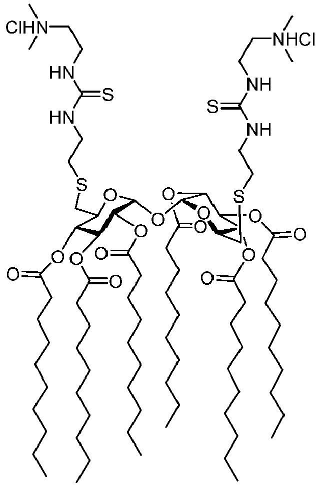 |
| JLF42 | 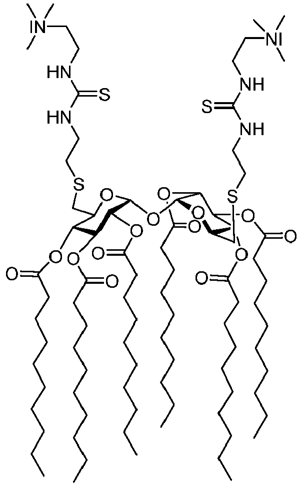 |
| JLF43 | 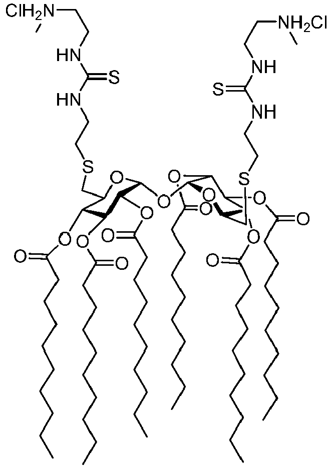 |
| JLF46 | 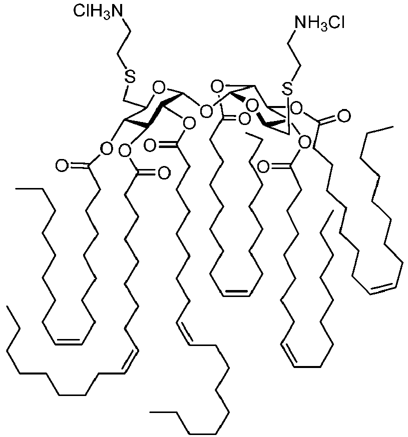 |
| JLF48 | 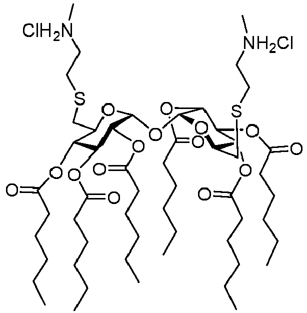 |
| JLF50 | 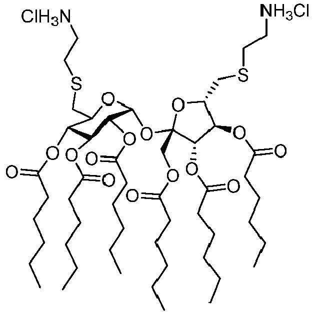 |
| JLF54 | 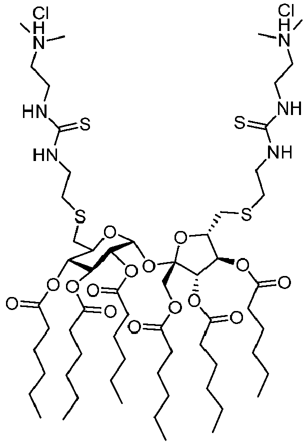 |
| JLF55 | 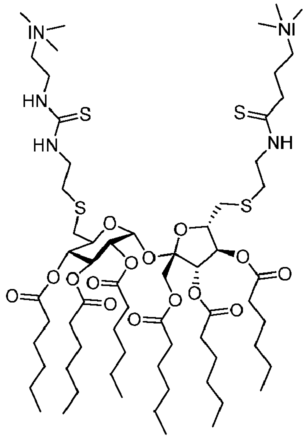 |
| JLF58 | 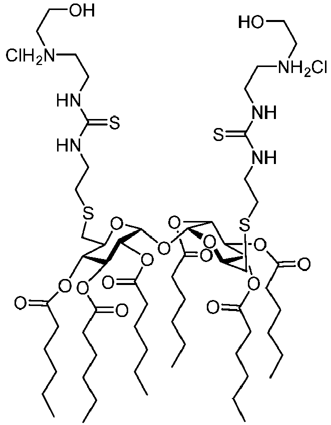 |
| JLF59 | 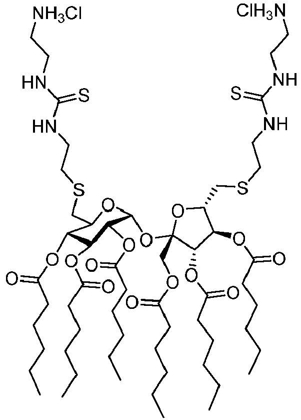 |
| JLF60 | 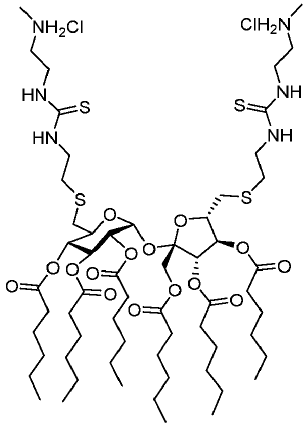 |
| JLF62 | 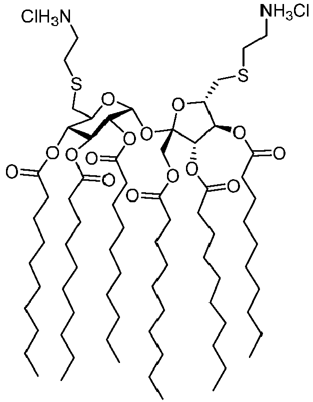 |
| JLF66 | 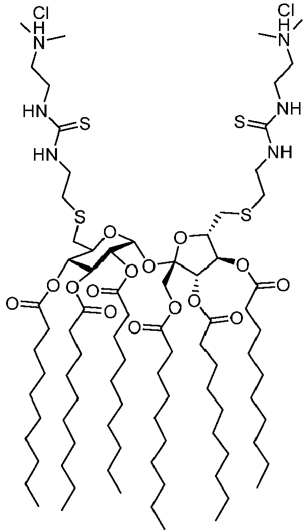 |
| JLF67 | 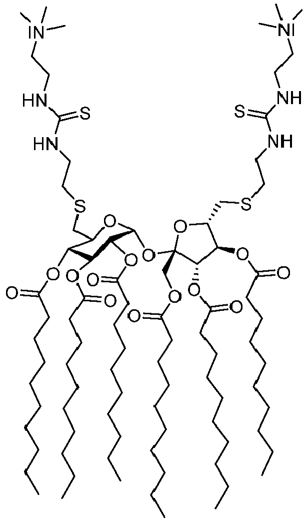 |
| JLF68 | 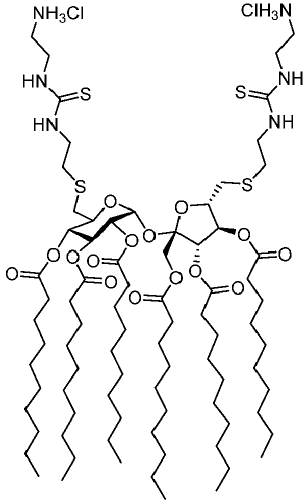 |
| JLF69 | 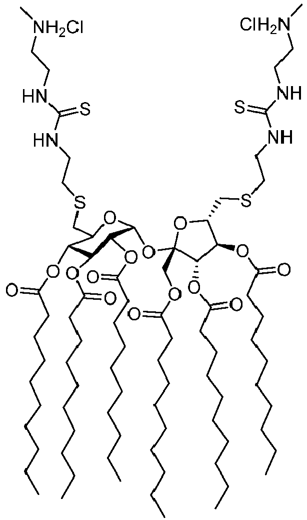 |
| JLF76 | 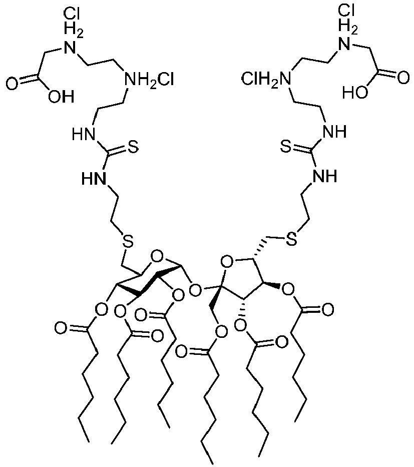 |
| JLF77 | 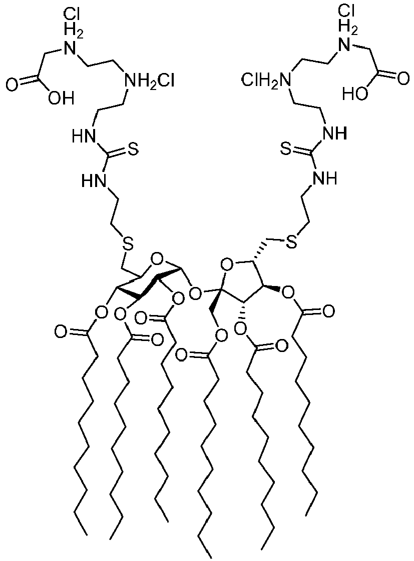 |
| JLF78 | 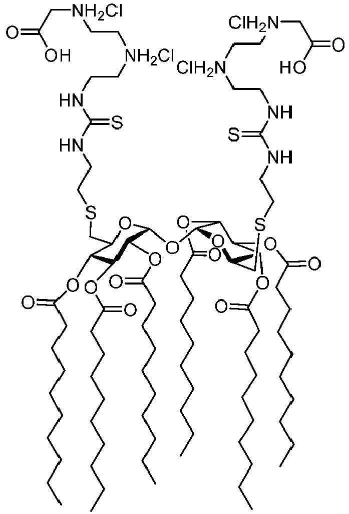 |
| JLF81 | 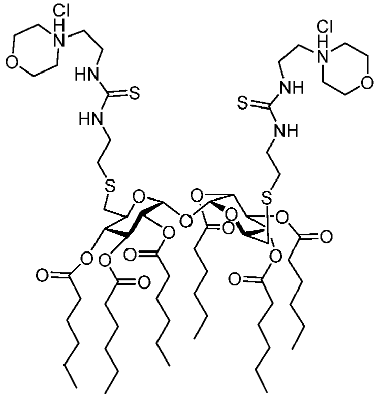 |
| JLF82 | 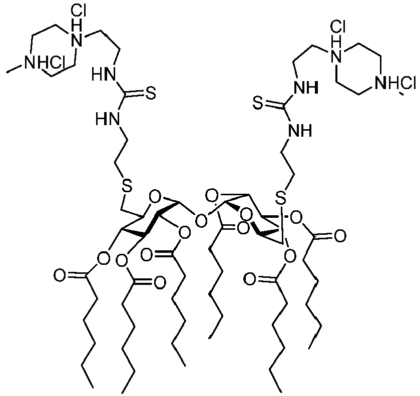 |
| JLF87 | 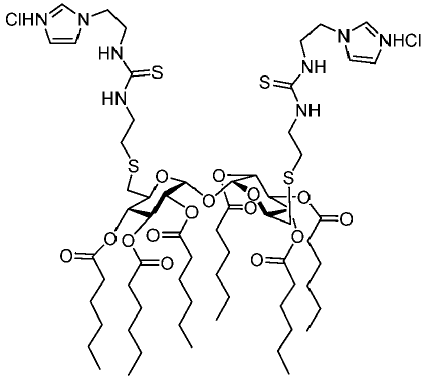 |
| JLF90 | 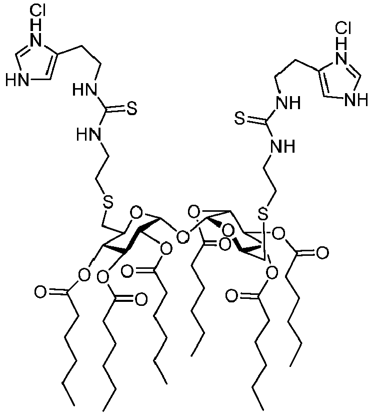 |
| JLF93 | 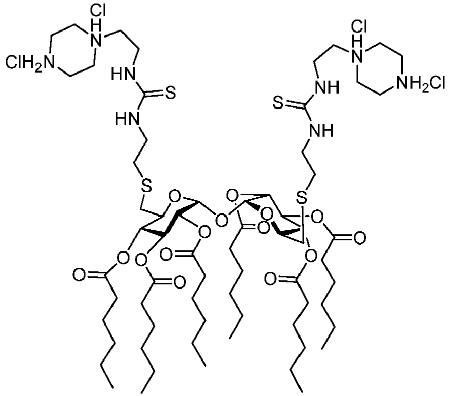 |
| JLF94 | 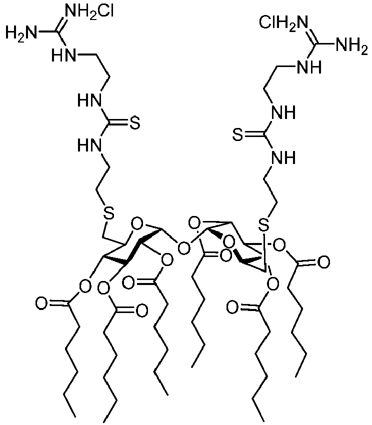 |
| JLF95 | 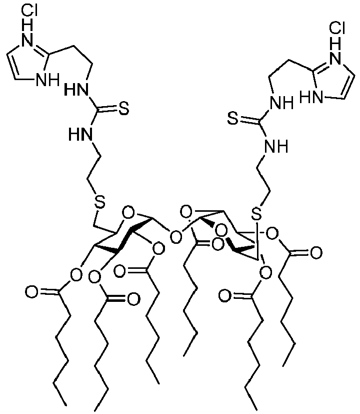 |
| JLF96 | 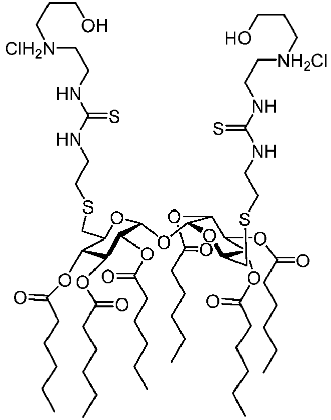 |
| JLF97 | 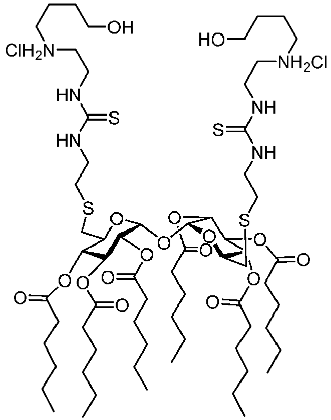 |
| JLF98 | 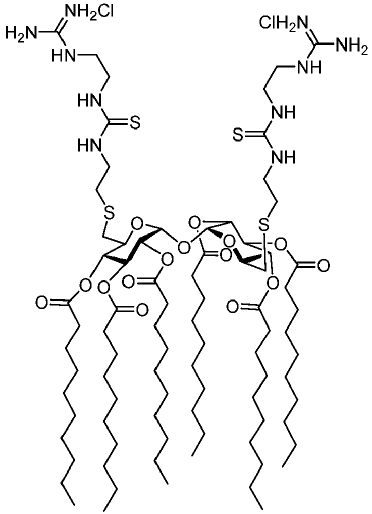 |
| JLF99 | 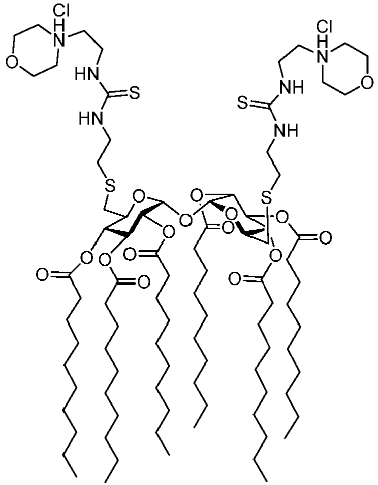 |
| JLF102 | 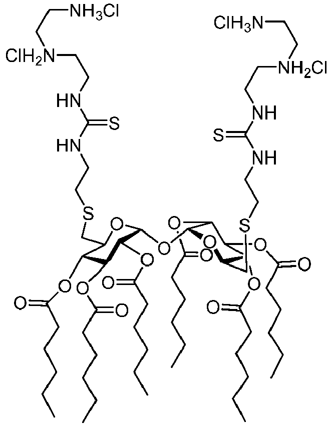 |
| JLF103 | 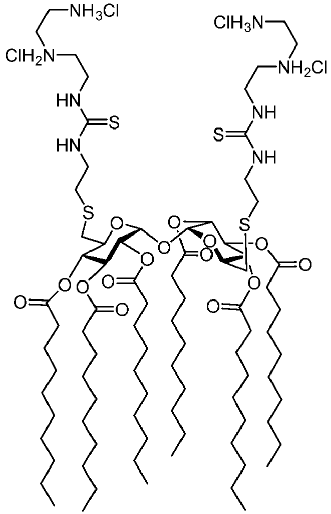 |
| JLF106 | 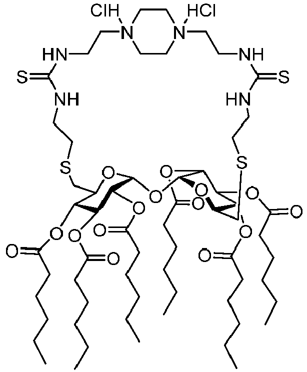 |
| JLF108 | 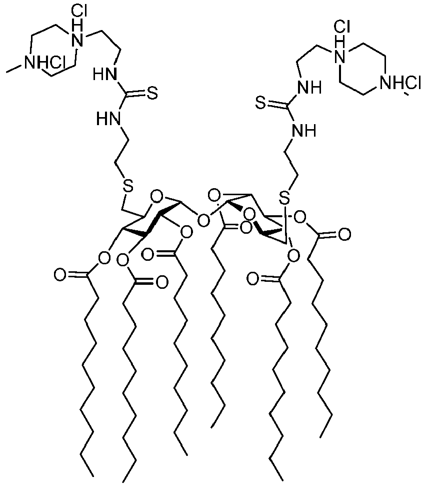 |
| JLF110 | 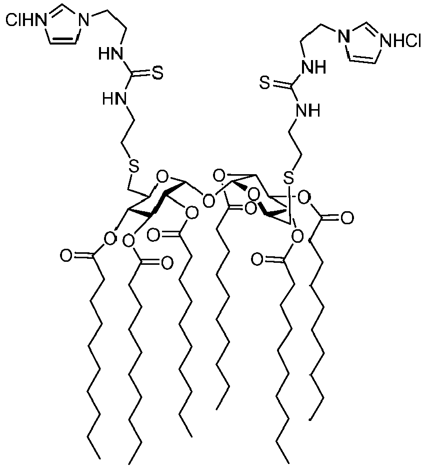 |
| JLF111 | 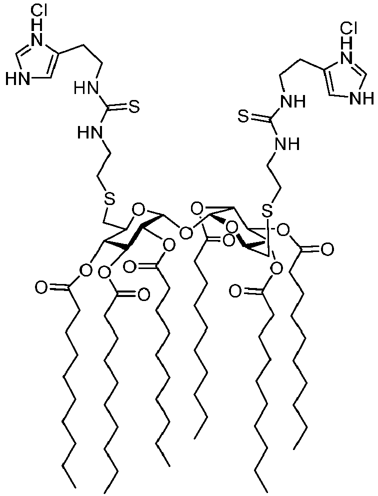 |
| JLF115 | 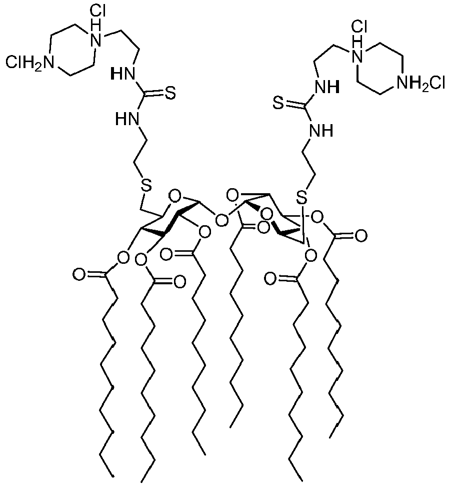 |
| JLF120 | 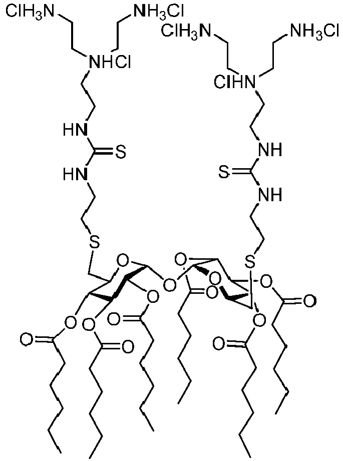 |
| JLF121 | 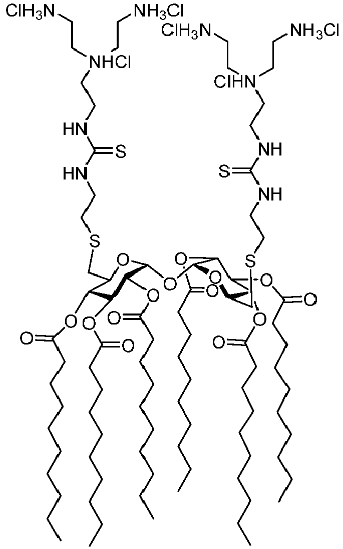 |
| JLF125 | 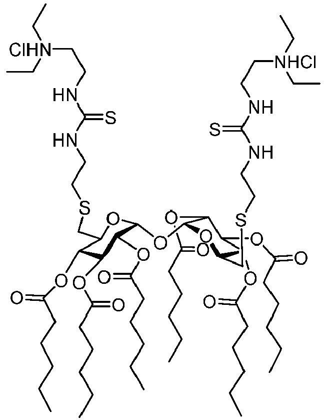 |
| JLF128 | 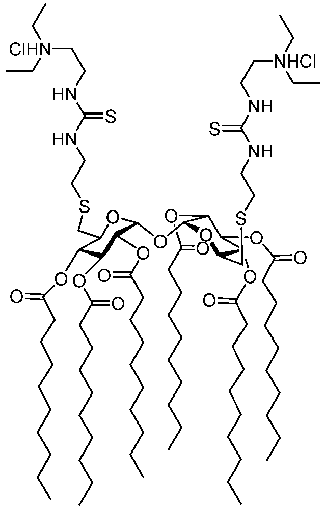 |
| JLF130 | 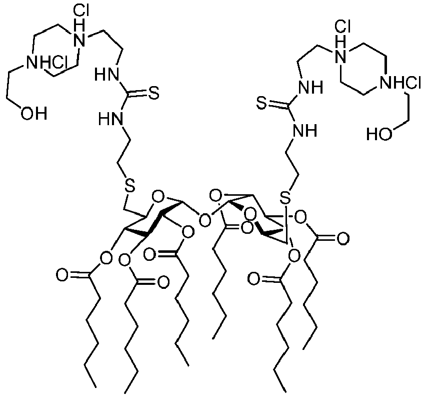 |
| JLF131 | 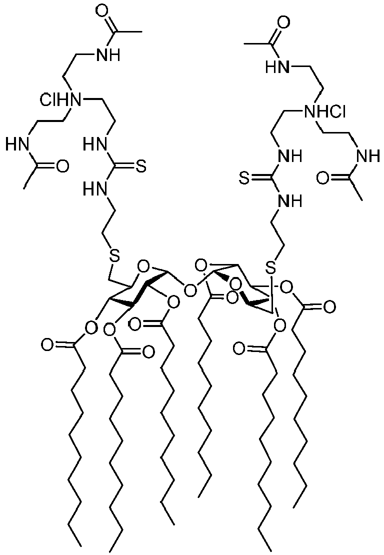 |
| JLF134 | 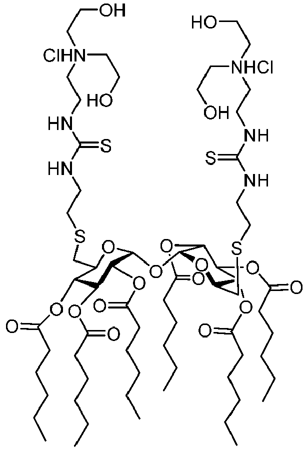 |
| JLF135 | 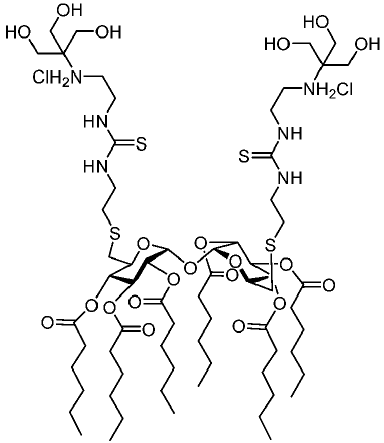 |
| JLF138 | 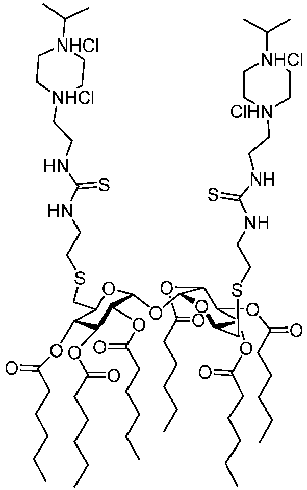 |
| JLF139 | 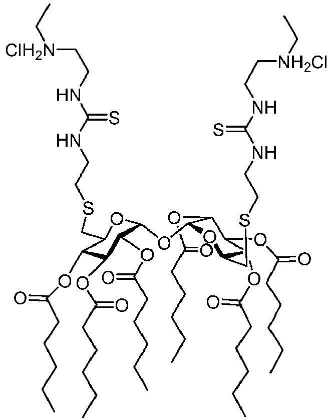 |
| JLF141 | 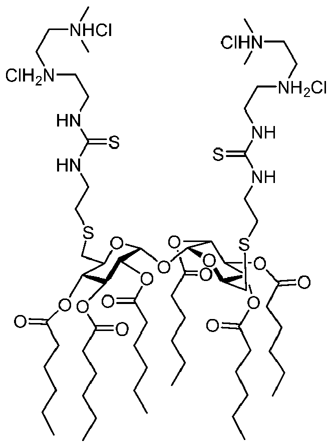 |
| JLF142 | 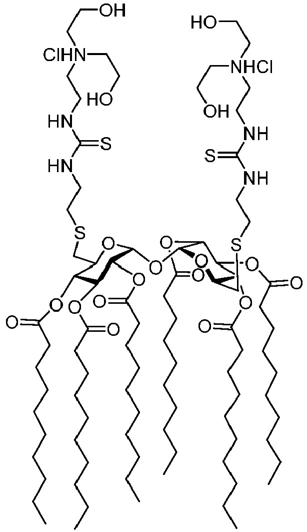 |
| JLJB604 | 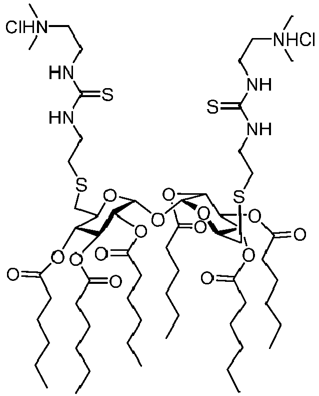 |
| JLJB605 | 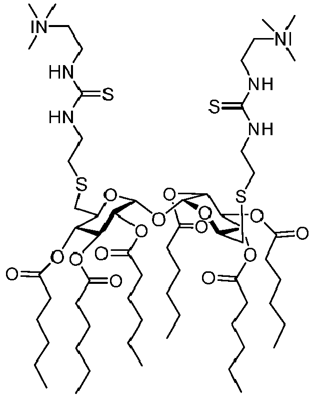 |
| JLJB617 | 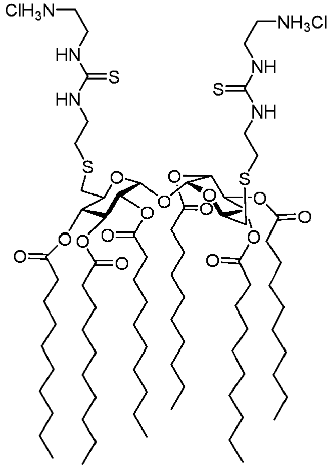 |
| JLJB618 | 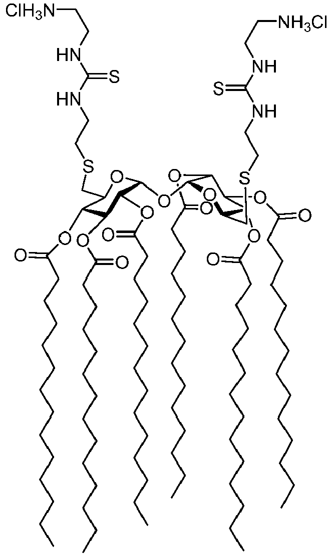 |
| JLJB619 | 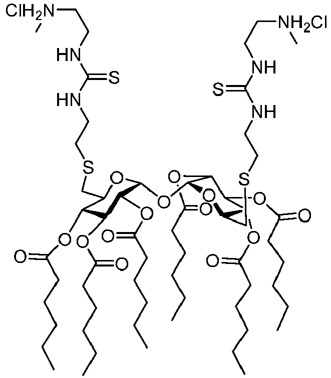 |
| PER20 | 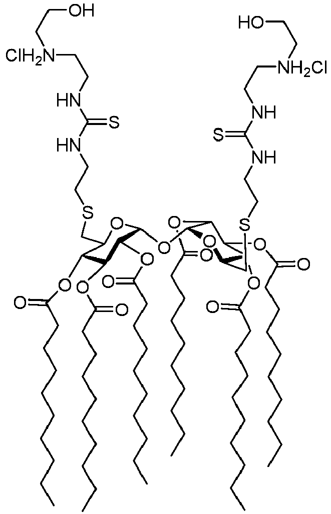 |
or a pharmaceutically acceptable salt thereof.

21. A complex comprising the composition oligosaccharide of any one of claims 1-20, and a nucleic acid.

22. The complex of claim 21, wherein the nucleic acid is RNA.

23. The complex of claim 22, wherein the RNA is a modRNA or a saRNA.

24. The complex of claims 22 or 23, wherein a ratio of N/P is less than 20:1.

25. The complex of claim 24, wherein a ratio of N/P is less than or equal to 12:1.

26. The complex of any one of claims 21-25, wherein the complex has a diameter of about 30 nm to about 300 nm.

27. The complex of any one of claims 21-26, wherein the complex has a diameter of about 50nm to 200nm.

28. The complex of any one of claims 21-27, further comprising a pharmaceutically acceptable surfactant.

29. The complex of claim 28, wherein the pharmaceutically acceptable surfactant is a polysorbate.

30. The complex of claims 28 or 29, wherein a ratio of the oligosaccharide to the pharmaceutically acceptable surfactant is from about 1:0.0075 to about 1:3.

31. A method of increasing or causing increased expression of RNA in a target in a subject comprising administering to the subject the complex of any one of claims 21-30.

32. The method of claim 31, wherein the target is selected from the lungs, liver, spleen, heart, brain, lymph nodes, bladder, kidneys, and pancreas.

33. A method of treating a disease, disorder, or condition in a subject comprising administering to the subject a complex of any one of claims 21-30.

34. The method of claim 33, wherein the disease, disorder, or condition is an infectious disease, cancer, a genetic disorder, an autoimmune disease, or a rare disease.

35. The method of any one of claims 31-34, wherein the complex is administered intramuscularly.

36. The method of claim 35, wherein the complex is of a diameter between 50 nm and 100 nm.

37. The method of any one of claims 31-34, wherein the complex is administered subcutaneously.

38. A method of preparing an oligosaccharide of formula II: 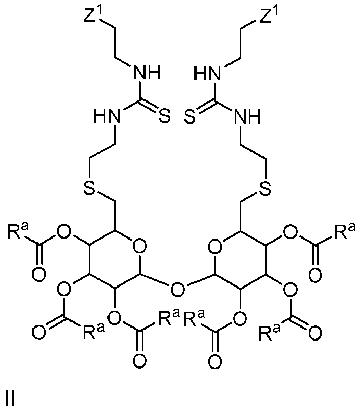 comprising contacting a compound of formula III: 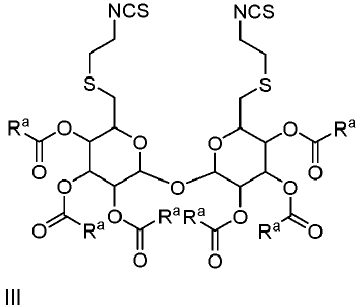 with a compound of formula IV: 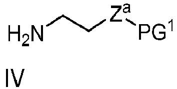
in the presence of an acid,
wherein
each R^{a} is optionally substituted -C₁-C₂₀ aliphatic;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃, 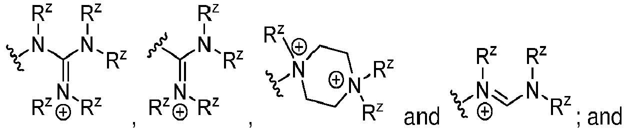
each M is independently ―C₀-C₆ aliphatic-R^{z} or ―C₀-C₆ aliphatic-N⁺(R^{z})₃;
each R^{z} is independently selected from H, optionally substituted C₁-C₆ aliphatic,
optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S;
Z^{a}-PG¹ is Z¹ wherein one R^{z} has been replaced with a PG¹ group; and
PG¹ a suitable nitrogen protecting group,
provided that Z¹ and Z² are not both ―N⁺(H)₃.

39. The method of claim 38, comprising first contacting a compound of formula V 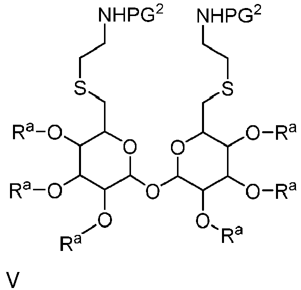
with an acid and then contacting the resulting product with CSCI₂ to provide a compound of formula III;
wherein PG² is a suitable acid sensitive nitrogen protecting group.

40. The method of claim 39, comprising contacting a compound of formula VI 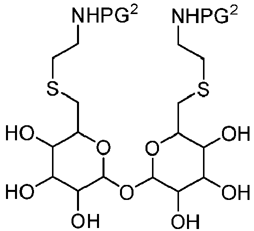 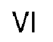 with a compound of formula VII:
R^{a}-COCl VII
to provide a compound of formula V.

41. The method of claim 40, comprising contacting a compound of formula VIII: 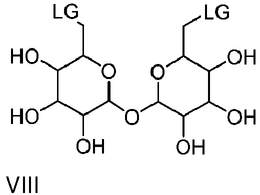 with a compound of formula IX:
HS(CH₂)₂NHPG² IX
to provide a compound of formula VI,
wherein LG is a suitable leaving group.
